(19) Europäisches Patentamt / European Patent Office / Office européen des brevets

(11) **EP 4 158 636 B1**

(12) **EUROPEAN PATENT SPECIFICATION**

(45) Date of publication and mention
of the grant of the patent:
**13.05.2026 Bulletin 2026/20**

(21) Application number: **21734692.3**

(22) Date of filing: **28.05.2021**

(51) International Patent Classification (IPC):
**G16B 20/00** (2019.01)     **C12Q 1/6816** (2018.01)

(52) Cooperative Patent Classification (CPC):
(C-Sets available)
**G16B 20/00; C12Q 1/6816**          (Cont.)

(86) International application number:
**PCT/US2021/034965**

(87) International publication number:
**WO 2021/243274 (02.12.2021 Gazette 2021/48)**

(54) **MACHINE LEARNING-BASED ANALYSIS OF PROCESS INDICATORS TO PREDICT SAMPLE REEVALUATION SUCCESS**

AUF MASCHINENLERNEN BASIERENDE ANALYSE VON PROZESSINDIKATOREN ZUR VORHERSAGE DES ERFOLGS EINER PROBENNEUAUSWERTUNG

ANALYSE BASÉE SUR L'APPRENTISSAGE AUTOMATIQUE D'INDICATEURS DE PROCESSUS POUR PRÉDIRE LA RÉUSSITE DE LA RÉÉVALUATION D'ÉCHANTILLON

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **29.05.2020  US 202063032083 P**
**27.05.2021  US 202117332904**

(43) Date of publication of application:
**05.04.2023  Bulletin 2023/14**

(73) Proprietor: **Illumina, Inc.**
**San Diego, CA 92122 (US)**

(72) Inventors:
• **GIETZEN, Kimberly Jean**
**San Diego, California 92122 (US)**
• **REZAEI, Naghmeh**
**San Diego, 92122 (US)**
• **FILIPE CRUZ, Pedro Miguel**
**San Diego, California 92122 (US)**

(74) Representative: **Robinson, David Edward Ashdown**
**Marks & Clerk LLP**
**1 New York Street**
**Manchester M1 4HD (GB)**

(56) References cited:
**WO-A1-2017/106918**

• HUANG CHING YU AUSTIN ET AL: "Auto-validation of fluorescent primer extension genotyping assay using signal clustering and neural networks", BMC BIOINFORMATICS, BIOMED CENTRAL , LONDON, GB, vol. 5, no. 1, 2 April 2004 (2004-04-02), pages 36, XP021000617, ISSN: 1471-2105, DOI: 10.1186/1471-2105-5-36
• ILLUMINA: "Evaluation of Infinium Genotyping Assay Controls Training Guide", 1 January 2012 (2012-01-01), XP055837234, Retrieved from the Internet <URL:https://support.illumina.com/content/dam/illumina-support/courses/eval-inf-controls/story_content/external_files/Infinium_Controls_Training_Guide.pdf> [retrieved on 20210902]

**(Cont. next page)**

(52) Cooperative Patent Classification (CPC): (Cont.)

C-Sets
**C12Q 1/6816, C12Q 2533/101, C12Q 2563/107,
C12Q 2565/537**

## Description

### PRIORITY APPLICATION

This application claims the benefit of U.S. Application 17/332,904, entitled "MACHINE LEARNING-BASED ANALYSIS OF PROCESS INDICATORS TO PREDICT SAMPLE REEVALUATION SUCCESS," filed May 27, 2021 (Attorney Docket No. ILLM 1027-2/IP-1973-US) which claims the benefit of U.S. Provisional Patent Application No.: 63/032,083, entitled "MACHINE LEARNING-BASED ANALYSIS OF PROCESS INDICATORS TO PREDICT SAMPLE REEVALUATION SUCCESS," filed May 29, 2020 (Attorney Docket No. ILLM 1027-1/IP-1973-PRV).

### FIELD OF THE TECHNOLOGY DISCLOSED

The technology disclosed relates to evaluation of readouts from process controls for production rerun decisions.

### BACKGROUND

The subject matter discussed in this section should not be assumed to be prior art merely as a result of its mention in this section. Similarly, a problem mentioned in this section or associated with the subject matter provided as background should not be assumed to have been previously recognized in the prior art. The subject matter in this section merely represents different approaches, which in and of themselves can also correspond to implementations of the claimed technology.

Genotyping is a process that can take multiple days to complete. The process is vulnerable to process and sample errors. Collected samples for genotyping are extracted and distributed in sections and areas of image-generating chips. The samples are then chemically processed through multiple steps to generate fluorescing images. The process generates a quality score for each section analyzed. This quality cannot provide insight into the root cause of failure of a low-quality process. The document Huang et al. BMC Bioinformatics 2004, 5:36 teaches a method of quality control of a genotyping assay using signal clustering and neural networks.

Accordingly, an opportunity arises to introduce new methods and systems to evaluate quality score and other outputs from the genotyping process to determine the root cause of failure.

### BRIEF DESCRIPTION OF THE DRAWINGS

In the drawings, like reference characters generally refer to like parts throughout the different views. Also, the drawings are not necessarily to scale, with an emphasis instead generally being placed upon illustrating the principles of the technology disclosed. In the following description, various implementations of the technology disclosed are described with reference to the following drawings, in which:

FIG. 1 shows an architectural level schematic of a system in which readouts from process control probes and call rates for one or more genotyping sample evaluation runs are scored to predict further sample reevaluation.

FIG. 2 illustrates subsystem components of the feature generator of FIG. 1.

FIG. 3 presents process steps for an example genotyping process.

FIG. 4 presents images of sections of an image-generating chip after successful completion of a production run.

FIG. 5 is an example illustrating process probes positioned on a section of an image-generating chip.

FIG. 6 is an example grouping of process probes into sample-independent and sample-dependent process probes.

FIGs. 7A-1 and 7A-2 present examples of staining process probes and signal intensities from staining process probes.

FIGs. 7B-1 and 7B-2 present examples of extension process probes and signal intensities from extension process probes.

FIGs. 7C-1 and 7C-2 present examples of target removal process probes and signal intensities from target removal process probes.

FIGs. 7D-1 and 7D-2 present examples of hybridization process probes and signal intensities from hybridization process probes.

FIGs. 7E-1 and 7E-2 present examples of stringency process probes and signal intensities from stringency process probes.

FIGs. 7F-1 and 7F-2 present examples of non-polymorphic process probes and signal intensities from non-polymorphic process probes.

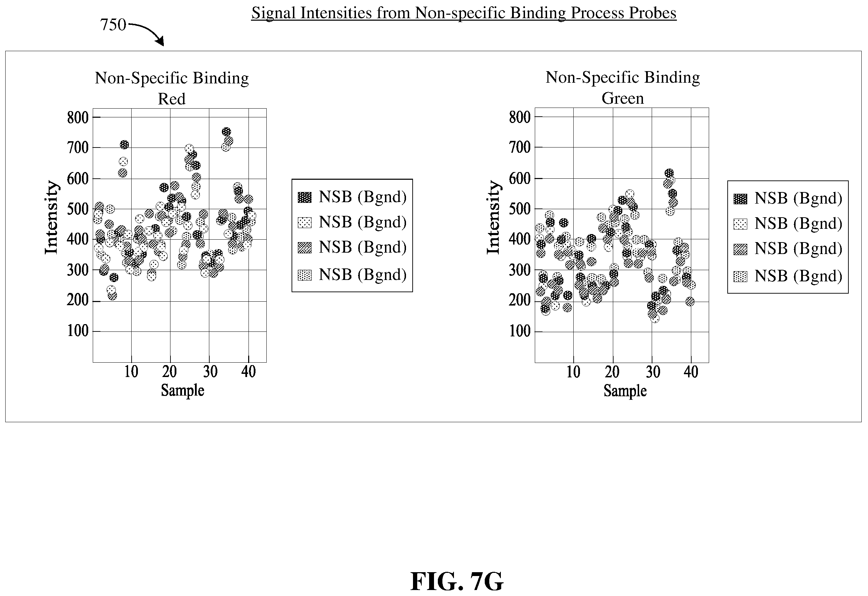

FIG. 7G presents signal intensities from non-specific binding process probes.

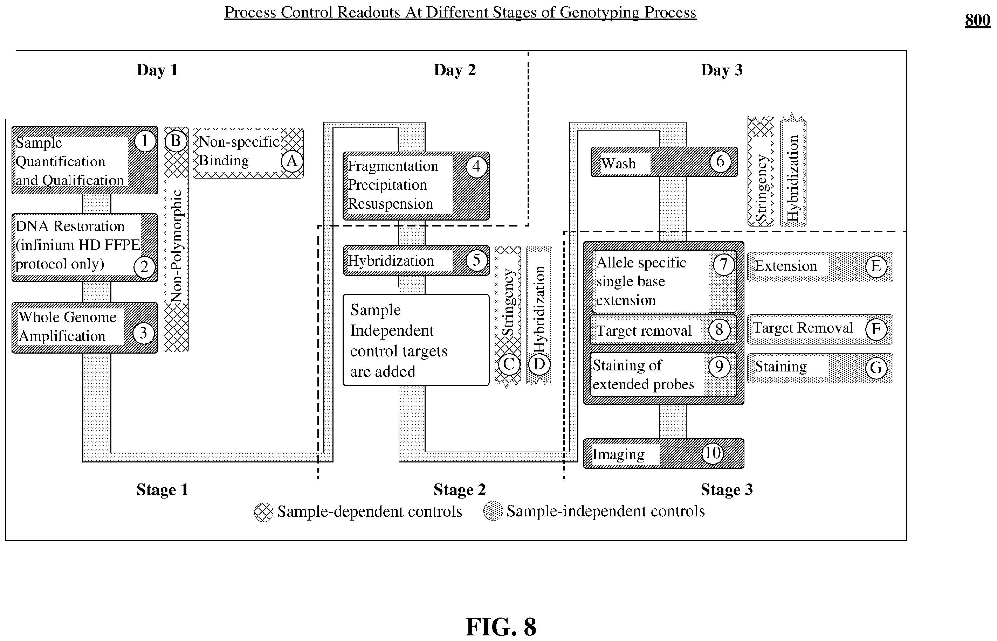

FIG. 8 is another example of grouping process probes according to different stages of the genotyping process.

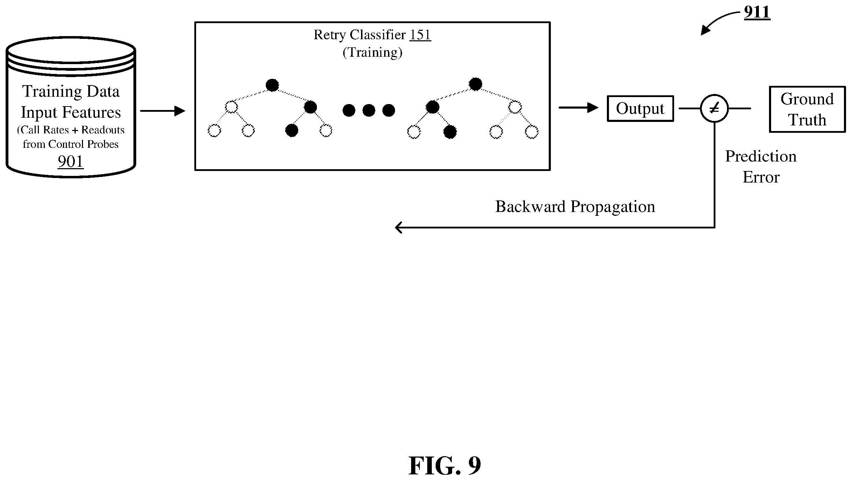

FIG. 9 illustrates the training of a retry classifier using labeled training data comprising call rates and readouts from production runs.

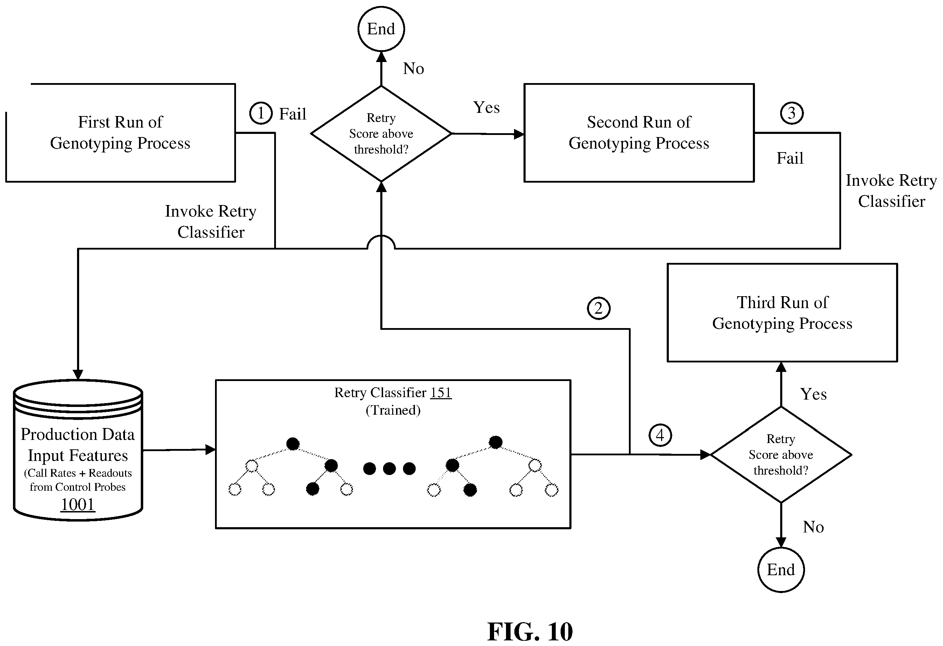

FIG. 10 illustrates a process in which call rates and readout from process probes from inconclusive production runs are given as input to a retry classifier to generate a retry success confidence score indicative of whether a sample reevaluation will produce a conclusive result.

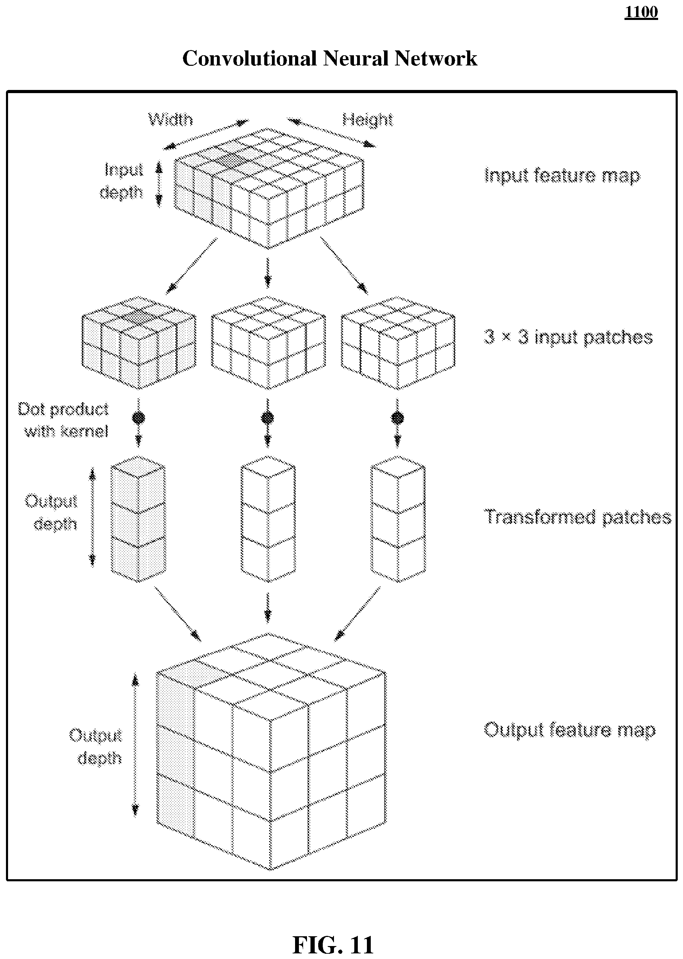

FIG. 11 is an example convolutional neural network to generate a retry success confidence score.

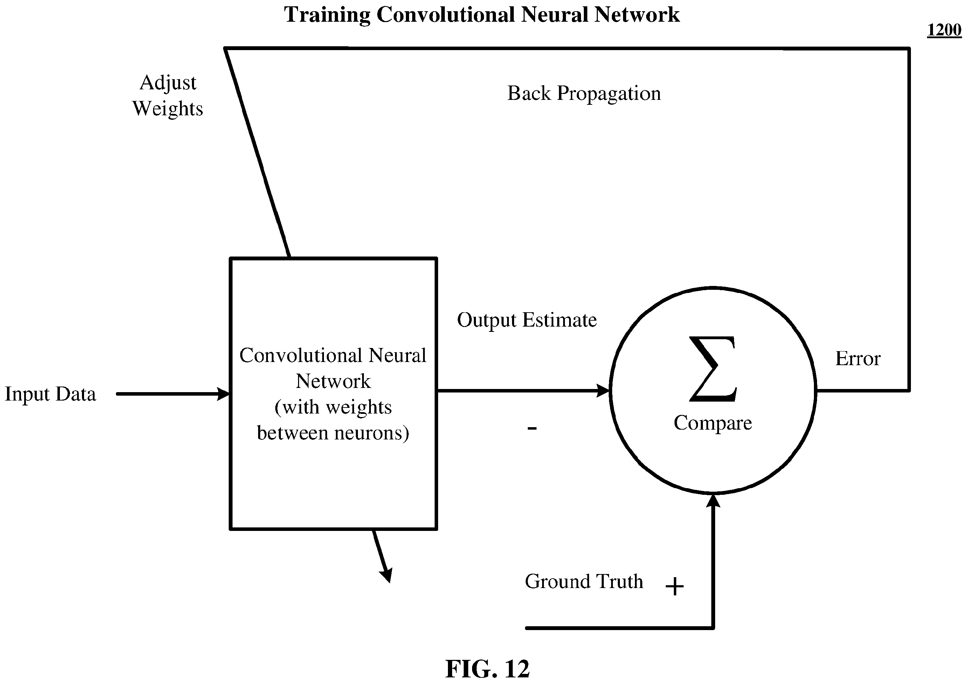

FIG. 12 is a block diagram illustrating the training of the convolutional neural network of FIG. 11.

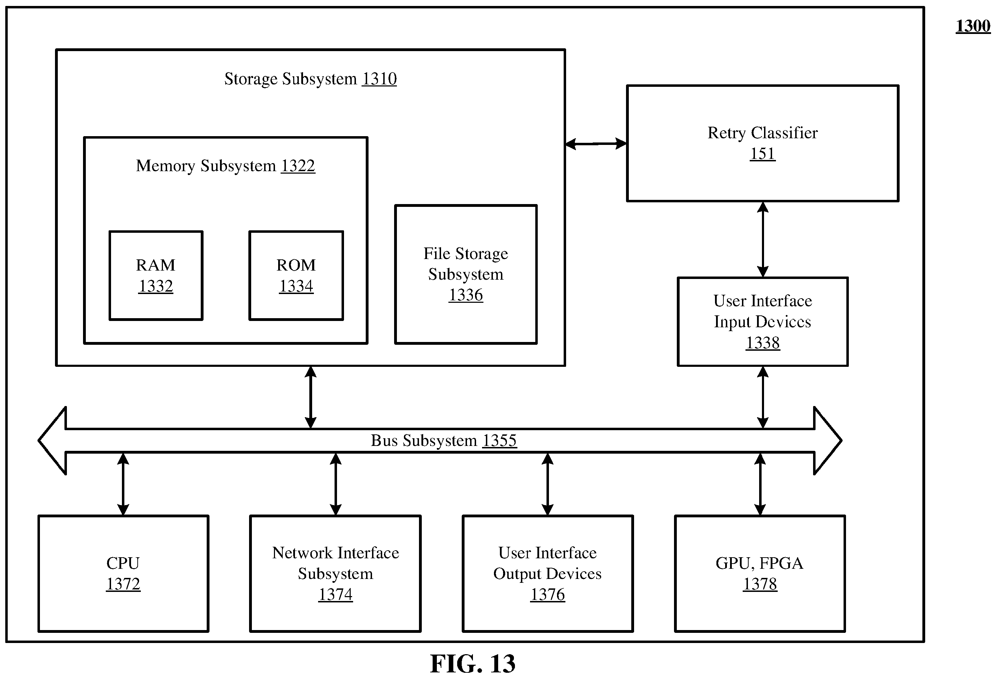

FIG. 13 is a simplified block diagram of a computer system that can be used to implement the technology disclosed.

## DETAILED DESCRIPTION

The following discussion is presented to enable any person skilled in the art to make and use the technology disclosed, and is provided in the context of a particular application and its requirements. The scope of protection is defined by the appended claims.

## Introduction

The technology disclosed is related to the evaluation of production processes to determine differences in genetic makeup (genotype). Genotyping is a complex, time consuming and expensive process that can take multiple days to complete. The production process is vulnerable to both process and sample errors. Collected samples are extracted, distributed in sections and areas of image-generating chips (such as BeadChips), then chemically processed through multiple steps to generate fluorescing images. The output from a process run is a "call rate" representing the percentage of single nucleotide polymorphisms (or SNPs) whose quality score (also referred to as GenCall score) is greater than a specified threshold. GenCall score is a quality metric that indicates the reliability of each genotype call. The GenCall score has a value between 0 and 1 assigned to every called genotype. Genotypes with lower GenCall scores are located further from the center of a cluster and have a lower reliability. GenCall scores are calculated using information from the clustering of the samples. To get a GenCall score, each SNP is evaluated based on characteristics of the clusters such as angle, dispersion, overlap, and intensity. An example value threshold for GenCall score is 0.15. The SNPs with a GenCall score of less than the threshold are referred to as no-calls and are not included in the calculation of the call rate. Other values of the threshold can be used e.g., 0.2, 0.25, etc. A separate call rate result is reported for each section of the image-generating chip. The call rate above a threshold (such as 98%) can be used to accept or reject the genotyping result for a section. Different values of the threshold can be used. If the call rate of a section is below the threshold, the genotyping process run is considered as inconclusive or failed.

An operator can rerun a failed (or inconclusive) production run using the same sample. However, reruns are not useful when failure of the production run is due to sample related errors. It is difficult to predict, with high confidence, whether a production run failure is caused by process errors or sample errors. Getting a new sample from a customer can increase process turn-around time from one to six months. On the other hand, production reruns can lead to high process costs, if failure is due to sample errors. Significant savings in processing time and cost can be achieved by providing an indication to operators regarding the likely success of reruns of inconclusive production runs.

Image-generating chips are equipped with internal process probes (also referred to as process control probes or control probes) designed to generate signals indicative of processing parameters at successive stages of sample evaluation during the genotyping process. A "probe" can be defined as a single-stranded nucleic acid that is used to find a (target) complementary nucleic acid sequence. The process probes can be attached to beads at known locations on sections of the image-generating chip and bind to complementary sequences mixed with reagents. Some types of process probes (such as hybridization and target removal) react with synthetic targets in reagents (such as a RA1 buffer) rather than reacting to targets in the sample DNA. Readouts from the process probes are available at the end of a production run along with call rates for sections. Process probes can respond to complementary sequences in a DNA sample, reagents, and other chemicals mixed with reagents to produce signals indicative of sample quality, reagent delivery, reagent quality, or other process conditions such as temperature, etc. We briefly present the different types of probes in two categories of process probes: sample-independent process probes and sample-dependent process probes.

A first type of process probes is referred to as sample-independent process probes. These probes evaluate the operating conditions during process steps in the genotyping process or the performance (or quality) of reagents. The readouts from sample-independent controls do not depend on sample quality as the probes respond to non-sample chemicals. Examples of sample-independent process probes include staining controls, extension controls, target removal controls, and hybridization controls. The staining process includes attaching fluorescent labels to single base extensions to probes attached to the bead. Staining controls consist of beads covered with chemicals that directly react (without a probe) with complementary non-sample chemicals mixed with the reagent to produce a fluorescent signal when illuminated by a laser. Readouts from staining controls can indicate the conditions for an efficient staining process step. During the extension process step, probes bind to complementary sequences (or DNA templates) and single nucleotides are added (single base extension) to the probes. The extension process probes have specially designed hairpin oligos that act both as the probe and the template (complementary DNA allele attached to the probe). During the extension process, extension process probes are extended at the 3' end using the probe strand itself acting as a template. Thus, extension control readouts can indicate conditions for efficient single base extension without requiring hybridized DNA samples. The target removal process strips off DNA templates attached to the probes after the extension reaction. In regular (or normal) probes, the extension of a single nucleotide occurs on the probe and then the DNA template attached to the probe is removed. For target removal process probes, the extension does not occur on the probe. Instead, a chemical mixed with the reagent attaches to the extension process probe and undergoes extension and labeling. If the target removal process is inefficient, these templates with extensions are not separated from the process probes, thus producing high intensity signals. During the hybridization process, DNA samples are attached to beads on the surface of an image-generating chip. Efficient completion of the hybridization process requires optimal temperature and other operating conditions. Hybridization process probes are designed to measure these operating conditions which are required for successful hybridization. Hybridization controls measure the temperature and other operating conditions required for hybridization without depending on sample quality. These probes react with non-sample chemicals mixed with a hybridization buffer reagent. The non-sample chemicals act as DNA templates and bind to hybridization process probes. Signal intensities from hybridization process probes are read to determine successful completion of the extension and staining process. Readouts from sample-independent process probes described above cannot be used to assess sample quality, which is the subject of the second type of process probes.

A second type of process probes is referred to as sample-dependent process probes that evaluate sample quality and assay performance. Examples of sample-dependent process probes include stringency controls, non-specific binding controls, and non-polymorphic controls. Stringency and non-polymorphic probes respond to human DNA samples while non-specific binding controls respond to non-human DNA in the samples. Stringency is defined as the degree of match between the probe sequence and the complementary DNA sequence that attaches to the probe. For example, if all nucleotides in a 50-mer single-stranded probe sequence match to corresponding nucleotides in the complementary DNA sequence then a 100% stringency is achieved. As the mismatches between the probe and complementary DNA sequence increase, stringency decreases. High stringency improves the quality of genotyping results. Stringency controls include two types of process probes. Perfect match (PM) controls include wild-type probes that are complementary to common sequence (or wild-type allele) in human DNA in the sample. Perfect match process probes produce high signal intensities in a good quality human DNA sample. As nucleotide sequences in perfect match probes match the complementary DNA sequence in the sample, this results in high degrees of hybridization indicating a strong bond. These bonds are less likely to be broken during the washing step following the hybridization. Mismatch controls have probe sequences that are designed to mismatch a certain number of nucleotides in complementary sequences in human DNA. For example, a mismatch process probe can have 20, 25, or 30 or more mismatched bases in a sequence of length 50. The mismatch control probes have lower stringency due to mismatched bases with sequences in the DNA sample. This results in a higher percentage of complementary sequences detached from the probes during the washing step. Thus, mismatch process probes produce low intensity signals in a good quality human DNA sample. Perfect match and mismatch control results are presented together to analyze sample quality. Non-polymorphic controls test the overall performance of the genotyping process from amplification to detection. Non-polymorphic controls are designed to match corresponding non-polymorphic regions of human DNA. Such regions are known to have no single nucleotide variants, thus the expected nucleotide to extend the probe sequence is known. Separate non-polymorphic binding control probes are designed to test the extension of each of the four nucleotides in a DNA sample (A, T, C, G). Signal intensities from the four types of non-polymorphic controls are used to calculate a performance metric. The values of the performance metric for different samples can be compared to determine assay performance across these samples. Binding controls test the presence of non-human (or bacterial) DNA in the sample. Binding process probes are complementary to non-human DNA and do not respond to human DNA samples. High intensity signals are produced from these process probes if a sample is contaminated by non-human DNA.

The sample-dependent process probes cannot identify all types of sample errors. Readouts from sample-dependent process probes can appear normal even if the sample is degraded but it hybridizes to the image-generating chip. A sample can be considered degraded for various reasons, for example, the mixing of two or more samples on a section, input quantity of the sample less than a minimum threshold quantity, etc. Therefore, an independent rerun confidence score is needed to predict the likely success or failure of sample reevaluation reruns.

The technology disclosed can generate a retry or rerun success confidence score for the sample after one or more failed production runs. The technology disclosed provides readouts from the process probes and call rates from the one or more failed production runs as inputs to a trained random forest classifier (retry classifier). Other types of classifiers such as support vector machines (SVM), deep learning-based approaches, gradient boosted trees, logistic regression, K-nearest neighbor, decision trees, Naïve Bayes, perceptron, and convolutional neural networks can be applied. The technology disclosed can also calculate a costed rerun score by applying a cost function to the rerun confidence score from the classifier. In addition, the technology disclosed can compare the rerun success confidence score with a threshold to determine whether to conduct an additional sample evaluation run of the sample.

In an alternate implementation of the technology disclosed, image quality scores from good vs. bad or root cause failure analysis classifiers for section images can also be given as an input to the retry classifier. The details of generating image quality scores for section images are presented in United States Provisional Patent Application No. 62/968,950, entitled "Machine Learning-Based Root Cause Analysis of Process Cycle Images", filed on January 31, 2020. Two distinct approaches to extract features from section images are described in the referenced application. One approach uses Principal components analysis (PCA) of section images and the other approach uses thresholding by areas. Outputs from these techniques are given as inputs to classifiers. Classifiers applied can include random forest, K-nearest neighbors (KNN), multinomial logistic regression, support vector machines (SVM), gradient boosted trees, Naive Bayes, etc.

## Environment

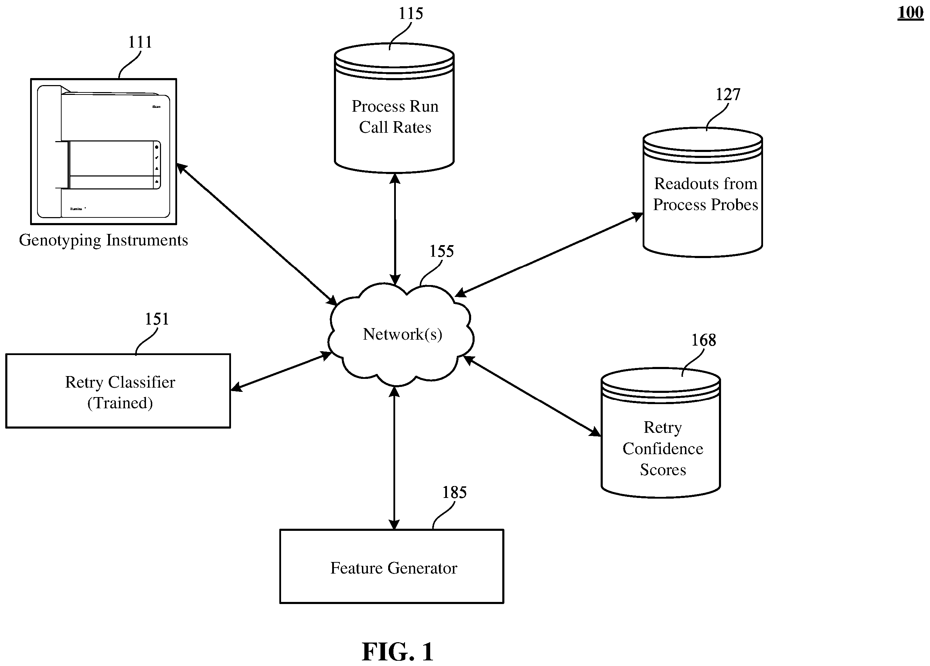

We describe a system for predicting the success of sample reevaluation by rerunning the genotyping production process. Genotyping is the process of determining differences in the genetic make-up (genotype) of an individual by examining the individual's DNA sequence using biological assays. Genotyping enables researchers to explore genetic variants such as single nucleotide polymorphisms (SNPs) and structural changes in DNA. The system is described with reference to **FIG. 1** showing an architectural-level schematic of a system in accordance with an implementation. Because **FIG. 1** is an architectural diagram, certain details are intentionally omitted to improve the clarity of the description. The discussion of **FIG. 1** is organized as follows. First, the elements of the figure are described, followed by their interconnection. Then, the use of the elements in the system is described in greater detail.

**FIG. 1** includes the system **100.** This paragraph names labeled parts of the system **100.** The figure illustrates genotyping instruments **111,** a process run call rates database **115,** readouts from a process probes database **127,** a retry classifier **151,** a retry confidence scores database **168,** a feature generator **185,** and a network(s) **155.**

The technology disclosed applies to a variety of genotyping instruments **111,** also referred to as genotyping scanners and genotyping platforms. The network(s) **155** couples the genotyping instruments **111,** the process run call rates database **115,** the readouts from the process probes database **127,** the retry classifier **151,** the retry confidence scores database **168,** and the feature generator **185,** in communication with one another.

The genotyping instruments can include Illumina's BeadChip imaging systems such as the ISCAN™ system. The instrument can detect fluorescence intensities of hundreds to millions of beads arranged in sections on mapped locations of image-generating chips. The genotyping instruments can include an instrument control computer that controls various aspects of the instrument, for example, laser control, precision mechanics control, detection of excitation signals, image registration, image extraction, and data output. The genotyping instruments can be used in a wide variety of physical environments and operated by technicians of varying skill levels. The sample preparation can take two to three days and can include manual and automated handling of samples.

The image-generating chips of genotyping instruments are equipped with internal process probes designed to support quality control of the genotyping process. A variety of process probes generate signals indicating the processing conditions and sample quality at different process steps of the genotyping process. Readouts from a first type of process probes indicate the operating conditions of different process steps of the production process independent of the sample quality. These process probes are referred to as sample-independent process probes. Examples of sample-independent process probes include staining controls, extension controls, target removal controls, and hybridization controls. Readouts from a second type of process probes indicate sample quality including whether the sample is contaminated with non-human DNA. These process probes are referred to as sample-dependent process probes. Examples of sample-dependent process probes include stringency controls, non-specific binding controls, and non-polymorphic controls. Both sample-independent and sample-dependent process probes are randomly positioned at known locations (or addresses) on the image-generating chips.

The readouts from process probes cannot be reliably used to decide whether to rerun a genotyping production run using the same sample used in an earlier inconclusive production process run. For example, sample contamination including the mixing of more than one sample on a section of the image-generating chip cannot be reliably predicted by sample-independent and sample-dependent controls. The technology disclosed includes a trained retry classifier 151 which is given readouts from process probes and call rates from inconclusive process runs as input. The classifiers generate a retry success confidence score indicative of whether a further sample evaluation run will produce a conclusive result. The technology disclosed reports the retry confidence scores to operators of the genotyping instruments to determine whether to conduct an additional sample evaluation run. Genotyping is an expensive process which can take up to three days to complete. Therefore, the technology disclosed can help reduce the production run costs by predicting when the sample reevaluation is likely to produce conclusive results.

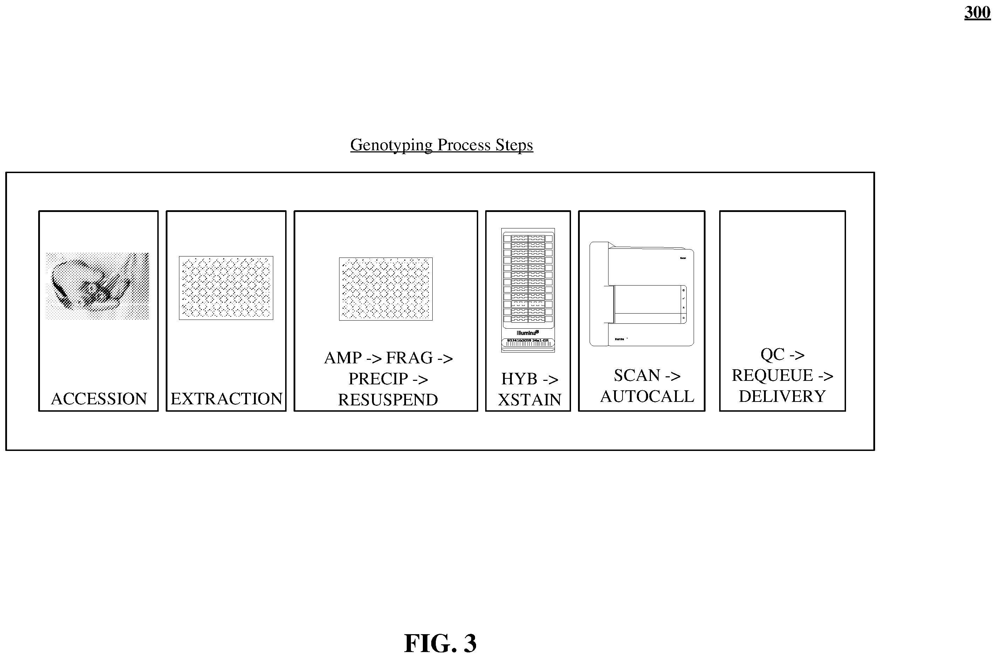

We illustrate process steps of an example genotyping process **300** in **FIG. 3.** This example genotyping process is referred to as Illumina's INFINIUM™ Assay Workflow. The process is designed to investigate many SNPs at extensive levels of loci multiplexing. Using a single bead type and dual-color (such as red and green) channel approach, the process scales genotyping from hundreds to millions of SNPs per sample. The process starts with the accession and extraction of DNA samples. The process can operate with a relatively low input sample such as 200 ng which can assay millions of SNP loci. The samples are amplified. The amplification process can take from a few hours to overnight to complete. The amplified sample undergoes controlled enzymatic fragmentation. This is followed by alcohol precipitation and resuspension. The image-generating chip is prepared for hybridization in a capillary flow-through chamber. The samples are then applied to prepared image-generating chips and incubated overnight. During this overnight hybridization, the samples anneal to locus-specific 50-mers covalently linked to up to hundreds of thousands of bead types. One bead type corresponds to each allele per SNP locus. The allelic specificity is conferred by enzymatic base extension followed by fluorescent staining. The genotyping instrument or scanner (such as ISCAN™ system) detects the fluorescence intensities of the beads and performs genotype calling.

In one example, the results of the genotyping are presented using a metric called "call rate". Call rate represents the percentage of single nucleotide polymorphisms (or SNPs) whose quality score (also referred to as GenCall score) is greater than a specified threshold. This metric represents the percentage of genotypes that were correctly scanned on the image-generating chip. A separate call rate is reported per section of the image-generating chip. A threshold can be used to accept or reject the results. For example, a call rate of 98% or more can be used to accept the genotyping results for a section. A different threshold value such as lower than 98% or higher than 98% can be used. If the call rate for a section is below the threshold, the genotyping process is considered as failed. The genotyping process can span over many days and is therefore expensive to repeat. Failures in the genotyping process can occur due to chemical processing errors or sample quality issues.

The genotyping systems can provide process readouts from process probes and call rates for sections of the image-generating chip upon completion of the genotyping process. The production run is considered inconclusive if the call rate is below a threshold value e.g., 98%. The technology disclosed can use the process probe readouts and call rates for section images for inconclusive production runs as inputs to a retry classifier to generate a retry success confidence score. The genotyping systems can also provide images of sections of the image-generating chip at the end of the production run. The images can be used to predict mechanical and reagent flow issues by examining the visual patterns in the section images. In one implementation, features extracted from section images are also given as input to the retry classifier. In another implementation, the technology disclosed can determine a costed retry confidence score by applying a cost function to the retry confidence score generated by the retry classifier. The confidence score can be evaluated against a threshold to determine whether to conduct a sample reevaluation run. The technology disclosed can be applied after one or more sample evaluation runs.

We now refer to **FIG. 1** to provide a description of the remaining components of the system **100**. The process run call rates can be stored in the database **115**. Readouts from process probes can be stored in the database **127**. The readouts from process probes are signal intensities. Intensities of some process probes are monitored in one color (green or red) channel. Some process steps of the genotyping process require evaluation in both the red and green channels. Process probes for these process steps are provided in both red and green color channels. The absolute signal intensity values observed from process probes can be different across different samples. Therefore, intensities from process probes relative to the background are used for input to the classifiers.

The feature generator **185** can be used to organize signal intensities from process probes for input to the retry classifier **151**. Different types of process probes are randomly positioned on sections of image-generating chips at known locations. At the end of the genotyping process run, the readouts from the process probes can be stored in the database **127**. In one implementation, the feature generator **185** can access the intensities of different types of process probes in the database **127** and calculate an average intensity for each process probe type on a section image. The average signal intensities for each type of process probe can then be given as input to the retry classifier **151** to generate a retry success confidence score. The technology disclosed can apply a trained random forest classifier to generate the retry confidence score for an inconclusive sample evaluation run. The retry confidence scores can be stored in the retry confidence score database **168** for reporting to an operator. A cost function can be applied to the retry confidence score to generate a costed retry score. The confidence score can be compared with a threshold for determining whether to conduct an additional sample reevaluation run of the sample.

Completing the description of **FIG. 1,** the components of the system **100,** described above, are all coupled in communication with the network(s) **155**. The actual communication path can be point-to-point over public and/or private networks. The communications can occur over a variety of networks, e.g., private networks, VPN, MPLS circuit, or Internet, and can use appropriate application programming interfaces (APIs) and data interchange formats, e.g., Representational State Transfer (REST), JavaScript Object Notation (JSON), Extensible Markup Language (XML), Simple Object Access Protocol (SOAP), Java Message Service (JMS), and/or Java Platform Module System. All of the communications can be encrypted. The communication is generally over a network such as the LAN (local area network), WAN (wide area network), telephone network (Public Switched Telephone Network (PSTN), Session Initiation Protocol (SIP), wireless network, point-to-point network, star network, token ring network, hub network, and Internet, inclusive of the mobile Internet, via protocols such as EDGE, 3G, 4G LTE, Wi-Fi and WiMAX. The engines or system components of **FIG. 1** are implemented by software running on varying types of computing devices. Example devices are a workstation, a server, a computing cluster, a blade server, and a server farm. Additionally, a variety of authorization and authentication techniques, such as username/password, Open Authorization (OAuth), Kerberos, Secured, digital certificates and more, can be used to secure the communications.

## Feature Generator - System Components

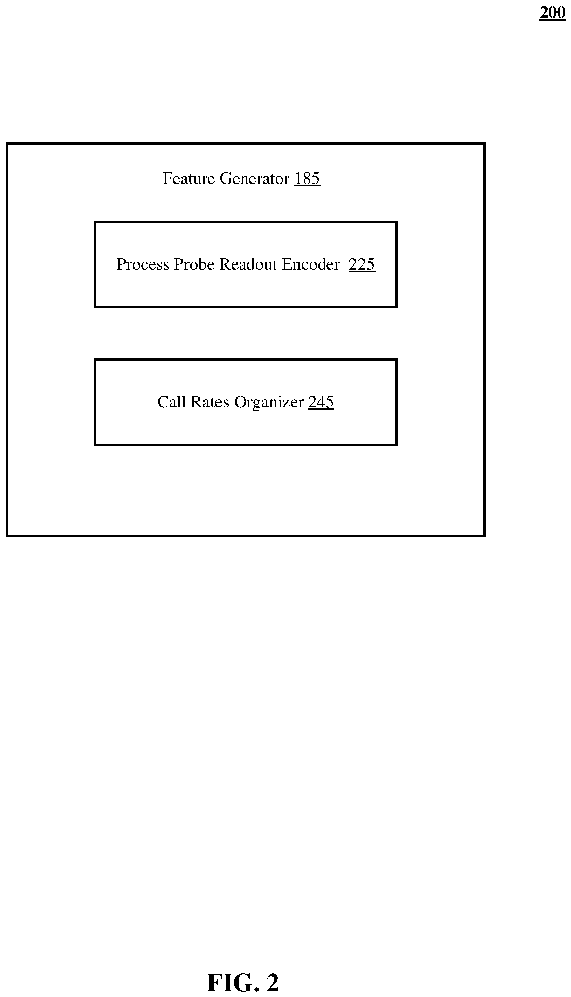

**FIG. 2** is a high-level block diagram **200** of components of the feature generator **185**. The feature generator **185** comprises of two sub-components that organize feature data for input to machine learning models. We present details of the components in the following sections.

### *Process probe Readout Encoder*

A process probe readout encoder 225 encodes the readouts from process probes for input to machine learning algorithms. The process probes report signal intensities at high intensities, low intensities, and background level intensities. Signal intensity emitted from a process probe is subject to variations in DNA sample preparation methods, sources of a sample, or tissue type. Signal intensities can also vary because of variability in which individuals perform the assay. Variations in genotyping instruments or scanners can also impact signal intensities emitted by process probes. Because of these variations, the readouts from process probes should not be assessed based on absolute values. The process probe readout encoder can calculate relative intensities of signals to the background for effective evaluation of readouts. The system can also include preprocessing of signal intensities from process probes. The process probe readout encoder can also calculate average intensities of readouts from process probes. Average values of signal intensities from process probes can be provided as readout inputs to the machine learning algorithm.

### *Call Rates Organizer*

A call rate organizer **245** can organize call rate per section from a production run to provide the call rate as input to the machine learning algorithm along with process probe readouts from the same section. A call rate represents the percentage of single nucleotide polymorphisms (or SNPs) whose quality score (also referred to as GenCall score) is greater than a specified threshold.

## Process Cycle Images

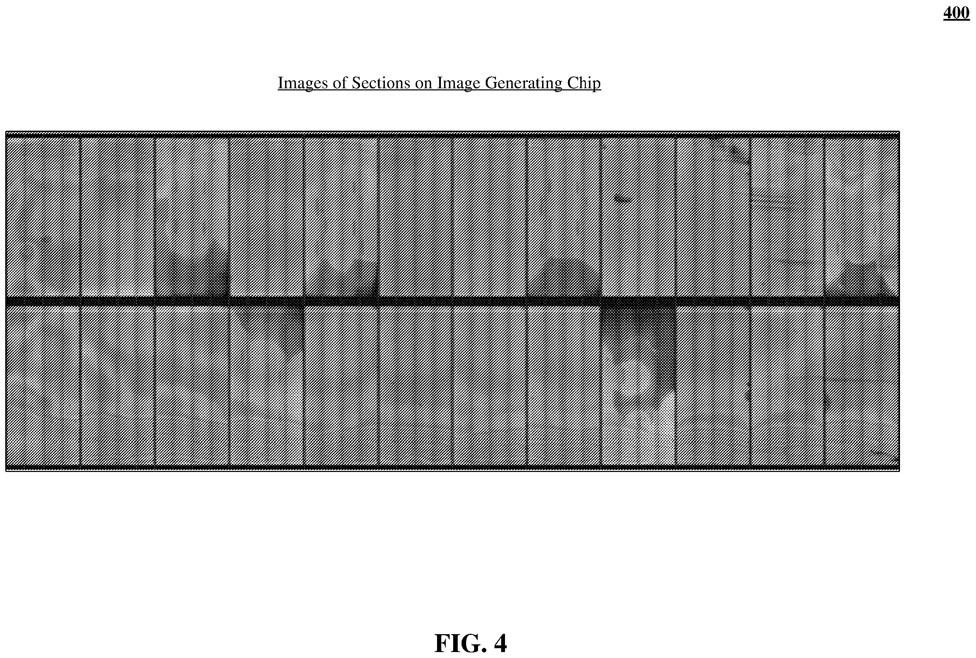

We now present examples of production images of sections on image-generating chips. **FIG. 4** is an illustration **400** of production images of 24 sections on an image-generating chip. The sections are arranged in twelve rows and two columns. Each section has four slots. The illustration **400** shows section images of a successful production cycle. Image-generating chips with other configurations of sections can also be used such as including 48, 96 or more sections. The production process is vulnerable to both chemical processing and sample errors. Chemical processing errors can be caused by issues in processing conditions, or the quality of reagents. Sample related issues can include quality or quantity of samples, contamination of samples with non-human DNA or mixing of more than one sample on a section of the image-generating chip. The genotyping instruments can provide grayscale images of the sections of the image-generating chip at the end of a production run. The images can be used to predict mechanical or chemical processing issues by examining the visual patterns in the section images.

Mechanical or operational issues can also impact the quality of results in a genotyping process in addition to chemical processing and sample errors. The majority of section images from a genotyping instrument are successful. The failed section images are understood to fit in five categories plus a residual failure category. The five failure categories are hybridization or hyb failures, spacer shift failures, offset failures, surface abrasion failures and reagent flow failures. The space shift failures, offset failures, surface abrasion failures, and reagent flow failures can be considered as mechanical or operations issues. Hybridization (hyb) failures can be caused by chemical processing issues due to reagent quality or sample issues. In addition, a sixth category of failed images is called a residual category which includes unhealthy patterns due to mixed effects, unidentified causes and weak signals. The details of image failure categories listed above including their possible causes are presented in United States Provisional Patent Application No. 62/968,950, entitled "Machine Learning-Based Root Cause Analysis of Process Cycle Images", filed on January 31, 2020. The referenced application discloses a technology in which features extracted from section images are provided to two types of image classifiers: a good vs bad classifier and a root cause analysis classifier. Classifiers applied can include random forest, K-nearest neighbors (KNN), multinomial logistic regression, support vector machines (SVM), gradient boosted trees, Naive Bayes, etc. As larger bodies of labeled images become available, convolutional neural networks such as ImageNet could also be used. The output from the good

vs bad classifier or the root cause classifier is a confidence score indicating image quality. The confidence score can be compared with a threshold to classify good images from bad images. For example, in one embodiment, a threshold of 80% is used. It is understood that different values of the threshold can be used. In one implementation of the technology disclosed, the image quality from good vs bad and root cause classifiers disclosed in the above reference can be given as an additional input to the retry classifier **151.**

## Image-Generating Chips

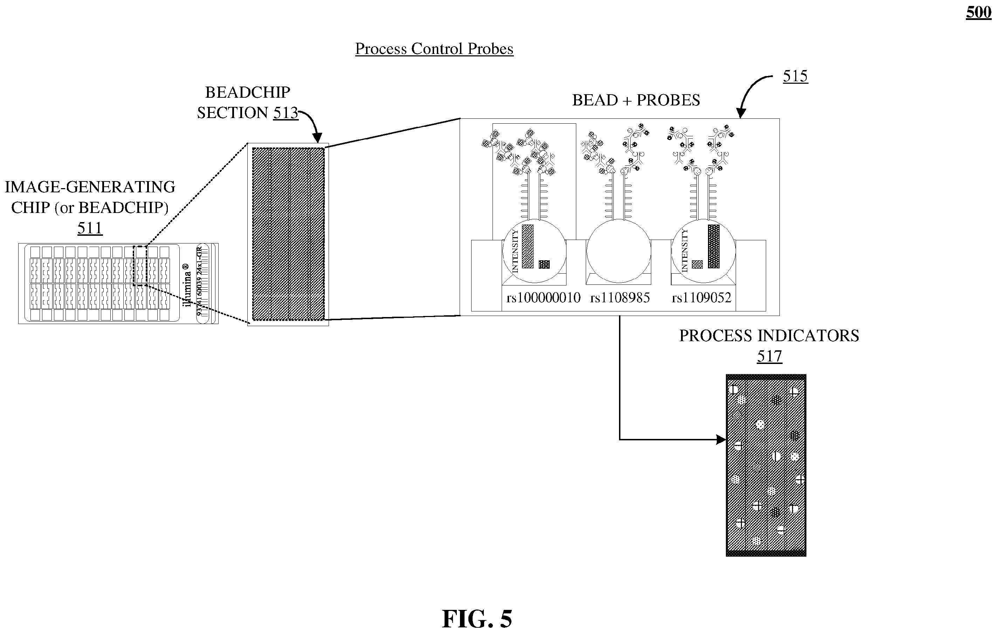

**FIG. 5** presents an illustration **500** indicating process control probes (or process probes) positioned on an image-generating chip. The genotyping instruments **111** can contain image-generating chips which are also referred to as BeadChips or Bead Arrays. An example image-generating chip **511** is shown in **FIG. 5.** An image-generating chip comprises multiple sections arranged in columns and rows on the image-generating chip. An example section **513** is shown in **FIG. 5.** An image-generating chip can have 12, 24, 96 or more sections, each of which can have a separate DNA sample. Millions of (glass) beads are positioned in wells or holes at known locations on the surface of an image-generating chip. Thousands or more different types of oligonucleotide probes are attached to the beads at random known locations and replicated many times (up to 10 times or more) on the image-generating chip. The high degree of replication makes it possible to get robust measurements for each type of probe even in the presence of spatial artefact or imaging errors on portions of the image-generating chip. The illustration **515** shows three probes on the surface of the image-generating chip. Different probes are designed to target specific positions in the complementary DNA sequences (also referred to as DNA templates). Probes can be of different lengths, e.g., a probe containing a sequence of 50 bases (or nucleotides) is referred to as a 50-mer.

## Process probes

The image-generating chips are also equipped with many process probes of different types positioned at random known locations. We show these process probes as dots with different patterns in **FIG. 5** as an illustration **517.** Readouts from process probes can indicate process conditions of different processing steps during a production run or indicate the quality of a sample. The readouts from process probes can help in quality control of the genotyping process. Individual controls can be informative of certain steps in the assay workflow, but they can be affected by the performance of other steps in the workflow. Signal intensities from process probes are read out during the imaging process along with intensities of regular (or normal) probes. Intensities of some process probes are reported in both green and red channels while intensities of some process probes are reported in either the green or red channel. Absolute intensity levels of signals from process probes can vary across genotyping projects due to variations in sample preparation, operators who perform the assay and genotyping instruments. Therefore, the intensities of signals relative to the intensity of background signals are evaluated.

## Grouping of Process probes based on Response to Sample

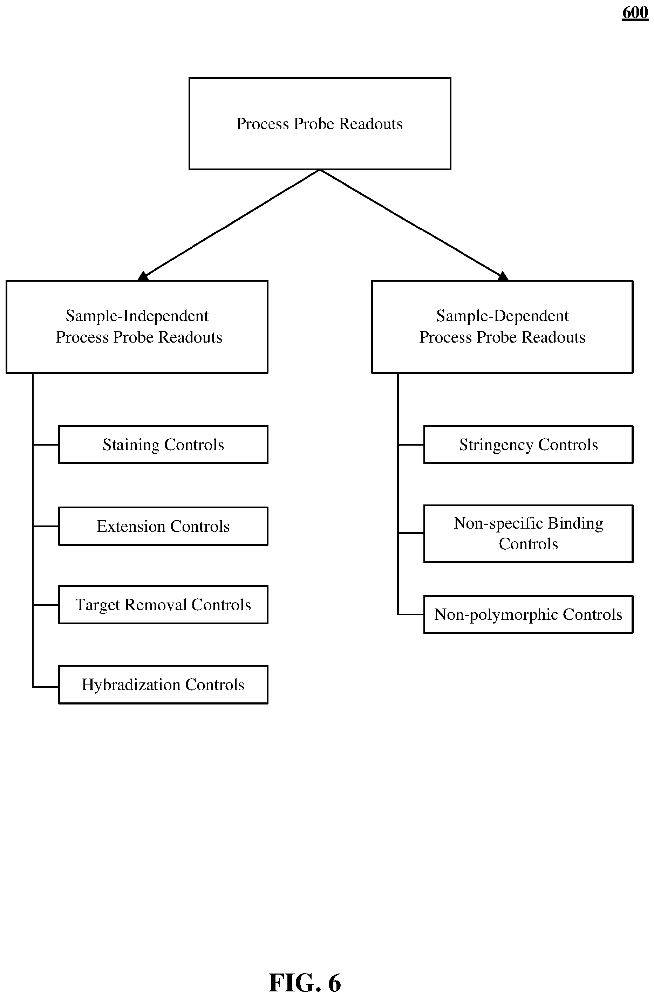

Process probes can be grouped based on their response to a sample during the genotyping process. Some process probes do not respond to a sample but measure the conditions at different steps of the genotyping process and reagent performance. This type of process probes is referred to as sample-independent process probes. Examples of sample-independent process probes include staining controls, extension controls, target removal controls, and hybridization controls. Process probes that react with a sample can assess sample quality and are grouped as sample-dependent process probes. Examples of sample-dependent process probes include stringency controls, non-specific binding controls, and non-polymorphic controls. **FIG. 6** presents a grouping **600** of process probes into sample-independent and sample-independent process probes. In the following sections, we present further details of the process probes.

## Sample-Independent Process probes

Sample-independent process probes respond to chemicals mixed with reagents and generate signals indicative of the delivery of reagents and process conditions. Therefore, signal intensities are emitted from sample-independent probes. In the following sections, we present details of sample-independent process probes.

## Staining Process probes

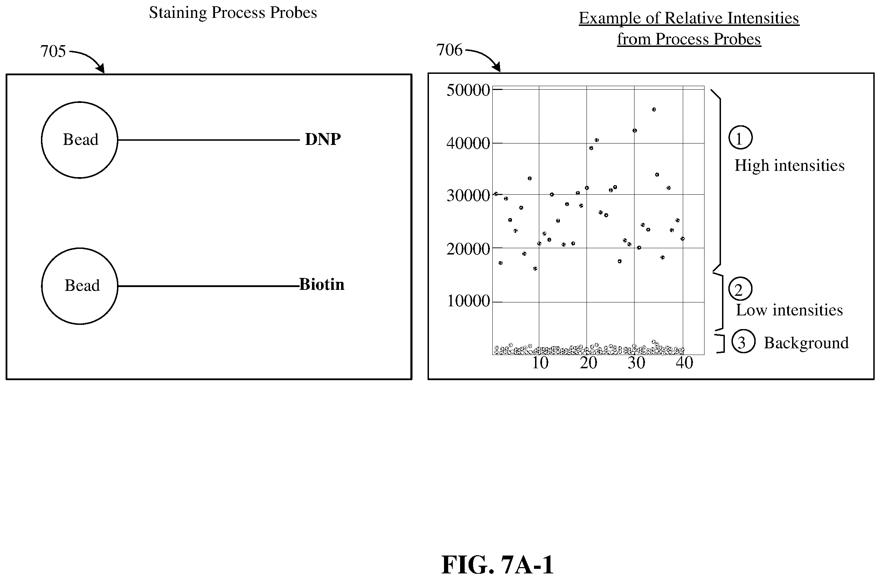

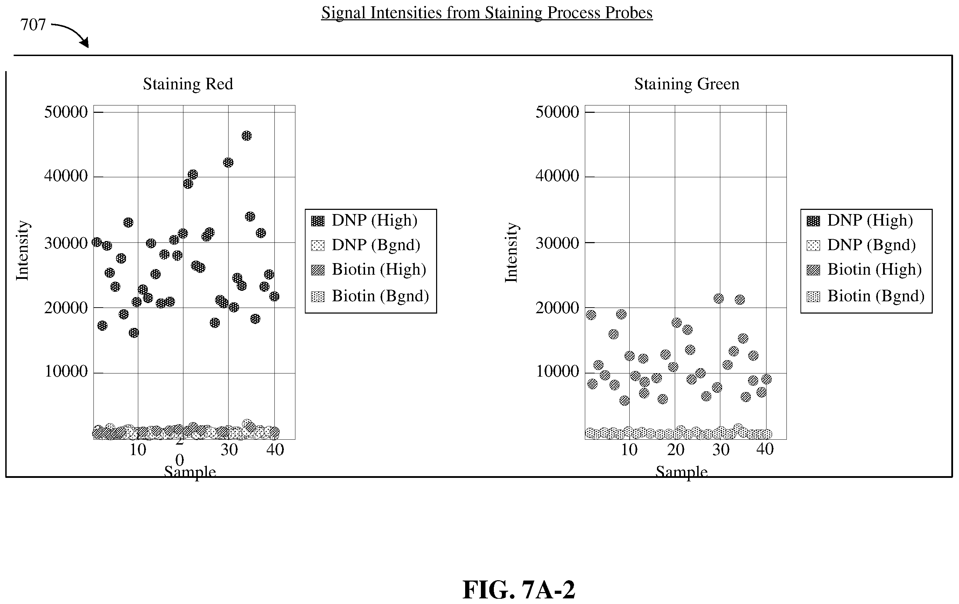

Readouts from staining controls can indicate the efficiency of the extension and the staining process steps. During the staining process fluorescent labels are attached to single base extensions to probes attached to beads. The staining process probes do not respond to the sample, therefore, the signal intensity emitted from these process probes is independent of the hybridization of the sample, single base extensions and attachment of fluorescent labels. Staining control probes are shown in **FIG. 7A-1** by a label **705.**

Staining controls consist of beads covered with high levels or small (background) levels of dinitrophenyl (DNP) or biotin and are directly labeled in successive rounds of adding green fluorescent streptavidin and red fluorescent anti-DNP antibody. Because DNP and biotin are directly attached (or bind) to the beads, staining controls do not depend on sample DNA hybridized to the image-generating chip and also do not require single base extension.

Staining controls are used to examine the efficiency of the staining process in both red and green channels. The green channel shows a higher signal for biotin staining when compared to the biotin background, whereas the red channel shows a higher signal for DNP staining when compared to the DNP background. The readout for the green channel is "biotin high value" divided by "biotin background value". The readout for the red channel is "DNP high value" divided by "DNP background value". Alternatively, absolute intensity values could be used, instead of intensity ratios.

An example of intensities for red and green channels is shown in an illustration 707 in **FIG. 7A-2.** In the 'staining red' plot on the left, strong positive signals are expected for the dinitrophenyl (DNP) (High) data points, while background-level signals are expected for the DNP (Bgnd), Biotin (High), and Biotin (Bgnd) signals. In the 'staining green' plot on the right, strong positive signals are expected for the Biotin (High) data points, while background-level signals are expected for the DNP (High), DNP (Bgnd), and Biotin (Bgnd) data points.

Note that intensity values reported for a genotyping project may be different across different genotyping projects. This is due to variations in DNA preparation methods, the source of a sample and tissue types. Variations between genotyping scanners and individual operators can also cause different absolute intensity values across different genotyping projects. One approach to correcting for these variations is to assess performance based on relative intensities of signals to background and/or staining process control intensity readouts. The illustration **706** in **FIG. 7A-1,** illustrates high, low and background level signal intensities in three bands 1, 2, and 3, respectively. The data points in the band 1 are considered to have high signal intensities, the data points in the band 2 are considered to have low signal intensities and the data points in the band 3 (at the bottom of the plot) have background-level signal intensities. The absolute values of high, low and background-level data points will differ across different process evaluation runs. When evaluating the data from a sample evaluation run, the data points can be assigned to high, low, or background-level signal intensities using the relative intensities of data from the current evaluation run.

### Extension Process probes

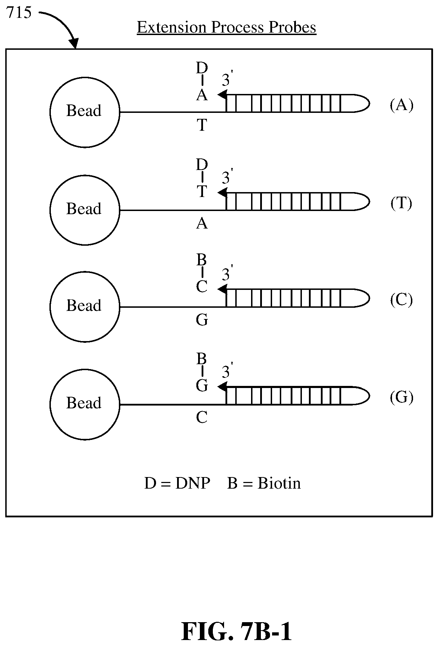

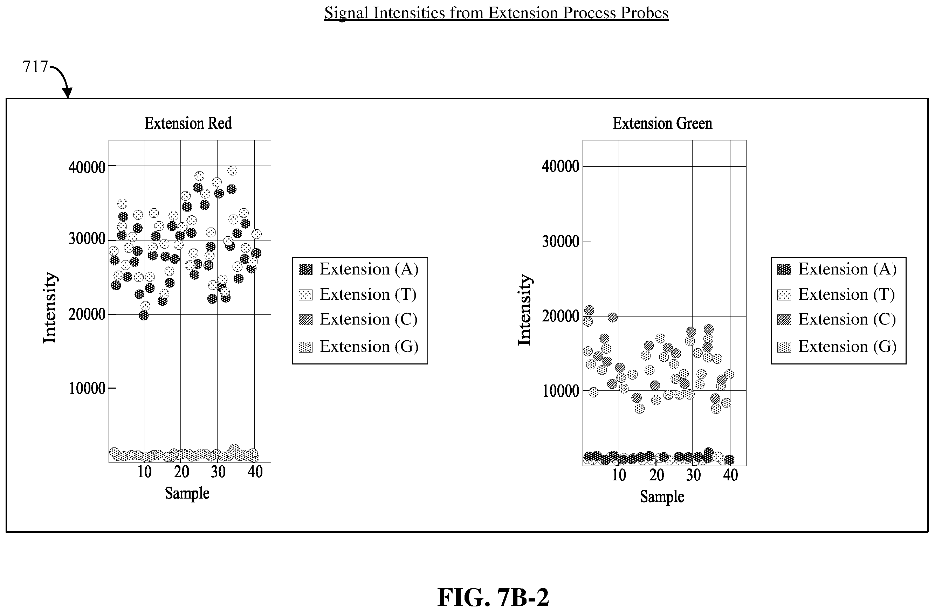

Extension controls indicate the conditions for an efficient single base extension process step without chemical reaction to sample DNA. During the extension process, the regular probes are extended by a single base on the 3' end. The single base extension depends on the nucleotide in the DNA strand covalently linked to the probe. As there are four possible nucleotides (A, C, G, T) that can extend the probe sequence, a separate extension process probe is designed for each nucleotide as shown in an illustration **715** in **FIG. 7B-1.** As shown in the figure, extension process probes have specially designed hairpin oligos that function both as the probe and the template (complementary sequence attached to the probe). The probe is extended at the 3' end using the probe strand itself as the DNA template. Extension controls, therefore, do not depend on DNA samples for hybridization to test conditions for successful single base extension and staining.

Readouts from extension process probes are monitored in both red (extension with A or T nucleotides) and green (extension with C or G nucleotides) channels. In the green channel the lowest intensity for C or G nucleotides is always greater than the highest intensity for A or T nucleotides. A metric provided from the readouts is calculated as "lowest of the C or G intensity" divided by "highest of the A or T intensity" for a single sample. A graphical plot **717** in **FIG. 7B-2** illustrates an example of signal intensities from extension process probes. The 'extension red' plot on the left shows that strong positive signals are expected for extension (A) and extension (T) data points, while background-level signal intensities are expected for extension (C) and extension (G) data points. The 'extension green' plot on the right shows that strong positive signals are expected for extension (C) and extension (G) data points, while background-level signal intensities are expected for extension (A) and extension (T) data points.

### Target Removal Process probes

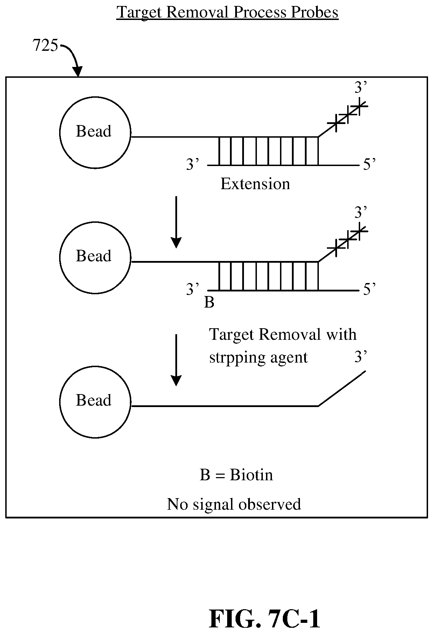

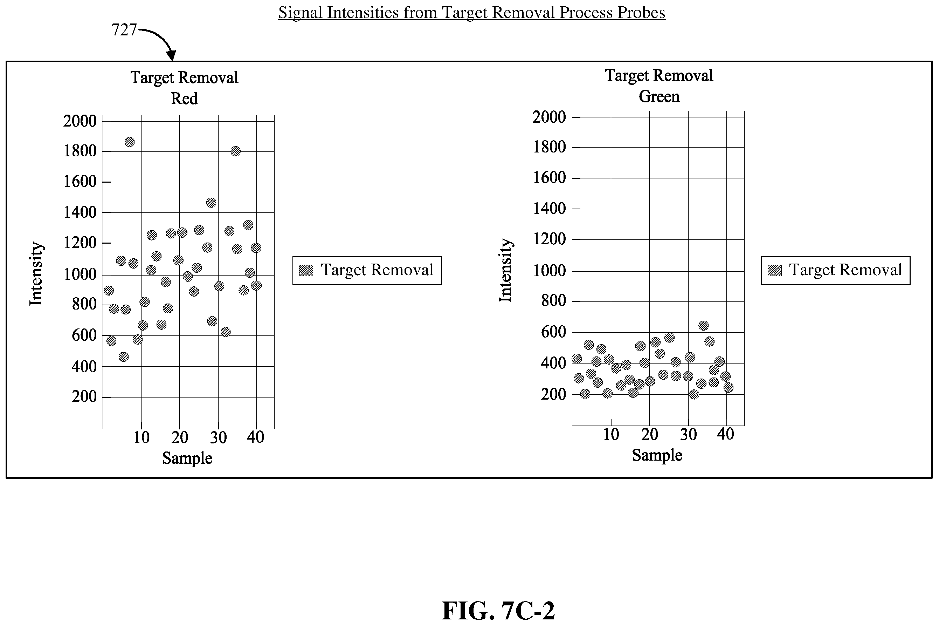

Target removal process probes indicate the conditions of the target removal process without requiring sample DNA templates covalently linked to the probes. The DNA templates attached to the probes are removed after the single base extension process. This process is also referred to as target removal. The probe sequence is designed such that single base extension does not occur at the 3' end of the probe. An example target removal process probe is shown in an illustration **725** in **FIG. 7C-1.** The probe attached to the bead does not extend. As opposed to other types of probes which covalently bind to DNA templates or synthetic templates mixed with a reagent, the target process probe itself acts as a (DNA) template. The control oligos mixed with a hybridization buffer covalently link with the target removal process probes and extend at the 3' ends. The 3' ends of the control oligos are attached to the ends of process probes close to the beads as shown in an illustration **725.** Following this, the staining process labels the control oligos that are covalently linked to process probes.

During the target removal process, the labeled control oligos (or targets) are removed from the process probes. Therefore, target removal controls emit low (or background level) signals as compared to hybridization controls indicating that the targets (control oligos) were removed after extension. Target removal controls are monitored in the red channel. If the target removal process is not efficient, the target removal controls will emit high intensity signals compared to background levels. The background intensity for the same DNA sample is close to or larger than the intensity of the signals from target process probes. A metric can be calculated as "background intensity" divided by "control intensity". A graphical plot **727** in **FIG. 7C-2** illustrates examples of intensity values observed in red and green channels. The 'target removal red' plot on the left with intensity values significantly above background level in the red channel is indicative of an inefficient target removal process. Signal intensity in the green channel is expected to be at the background level. The graphical plot labeled 'target removal green' illustrates signal intensities in the green channel at the background level.

## Hybridization Process probes

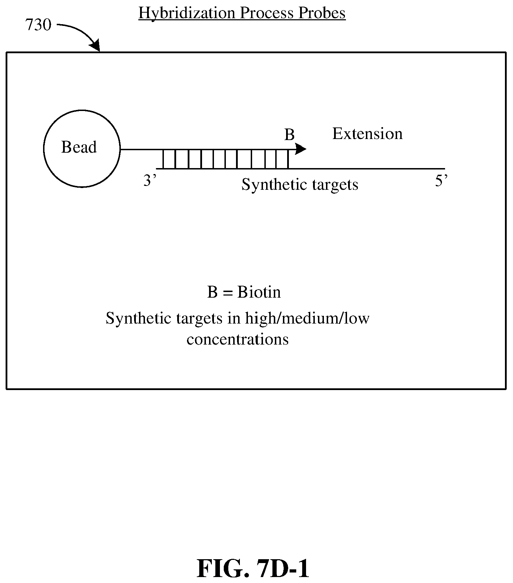

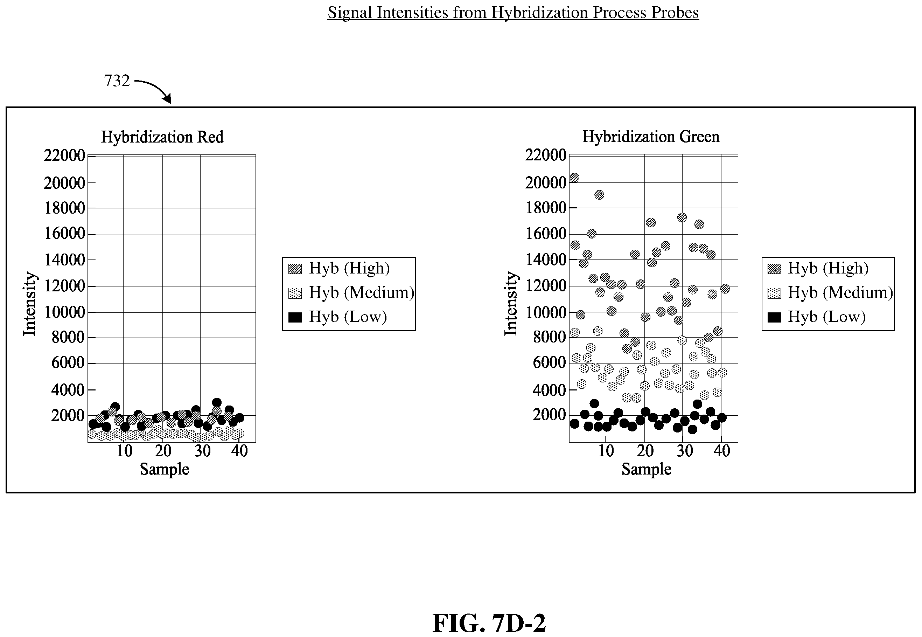

Hybridization process probes test the assay performance using synthetic templates (or targets) mixed in the reagent (also referred to as the hybridization buffer or RA1 buffer) instead of DNA sequences in the sample. Hybridization includes the application of DNA samples to image-generating chips and overnight incubation. During this overnight incubation, the samples anneal to locus-specific 50-mers covalently linked to up to hundreds of thousands of bead types. The synthetic templates bind to complementary hybridization process probes that are attached to beads. Following the binding of templates with process probes, single base extensions occur on the probes. An example hybridization process probe is shown in the illustration **730** in **FIG. 7D-1.** The synthetic templates can be mixed in the reagent at three levels, monitoring the response from a high concentration (5 pM), medium concentration (1 pM) and low concentration (0.2 pM). Hybridization controls appear in the green channel as signals with various intensities corresponding to intensities at which synthetic templates are mixed in the reagent.

Hybridization controls are monitored in the green channel. Data points at three different intensity levels are expected (low, medium, and high intensities). A distinct separation of data points at three intensity levels is expected in the sample. The readouts from hybridization controls can indicate conditions for the stringency (strength of covalently linked DNA templates to probes) and washing step after single base extension. Results can also indicate expected conditions for the single base extension and staining steps that follow the hybridization and washing steps. A graphical plot **732** in **FIG. 7D-2** illustrates signal intensities from hybridization process probes in red and green channels. The 'hybridization red' plot on the left shows the signal intensities to be around the background level which is expected when conditions for hybridization are good. The 'hybridization green' plot on the right shows data points at three different intensity levels (high, medium, and low) as expected in the green channel.

## Sample-Dependent Process probes

In the following sections, we present details of sample-dependent process probes.

## Stringency Process probes

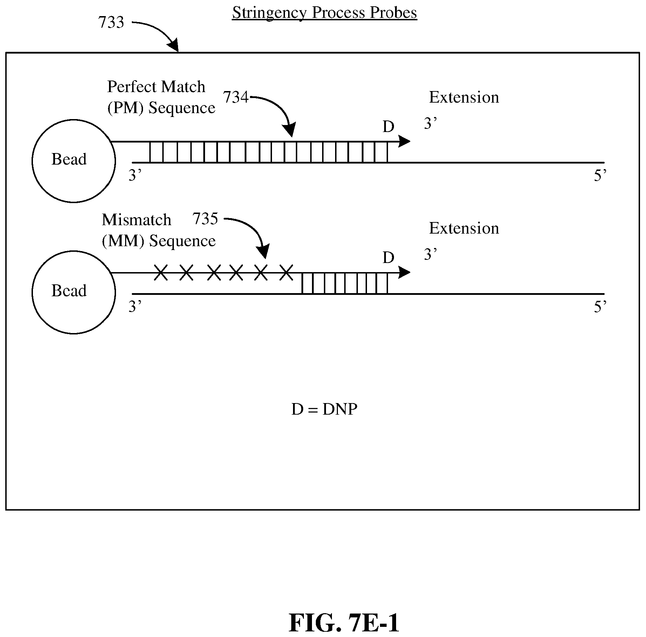

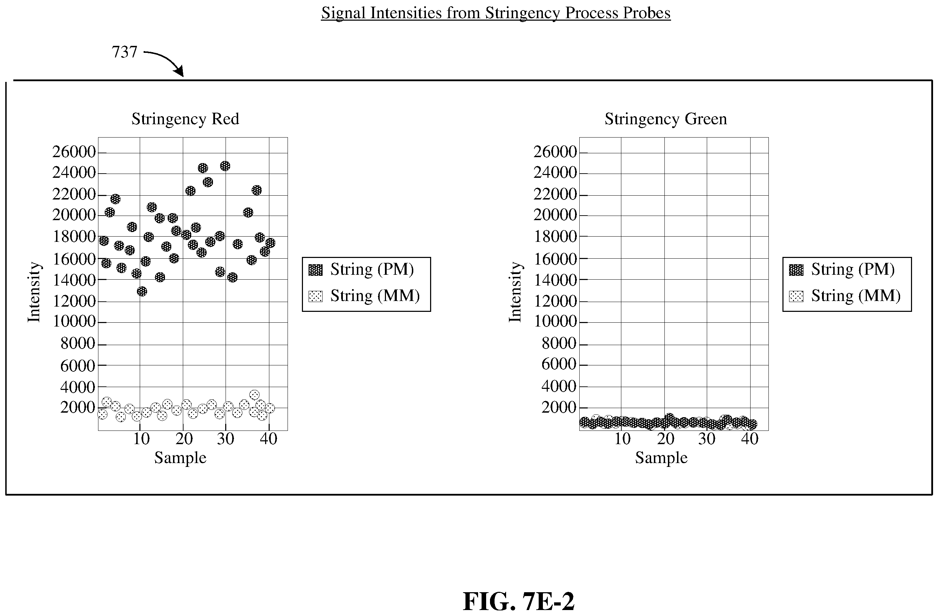

Stringency is a term used in chemical processes that include hybridization to represent the degree of match between the probe and the complementary DNA sequence that binds to the probe. For example, maximum stringency is achieved if all the bases in a 50-mer probe sequence covalently bind with respective bases in the DNA template. This results in a strong binding between the process probe and complementary DNA sequence, creating a stable dimer under reaction conditions. Operating temperature and composition of the hybridization buffer can impact the level of stringency achieved during hybridization. Higher stringency results in a strong bond between the probe and the DNA template. Hybridization is followed by a washing step in which non-specifically bound DNA templates are removed. During the washing step, DNA templates that have low stringency can be detached from probes. Stringency process probes respond to DNA templates in the sample. Stringency process probes are shown in an illustration **733** in **FIG. 7E-1.** There are two types of stringency process probes: perfect match (PM) probes **734** and mismatch (MM) probes **735.**

Perfect match process probes include probes with wild-type (common) sequences in human DNA that respond to complementary wild-type alleles (common) in a human DNA sample. In a perfect match process probe, all or a majority of the bases in the probe sequence (50-mer) covalently bind with respective bases in the DNA template resulting in a stable dimer under reaction conditions. As shown in the illustration **734,** the 3' end of the perfect match probe is extended producing high intensity signals indicative of good sample composition and/or binding conditions. The high intensity signals are a consequence of successful extension and staining processes of perfect match probes because the DNA template remains attached to the probe during the washing process due to high stringency.

Mismatch controls include probes that have many mismatched nucleotide bases to the complementary human DNA sample. Due to mismatch, covalent bonds between corresponding bases in the probe and DNA template are not formed. The mismatched locations are shown as crosses on the mismatch probe **735** in **FIG. 7E-1.** Mismatch at many nucleotide positions in the 50-mer sequence results in lower stringency between the probe and the DNA template. Mismatch process probes bind weakly to the complementary DNA sequence as compared to perfect match controls because there are fewer bases in the sequence that anneal to the target. The mismatches are located towards the 5' end of the probe sequence. This weaker binding results in a higher off rate for the mismatch probes attached to the bead. This results in significantly fewer extensions at the 3' end resulting in background-level signal intensities. It is also expected that many of the DNA templates will be detached from mismatch DNA probes during the washing process. This results in no extension and staining of the probes on these controls. Consequently, mismatch probes are expected to yield signals of much lower intensity (or background-level intensities) when sample composition and binding conditions are good.

Readouts from perfect match and mismatch controls are presented together for a sample to indicate a good sample composition and binding conditions. Stringency controls are monitored in the red channel. Strong intensity is expected for perfect match process probes and low (background) level intensity signals are expected for mismatch process probes. Stringency controls respond to human DNA, therefore signal intensities at background levels are expected when exposed to non-human DNA. Example signal intensities from stringency process probes are presented in graphical plots **737** in **FIG. 7E-2.** In the graphical plot labeled as 'stringency red' (on the left) strong positive signals (high intensity values) are expected for perfect match 'String (PM)' data points while background-level intensities are expected for mismatch 'String (MM)' data points. The stringency process control probes are monitored in the red channel, therefore, signal intensities in the green channel for perfect match and mismatch data points are expected to be at background levels as shown in the 'stringency green' graphical plot (on the right).

## Non-polymorphic Process probes

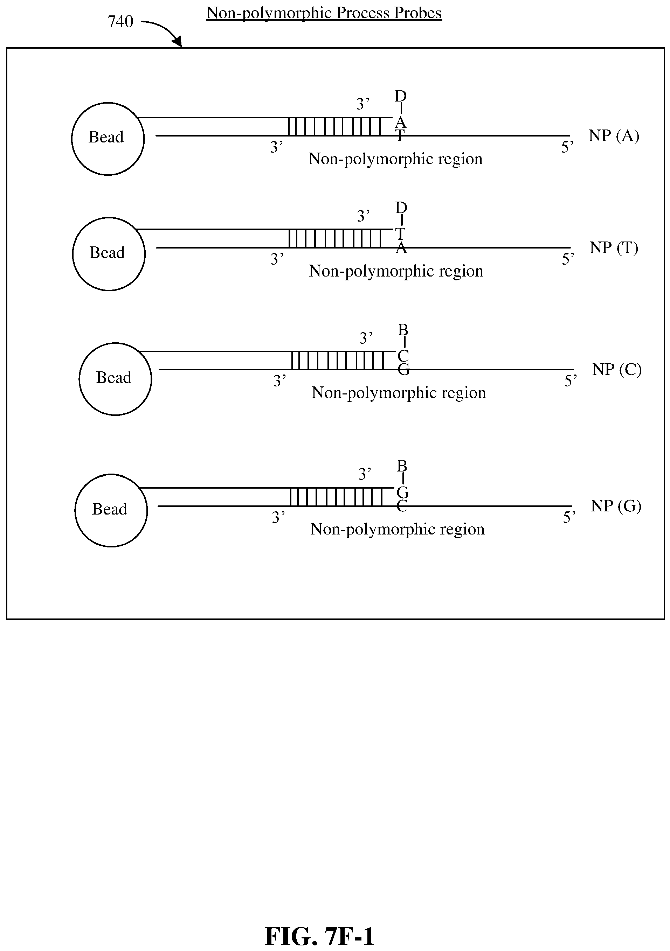

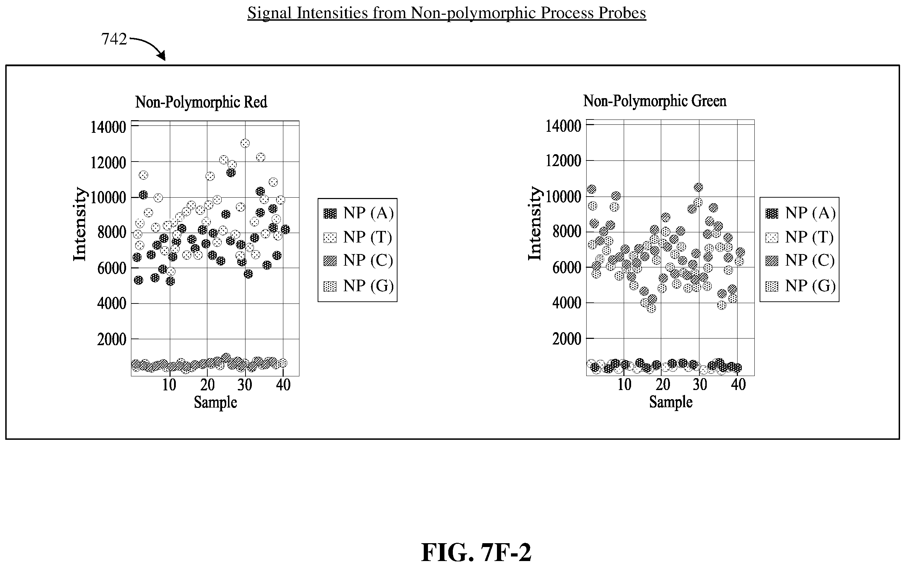

Non-polymorphic controls include probes with engineered single base extensions that respond to alternative bases in a complementary non-polymorphic region of the human DNA. One non-polymorphic process probe is designed for each of the four nucleotides A, C, G, T. The four example probes are shown in an illustration **740** in **FIG. 7F-1.** Process probes for A and T nucleotides are monitored in the red channel and process probes for C and G nucleotides are monitored in the green channel. Non-polymorphic controls test the overall performance of the assay, from amplifications to detection. Results from non-polymorphic controls can be used to compare assay performance across different samples. Non-polymorphic controls respond to human DNA, therefore signals at background levels are expected when non-human DNA is used.

We present examples of signal intensities from non-polymorphic process probes in an illustration **742** in **FIG. 7F-2.** In the red channel, strong positive signals are expected for the dark gray NP(A) and light gray NP (T) data points as shown in a graphical plot 'non-polymorphic red' (on the left). Background-level signal intensities are expected for the NP (C) and NP (G) data points as shown in the 'non-polymorphic red' graphical plot (on the left).

The NP (C) and NP (G) data points are illustrated in two medium shades of gray. In the green channel, strong positive signals are expected for the NP (C) and NP (G) data points, while background-level signals are expected for the NP (A) and NP (T) data points as shown in a 'non-polymorphic green' graphical plot (on the right).

## Non-specific Binding Process probes

Non-specific binding controls respond to DNA sequences not present in human DNA and produce signals indicative of contamination of the sample by non-human DNA. The probe sequences for non-specific binding controls are complementary to bacterial sequences and should not hybridize to human sequences under standard hybridization conditions. Binding of non-human DNA sequences to process probes can result in high signal intensity from these process probes. The process probes are monitored both in the green and the red channel and should show intensities at the background level in the presence of a good composition of a human DNA sample. While human samples are described herein, it is understood that sample-dependent probes can used for samples obtained from other species, including but not limited to cattle, sheep, pigs, cats, and other animals, and crops such as wheat, rice, and the like. Thus, where the term "human sample" is used herein, it will be understood that sample-dependent probes specific for genetic material for any one of a number of target organisms can be used in the methods described herein. Likewise, it will be understood that the term "non-human contamination" can more broadly refer to genetic material that is from an organism other than the target species.

We present examples of signal intensities from non-specific binding controls in a graphical plot **750** in **FIG. 7G.** The probe sequences for non-specific binding controls are complementary to bacterial sequences and signal intensities at a background level are expected under standard hybridization conditions. Signals from non-specific binding controls are observed in both red and green channels. Note the difference in the scales of the vertical axes in the plots **750** and the graphical plots **737** for stringency probes and the graphical plots **742** for non-polymorphic process probes. Compared to intensity levels of positive signals from stringency and non-polymorphic process probes, the intensity levels of data points for non-specific binding controls are expected to be at the background levels.

## Grouping of Process probes based on Stages of Genotyping Probes

The process probes presented above alternatively can be grouped according to the process stage during which they respond to reagents or a sample. **FIG. 8** presents an illustration in which process steps of the genotyping process are shown along with process probes that respond to reagents or samples in respective process steps. The process steps depicted take three days to complete. The genotyping process can be divided in three stages as shown in an illustration **800,** based on the grouping of related process tasks. During stage 1, the sample is in liquid form and not attached to beads on the image-generating chip. Stage 2 comprises of process tasks that are carried out when the sample is hybridized to the image-generating chip. Tasks in stages 1 and 2 are the preparation of the sample for single base extension and labeling which are the core activities in the genotyping process. Stage 3 comprises of tasks related to these core activities and includes the collection of images as a final process step.

We now briefly present the tasks and process probes to monitor these in three stages of the production run. The tasks are labeled by numbers 1 to 10 and process probes are labeled by alphabet letters A to H on **FIG. 8.** The more detailed descriptions of individual process probes, above, are, in the interest of brevity, not repeated. **FIG. 8** also maps process control probes to sample-dependent and sample-independent groups as indicated by two different hatching patterns.

### Stage 1 - Pre-Hybridization

This stage can include three tasks performed before the hybridization of a sample to the image-generating chip in the following stage. The first task (labeled 1) is sample quantification and qualification. The sample and any reagents added to the sample are dispensed at the volume set by the protocol. Sample quantification needs to be accurate to achieve good results from the production run. The non-specific binding process probes (labeled A), respond to sample DNA during this process step. Non-polymorphic process probes (labeled B) also respond to sample DNA during the first process step as shown in **FIG. 8.** The non-polymorphic process probes (labeled B) react to sample DNA during DNA restoration (labeled 2) and whole genome amplification (labeled 3) process steps. The DNA sample undergoes an overnight amplification process. The amplified DNA sample undergoes controlled enzymatic fragmentation (labeled 4) on the second day of the process run. The step 4 also includes alcohol precipitation and DNA resuspension.

### Stage 2 - Hybridization

The second stage of the production process includes process steps that hybridize the sample to the image-generating chip. The image-generating chip is prepared for hybridization in a capillary flow-through chamber. Following this, the DNA sample is hybridized (labeled 5) to the image-generating chip. Chemicals that react with sample-independent process probes are then added to the reagent that flows over the image-generating chip. Stringency (labeled C) and hybridization (labeled D) process probes produce signals during this stage. These two probes also emit signals during the washing step (labeled 6) that follows the hybridization.

### Stage 3 - Extension

The stage 3 of the process includes the core genotyping activities of extension and staining. Single bases are added to the probes during the single base extension (labeled 7) process step. Extension controls (labeled E) respond to non-sample chemicals and emit signals during this process step. The target removal (labeled 8) process step includes removing sample DNA templates attached to the probes. Target removal process probes (labeled F) generate low intensity signals indicating a successful target removal process. Finally, fluorescent labels are added to single base extensions in the staining step (labeled 9). Staining controls include perfect match and mismatch process probes that generate high intensity and low (background level) intensity signals respectively, to indicate successful completion of the staining process. The imaging step (labeled 10) completes the process run.

### Random Forest Classifiers

When the call rate from a genotyping process run is below a threshold, the operator is faced with a decision to either rerun the production run using the same sample or get a new sample from the client. Getting a new sample from a client can delay the process by one to six months. On the other hand, rerunning the production run is only helpful if the inconclusive result from the production run is due to operational or chemical processing errors and not due to sample error. The production process can be inconclusive due to a combination of mechanical, chemical processing or sample issues. It is difficult to identify the root cause of the failed production run. The technology disclosed includes a retry classifier that can take output from an inconclusive production run such as call rate and readouts from process probes to generate a retry success confidence score indicative of whether a further sample reevaluation run of the sample will produce a conclusive result. The retry confidence score can be presented to an operator to determine whether to conduct an additional evaluation run of the sample. In one implementation, the technology disclosed can also provide an image quality score generated by a good vs. bad classifier or the root cause classifier to the retry classifier to generate the retry confidence score. The technology disclosed can apply a variety of classifiers to generate a retry success confidence score indicative of whether a further sample evaluation run of the sample will produce a conclusive result. Classifiers applied can include random forest, K-nearest neighbors, multinomial logistic regression, gradient boosted trees, decision trees, Naive Bayes, perceptron, convolutional neural networks, and support vector machines. We present the implementation of the technology disclosed using a random forest classifier as an example.

A random forest classifier (also referred to as random decision forest) is an ensemble machine learning technique. Ensembled techniques or algorithms combine more than one technique of the same or different kind for classifying objects. The random forest classifier consists of multiple decision trees that operate as an ensemble. Each individual decision tree in the random forest acts as a base classifier and outputs a class prediction. The class with the most votes becomes the random forest model's prediction. The fundamental concept behind random forests is that a large number of relatively uncorrelated models (decision trees) operating as a committee will outperform any of the individual constituent models.

The technology disclosed applies the random forest classifier to outputs from an inconclusive genotyping production run. In one implementation, a trained random forest classifier is given call rates and readouts from process probes of a section of the image-generating chip from an inconclusive production run. The output from the trained classifier can be used by the operator to decide whether to reevaluate the sample in a second run of the genotyping process. A similar process can be repeated for the second run of the genotyping process to decide whether to conduct a third run of the genotyping process. In another implementation, image quality of sections from an inconclusive production run can also be provided as input to the trained random forest classifier to generate the retry success confidence score. The retry success confidence score can be compared with a threshold. If the retry confidence score is above the threshold then the operator can reevaluate the sample, otherwise, the operator can request a new sample from the client.

### Training of Random Forest Classifiers

**FIG. 9** describes the training of a random forest classifier as shown in an illustration **911**. The training data comprises input features for the labeled process run call rates stored in the database **115** as shown in **FIG. 1.** The training data also includes readouts from process probes stored in the database **127** in **FIG. 1.** In one example training of the classifier, we used call rates and probe readouts from 20,000 process runs. The readings included data from both successful and failed or inconclusive process runs. The size of the training data will grow as more results of process runs are received from laboratories performing the genotyping process.

A random forest classifier with 200 decision trees and a depth of 20 worked well. It is understood that random forest classifiers with a range of 200 to 500 decision trees and a range of depth from 10 to 40 are expected to provide good results for this implementation. We tuned the hyperparameters using randomized search cross-validation. The search range for depth was from 5 to 150 and the search range for number of trees was from 100 to 500. Increasing the number of trees can increase the performance of the model, however, it can also increase the time required for training. A training database **901** including features for 20,000 production cycle images is used to train the retry classifier **151.** In another implementation, image quality from good vs. bad and root cause classifiers can also be used to train the retry classifier.

Decision trees are prone to overfitting. To overcome this issue, a bagging technique is used to train the decision trees in random forest. Bagging is a combination of bootstrap and aggregation techniques. In bootstrap, during training, we take a sample of rows from our training database and use it to train each decision tree in the random forest. For example, a subset of features for the selected rows can be used in the training of decision tree 1. Therefore, the training data for decision tree 1 can be referred to as row sample 1 with column sample 1 or RS1+CS1. The columns or features can be selected randomly. The decision tree 2 and subsequent decision trees in the random forest are trained in a similar manner by using a subset of the training data. Note that the training data for decision trees is generated with replacement i.e., the same row data can be used in the training of multiple decision trees.

The second part of the bagging technique is the aggregation part which is applied during production. Each decision tree outputs a classification for each class. In the case of binary classification, it can be 1 or 0. The output of the random forest is the aggregation of outputs of decision trees in the random forest with a majority vote selected as the output of the random forest. By using votes from multiple decision trees, a random forest reduces high variance in the results of decision trees, thus resulting in good prediction results. By using row and column sampling to train individual decision trees, each decision tree becomes an expert with respect to training records with selected features.

During training, the output of the random forest is compared with ground truth labels and a prediction error is calculated. During backward propagation, the weights of the readouts from process probes and call rates are adjusted so that the prediction error is reduced. The parameters (such as weights) of the trained random forest classifier are stored for use in the retry classification of production cycle call rates and readouts during inference.

### Classification using Random Forest Classifiers

We now describe the generation of a retry score using a trained classifier **151. FIG. 10** shows a classification of production runs using the retry classifier **151.** The process is presented using a sequence of steps labeled from 1 to 4. The process starts at a step 1 after an inconclusive first run of the production genotyping process. Input features of production call rates and readouts from process probes stored in a database **1001** are provided as input to the classifier **151**. The classifier generates a retry success confidence score indicative of whether a further sample evaluation run of the sample will produce conclusive results (step 2). The operator can evaluate the retry success confidence score to determine whether to conduct an additional sample evaluation run of the sample. In one implementation, the retry success confidence score is compared with a threshold to decide whether to reevaluate the sample. If the operator decides to rerun the genotyping process, a second run of the production process generates genotyping results (step 3). If the results are inconclusive indicating failure of the genotyping process, the retry classifier **151** is invoked again and provided call rates and readouts from process probes as inputs. The trained classifier generates a retry success confidence score. The retry success confidence score can be used by the operator to decide whether to further reevaluate the sample or not (step 4). In one implementation, the retry classifier is invoked only after a second inconclusive production run to decide whether to conduct a third production run.

### Convolutional Neural Network

We now present another example of a machine learning model that can be used to generate a retry success confidence score from one or more sample evaluation runs that produced inconclusive results. The retry success confidence score can indicate whether a further sample evaluation run of the sample will produce a conclusive result. The score can be reported to an operator to evaluate when determining whether to conduct an additional sample evaluation run of the sample.

A convolutional neural network is a special type of neural network. The fundamental difference between a densely connected layer and a convolution layer is this: Dense layers learn global patterns in their input feature space, whereas convolution layers learn local patterns: in the case of images, patterns found in small 2D windows of the inputs. This key characteristic gives convolutional neural networks two interesting properties: (1) the patterns they learn are translation invariant and (2) they can learn spatial hierarchies of patterns.

Regarding the first, after learning a certain pattern in the lower-right corner of a picture, a convolution layer can recognize it anywhere: for example, in the upper-left corner. A densely connected network would have to learn the pattern anew if it appeared at a new location. This makes convolutional neural networks data-efficient because they need fewer training samples to learn representations, as they have generalization power.

Regarding the second, a first convolution layer can learn small local patterns such as edges, a second convolution layer will learn larger patterns made of the features of the first layers, and so on. This allows convolutional neural networks to efficiently learn increasingly complex and abstract visual concepts.

A convolutional neural network learns highly non-linear mappings by interconnecting layers of artificial neurons arranged in many different layers with activation functions that make the layers dependent. It includes one or more convolutional layers, interspersed with one or more sub-sampling layers and non-linear layers, which are typically followed by one or more fully connected layers. Each element of the convolutional neural network receives inputs from a set of features in the previous layer. The convolutional neural network learns concurrently because the neurons in the same feature map have identical weights. These local shared weights reduce the complexity of the network such that when multi-dimensional input data enter the network, the convolutional neural network avoids the complexity of data reconstruction in the feature extraction and regression or classification process.

Convolutions operate over 3D tensors, called feature maps, with two spatial axes (height and width) as well as a depth axis (also called the channels axis). For an RGB image, the dimension of the depth axis is 3, because the image has three color channels; red, green, and blue. For a black-and-white picture, the depth is 1 (levels of gray). The convolution operation extracts patches from its input feature map and applies the same transformation to all of these patches, producing an output feature map. This output feature map is still a 3D tensor: it has a width and a height. Its depth can be arbitrary, because the output depth is a parameter of the layer, and the different channels in that depth axis no longer stand for specific colors as in RGB input; rather, they stand for filters. Filters encode specific aspects of the input data: at a height level, a single filter could encode the concept "presence of a face in the input," for instance.

For example, the first convolution layer takes a feature map of size (28, 28, 1) and outputs a feature map of size (26, 26, 32): it computes 32 filters over its input. Each of these 32 output channels contains a 26 x 26 grid of values, which is a response map of the filter over the input, indicating the response of that filter pattern at different locations in the input. That is what the term feature map means: every dimension in the depth axis is a feature (or filter), and the 2D tensor output [:, :, n] is the 2D spatial map of the response of this filter over the input.

Convolutions are defined by two key parameters: (1) the size of the patches extracted from the inputs - these are typically 1 x 1, 3 x 3 or 5 x 5 and (2) the depth of the output feature map - the number of filters computed by the convolution. Often these start with a depth of 32, continue to a depth of 64, and terminate with a depth of 128 or 256.

A convolution works by sliding these windows of size 3 x 3 or 5 x 5 over the 3D input feature map, stopping at every location, and extracting the 3D patch of surrounding features (shape (window_height, window_width, input_depth)). Each such 3D patch is then transformed (via a tensor product with the same learned weight matrix, called the convolution kernel) into a 1D vector of shape (output_depth). All of these vectors are then spatially reassembled into a 3D output map of shape (height, width, output_depth). Every spatial location in the output feature map corresponds to the same location in the input feature map (for example, the lower-right corner of the output contains information about the lower-right corner of the input). For instance, with 3 x 3 windows, the vector output [i, j, :] comes from the 3D patch input [i-1: i+1, j-1 : J+1, :]. The full process **1100** is detailed in **FIG. 11.**

The convolutional neural network comprises convolution layers which perform the convolution operation between the input values and convolution filters (matrix of weights) that are learned over many gradient update iterations during the training. Let $(m, n)$ be the filter size and $W$ be the matrix of weights, then a convolution layer performs a convolution of the $W$ with the input $X$ by calculating the dot product $W \cdot x + b$, where $x$ is an instance of $X$ and $b$ is the bias. The step size by which the convolution filters slide across the input is called the stride, and the filter area $(m \times n)$ is called the receptive field. A same convolution filter is applied across different positions of the input, which reduces the number of weights learned. It also allows location-invariant learning, i.e., if an important pattern exists in the input, the convolution filters learn it no matter where it is in the sequence.

## Training a Convolutional Neural Network

**FIG. 12** depicts a block diagram **1200** of training a convolutional neural network in accordance with one implementation of the technology disclosed. The convolutional neural network is adjusted or trained so that the input data leads to a specific output estimate. The convolutional neural network is adjusted using back propagation based on a comparison of the output estimate and the ground truth until the output estimate progressively matches or approaches the ground truth.

The convolutional neural network is trained by adjusting the weights between the neurons based on the difference between the ground truth and the actual output. This is mathematically described as:

$$\Delta w_i = x_i \delta$$

where

$$\delta = (ground\ truth) - (actual\ output)$$

In one implementation, the training rule is defined as:

$$W_{nm} \leftarrow W_{nm} + \alpha(t_m - \varphi_m)a_n$$

In the equation above: the arrow indicates an update of the value; $t_m$ is the target value of neuron $m$; $\varphi_m$ is the computed current output of neuron $m$; $a_n$ is input $n$; and $\alpha$ is the learning rate.

The intermediary step in the training includes generating a feature vector from the input data using the convolution layers. The gradient with respect to the weights in each layer, starting at the output, is calculated. This is referred to as the backward pass, or going backwards. The weights in the network are updated using a combination of the negative gradient and previous weights.

In one implementation, the convolutional neural network uses a stochastic gradient update algorithm (such as ADAM) that performs backward propagation of errors by means of gradient descent. One example of a sigmoid function-based back propagation algorithm is described below:

$$\varphi = f(h) = \frac{1}{1 + e^{-h}}$$

In the sigmoid function above, $h$ is the weighted sum computed by a neuron. The sigmoid function has the following derivative:

$$\frac{\partial \varphi}{\partial h} = \varphi(1 - \varphi)$$

The algorithm includes computing the activation of all neurons in the network, yielding an output for the forward pass. The activation of neuron $m$ in the hidden layers is described as:

$$\varphi_m = \frac{1}{1 + e^{-hm}}$$

$$h_m = \sum_{n=1}^{N} a_n w_{nm}$$

This is done for all the hidden layers to get the activation described as:

$$\varphi_k = \frac{1}{1 + e^{hk}}$$

$$h_k = \sum_{m=1}^{M} \varphi_m v_{mk}$$

Then, the error and the correct weights are calculated per layer. The error at the output is computed as:

$$\delta_{ok} = (t_k - \varphi_k)\varphi_k(1-\varphi_k)$$

The error in the hidden layers is calculated as:

$$\delta_{hm} = \varphi_m(1-\varphi_m)\sum_{k=1}^{K} v_{mk}\delta_{ok}$$

The weights of the output layer are updated as:

$$v_{mk} \leftarrow v_{mk} + \alpha\delta_{ok}\varphi_m$$

The weights of the hidden layers are updated using the learning rate $\alpha$ as:

$$v_{nm} \leftarrow w_{nm} + \alpha\delta_{hm}a_n$$

In one implementation, the convolutional neural network uses a gradient descent optimization to compute the error across all the layers. In such an optimization, for an input feature vector *x* and the predicted output *ŷ*, the loss function is defined as *l* for the cost of predicting *ŷ* when the target is *y*, i.e. *l (ŷ, y)*. The predicted output *ŷ* is transformed from the input feature vector *x* using function *f*. Function *f* is parameterized by the weights of the convolutional neural network, i.e. *ŷ = f_w(x)*. The loss function is described as *l (ŷ, y) = l (f_w (x), y)*, or *Q (z, w) = l (f_w(x), y)* where *z* is an input and output data pair *(x, y)*. The gradient descent optimization is performed by updating the weights according to:

$$V_{t+1} = \mu V_t - \alpha \frac{1}{n}\sum_{i=1}^{N} \nabla_{w_t}Q(z_t, w_t)$$

$$W_{t+1} = W_t + V_{t+1}$$

In the equations above, $\alpha$ is the learning rate. Also, the loss is computed as the average over a set of *n* data pairs. The computation is terminated when the learning rate $\alpha$ is small enough upon linear convergence. In other implementations, the gradient is calculated using only selected data pairs fed to a Nesterov's accelerated gradient and an adaptive gradient to inject computation efficiency.

In one implementation, the convolutional neural network uses a stochastic gradient descent (SGD) to calculate the cost function. A SGD approximates the gradient with respect to the weights in the loss function by computing it from only one, randomized, data pair, $Z_t$, described as:

$$V_{t+1} = \mu V - \alpha \nabla_w Q(z_t, w_t)$$

$$W_{t+1} = W_t + V_{t+1}$$

In the equations above: $\alpha$ is the learning rate; $\mu$ is the momentum; and *t* is the current weight state before updating. The convergence speed of SGD is approximately $O(1/t)$ when the learning rate $\alpha$ is reduced both fast and slow enough. In other implementations, the convolutional neural network uses different loss functions such as Euclidean loss and softmax loss. In a further implementation, an Adam stochastic optimizer is used by the convolutional neural network.

**Particular Implementations**

The technology disclosed is related to scoring whether to reevaluate a sample after one or more inconclusive sample evaluation runs. The system includes logic to process readouts from a plurality of process probes which generate signals indicative of processing parameters at successive stages of sample processing. This disclosure describes eight different process probes (also referred to as control probes or probes). The technology disclosed can use three, four, five, six, seven or eight process probes in combination. Two groupings of process probes are presented. A first grouping organizes process probes in two groups based on response to a sample. In this grouping the probes are grouped in sample-dependent process probes and sample-independent process probes. A second grouping organizes process probes in three groups corresponding to three stages of a sample evaluation run.

We describe various implementations of the technology disclosed that can be practiced as a system, method, or article of manufacture. One or more features of an implementation can be combined with a base implementation. Implementations that are not mutually exclusive are taught to be combinable. One or more features of an implementation can be combined with other implementations. This disclosure periodically reminds the user of these options. In the interest of conciseness, alternative combinations of system features are not individually enumerated. Features applicable to systems, methods, and articles of manufacture are not repeated for each statutory class set of base features. Omission from some implementations of recitations that repeat these options should not be taken as limiting the combinations taught in the preceding sections - these recitations are hereby incorporated forward by reference into each of the following implementations.

**Production - List of Probes**

One implementation of the technology disclosed can be practiced as a production method of scoring whether to reevaluate a sample after one or more sample evaluation runs produced inconclusive results. Scoring can be based on call rates indicating a percentage of sample locations that capture genotype data with a quality score above a threshold and on readouts from four or more types of process probes. A trained classifier can be used to generate scores that are reported to a user or operator or to a cost algorithm to assist in deciding whether to rerun a sample.

The readouts are from the eight types of process probes presented below. The process probes include a plurality of first readouts from process probes (hybridization process probes) that respond to synthetic sequences mixed with reagents in at least high, medium, and low concentration levels and respectively produce high, medium, and low intensity signals indicative of good reagent delivery. Alternatively, two or four or five concentration levels and corresponding process probes could be used instead of the trio of high, medium, and low. The readouts include a plurality of second readouts from process probes (extension process probes) including built-in hairpin complementary sequences that respond to chemicals mixed in reagent to perform single-base extensions and produce signals indicative of good conditions for single-base extensions. The readouts include a plurality of third readouts from process probes (target removal process probes) engineered to block extensions on a 3' end of probe sequences. The synthetic targets mixed in reagents and extensions of the synthetic targets are removed after the extension and the staining process steps causing the process probes to produce low intensity signals indicative of good conditions for target removal. The readouts include a plurality of fourth readouts from process probes (staining process probes) covered with chemicals that bind fluorescent labels mixed in reagent and produce high intensity signals indicative of a good quality staining process.

Further, the readouts from these types of probes include a plurality of fifth readouts from process probes (perfect match stringency process probes) that respond to a common sequence (wild-type allele) in human DNA and produce high intensity signals indicative of good sample composition and binding conditions. The readouts include a plurality of sixth readouts from process probes (mismatch stringency process probes), including mismatched complementary bases, that respond to a common sequence in human DNA by binding weakly to the human DNA. This weak binding results in separation of the human DNA from the process probes, causing the process probes to produce approximately background-level intensity signals.

The list of readout types includes a plurality of seventh readouts from process probes (non-specific binding process probes) that respond to non-human bacterial DNA not present in human DNA and produce signals indicative of contamination of the sample by non-human bacterial DNA. The method includes a plurality of eighth readouts from at least four process probes (collectively referred to as non-polymorphic process probes) that respond to extensions by A, C, T, and G bases. These process probes target bases at non-polymorphic sites in the genome and produce signals indicative of good extensions for each of four alternative extension reagents. There are several alternative rationales for how many probes to have the classifier evaluate and how to select probes to use, as explained below.

This method and other implementations of the technology disclosed can include one or more of the following features and/or features described in connection with additional methods disclosed. In the interest of conciseness, the combinations of features disclosed in this application are not individually enumerated and are not repeated with each base set of features.

In one grouping, the technology disclosed can apply a trained classifier to three, four, five, six, seven or eight of the types of probes listed above, in combination.

This method implementation can apply the trained classifier to sections of an image-generating chip that represent samples from different genetic sources. The classifier can generate the retry success confidence score for the different genetic sources and report the scores to a user or operator on a genetic source-by-genetic source basis.

In another grouping, probe types on which the scoring is based include readouts from at least two sample-independent readouts and two sample-dependent readouts. Of course, using three sample-independent readouts would still be within the scope of the technology disclosed, if two were from the list stated and a third one was from outside the list. The sample-independent readouts are the first through fourth readouts presented above in the preceding method. The sample-dependent readouts above are the fifth through eighth readouts.

In a further grouping, probe types on which scoring is based are drawn from each of three processing stages: a first pre-hybridization stage, a second hybridization stage and a third extension stage. The readouts from the first stage are the seventh and eighth readouts. The readouts from the second stage are the first, fifth and the sixth readouts. The readouts from the third stage are the second, third and fourth readouts.

The number of readouts used in scoring can be four, five, six, seven or eight of the eight listed readouts. For instance, the scoring can include readouts from five or more of the types of process probes presented in the first method implementation. Or, the scoring can include readouts from six or more of the types of process probes presented in the first method implementation. Alternatively, the scoring can include readouts from seven or more of the types of process probes presented in the first method implementation. The scoring includes readouts from all eight of the types of process probes presented in the first method implementation.

In one implementation, the trained classifier is a random forest classifier. The random forest classifier can have a range of 200 to 500 decision trees and a range of depth from 10 to 40 branches. Other ranges for the parameters of the random forest classifiers can be used such as 250 to 450 decision trees or 300 to 400 decision trees and a range of depth from 15 to 35 or 20 to 30. In one implementation, the trained classifier is a convolutional neural network. Other types of classifiers such as support vector machines (SVM), deep learning-based approaches, gradient boosted trees, logistic regression, K-nearest neighbor, decision trees, Naive Bayes, and perceptron can be applied

Other implementations consistent with this method may include a non-transitory computer readable storage medium storing instructions executable by a processor to perform any of the methods described above. Yet another implementation may include a system with memory loaded from a computer readable storage medium with program instructions to perform any of the methods described above. The system can be loaded from either a transitory or a non-transitory computer readable storage medium.

## Short Training - Sample-Independent and Sample-Dependent Probes

Another implementation of the technology disclosed can be practiced as a training method for training a classifier to score whether to reevaluate a sample after one or more inconclusive sample evaluation runs. The method can include assembling a training set of sample evaluations. The training set can include data from one or more inconclusive sample evaluation runs that produced inconclusive results for samples, followed by an additional sample evaluation run. The training data for each of the inconclusive sample evaluation runs can include one or more call rates indicating a percentage of sample locations with a quality score above a threshold and pluralities of readouts of radiant signals from process probes.

This method can use the grouping of process probes into sample-independent and sample-dependent probes. The sample-independent probes respond to substances in reagent flow over an evaluation cell and independent of sample material to be evaluated, and generate radiant signals that reveal qualities of reagent delivery, reaction conditions, or reaction progress. The sample-dependent probes respond to sample material to be evaluated using the evaluation cell, and generate radiant signals that reveal qualities of human sample presence, non-human contamination, or effective sample extension with alternative bases.

The training data for each of the additional sample evaluation runs can include one or more ground truth indicators of a conclusive or inconclusive result. The method includes using the training data to train the classifier, to score whether an additional sample evaluation run for a particular sample is likely to produce a conclusive result. The method includes saving parameters of the trained classifier for use in determining whether to reevaluate production samples after one or more inconclusive sample evaluation runs.

This method and other implementations of the technology disclosed can include one or more of the following features and/or features described in connection with additional methods disclosed. In the interest of conciseness, the combinations of features disclosed in this application are not individually enumerated and are not repeated with each base set of features. The technology disclosed can use three, four, five, six, seven or eight features listed below, in combination.

This method can use readouts from one, two, three or four of the following sample-independent process probes. The method for training the classifier further includes using readouts from hybridization probes. Hybridization probes respond to synthetic sequences mixed with a reagent in high, medium, and low concentration levels and respectively produce high, medium, and low radiant signals indicative of good reagent delivery. Alternatively, two or four or five concentration levels and corresponding process probes could be used instead of the trio of high, medium, and low. The method disclosed can use readouts from extension probes that include a hairpin complementary sequence. The hairpin complementary sequence responds to chemicals mixed in reagent to perform single-base extensions and produces radiant signals indicative of good conditions for single base extensions. The method disclosed can use readouts from target removal probes that are engineered to block extensions on a 3' end of probe sequences, such that synthetic targets mixed in reagent and extensions of the synthetic targets are removed after extension and staining. Target removal probes produce low radiant signals indicative of good conditions for target removal. The method disclosed can use readouts from staining probes that are covered with chemicals that bind fluorescent labels mixed in reagent and produce high radiant signals indicative of a good quality staining process.

This method can use readouts from one, two, three, or four of the following sample-dependent process probes. The method disclosed can use readouts from perfect match stringency probes that respond to a common sequence (wild-type allele) in a human sample and produce high radiant signals indicative of good sample composition and binding conditions. The method disclosed can use readouts from mismatch stringency probes that respond to a common sequence in a human sample by binding weakly with the common sequence. This weak binding results in separation of the common sequence from the probes with the mismatched complementary bases, producing approximately background-level radiant signals. The method includes using readouts from non-specific binding process probes that respond to non-human contamination not present in human samples and produce radiant signals indicative of contamination of the sample by non-human sequences. The method includes using readouts from non-polymorphic process probes that respond to extensions of target bases at non-polymorphic sites of common human sample sequences, and produce radiant signals indicative of good extensions for each of four complementary extension reagents. While human samples are described herein, it is understood that sample-dependent probes in general and non-specific binding probes in particular can be used for samples obtained from other species, including but not limited to, cattle, sheep, pigs, cats, and other animals, and crops such as wheat, rice, and the like. Thus, where the term "human sample" is used herein, it will be understood that sample-dependent probes specific for genetic material for any one of a number of target organisms can be used in the methods described herein. Likewise, it will be understood that the term "non-human contamination" can more broadly refer to genetic material that is from an organism other than the target species.

Each of the features discussed in the particular implementation section for the first method implementation apply equally to this method implementation. As indicated above, all the features not repeated here should be considered repeated by reference.

Other implementations consistent with this method may include a non-transitory computer readable storage medium storing instructions executable by a processor to perform any of the methods described above. Yet another implementation may include a system with memory loaded from a computer readable storage medium with program instructions to perform any of the methods described above. The system can be loaded from either a transitory or a non-transitory computer readable storage medium.

## Long Training - Sample-Independent and Sample-Dependent Probes

Another implementation of the technology disclosed can be practiced as a training method for training a classifier to score whether to reevaluate a sample after one or more inconclusive sample evaluation runs. The method can include assembling a training set of sample evaluations. The training set can include data from one or more inconclusive sample evaluation runs that produced inconclusive results for samples, followed by an additional sample evaluation run. The training data for each of the inconclusive sample evaluation runs can include one or more call rates indicating a percentage of sample locations with a quality score above a threshold. The training data can also include pluralities of readouts of radiant signals from process probes for each of the inconclusive sample evaluation runs.

This method can use the grouping of process probes into sample-independent and sample-dependent probes. Sample-independent probes respond to substances in reagent flow over an evaluation cell and generate radiant signals independent of the sample material to be evaluated. These radiant signals reveal qualities of reagent delivery, reaction conditions, or reaction progress. Sample-independent probes are selected from a set of four sample-independent process probes. Readouts from one, two, three, or four sample-dependent probes can be used in assembling the training data set.

Sample-independent probes can be selected from a set of four sample-independent probes presented below. Readouts from one, two, three, or four sample-independent probes can be used in assembling the training data set. The sample-independent probes include first probes (referred to as hybridization process probes) that respond to synthetic sequences mixed with a reagent in high, medium, and low concentration levels and respectively produce high, medium, and low radiant signals indicative of good reagent delivery.

The sample-independent probes can include second probes (referred to as extension process probes) that include a hairpin complementary sequence that responds to chemicals mixed in reagent to perform single-base extensions. Extension process probes produce radiant signals indicative of good conditions for single base extensions.

The sample-independent probes can include third probes (referred to as target removal process probes) engineered to block extensions on a 3' end of probe sequences, such that synthetic targets mixed in reagent and extensions of the synthetic targets are removed after extension and staining. Target removal process probes produce low radiant signals indicative of good conditions for target removal.

The sample-independent probes can include fourth probes (referred to as staining probes) that are covered with chemicals that bind fluorescent labels mixed in reagent and produce high radiant signals indicative of a good quality staining process.

Sample-dependent probes can respond to sample material to be evaluated using the evaluation cell and generate radiant signals that reveal qualities of human sample presence, non-human contamination, or effective sample extension with alternative bases. Sample-dependent probes can be selected from a group including four sample-dependent process probes. Readouts from one, two, three, or four sample-dependent probes can be used in assembling the training data set. The sample-dependent probes include fifth probes (referred to as perfect match stringency process probes) that respond to a common sequence (wild-type allele) in a human sample and produce high radiant signals indicative of good sample composition and binding conditions.

Sample-dependent probes can include sixth probes (referred to as mismatch stringency process probes) that include mismatched complementary bases, which respond to a common sequence in a human sample by binding weakly, resulting in separation of the common sequence from the probes with the mismatched complementary bases, and produce approximately background-level radiant signals.

Sample-dependent probes can include seventh probes (referred to as non-specific binding process probes) that respond to non-human contamination not present in human samples and produce radiant signals indicative of contamination of the samples by non-human bacterial DNA.

Sample-dependent probes can include eighth probes (referred to as non-polymorphic process probes) that include four complementary process probes, that respond to extensions of target bases at non-polymorphic sites of common human sample sequences. Non-polymorphic process probes produce radiant signals indicative of good extension conditions.

The training data for each of the additional sample evaluation runs can include one or more ground truth indicators of a conclusive or inconclusive result. The method includes using the training data to train the classifier, to score whether an additional sample evaluation run for a particular sample is likely to produce a conclusive result. The method includes saving parameters of the trained classifier for use in determining whether to reevaluate production samples after one or more inconclusive sample evaluation runs.

Each of the features discussed in the particular implementation section for the method implementations presented above apply equally to this method implementation. As indicated above, all the features not repeated here should be considered repeated by reference.

Other implementations consistent with this method may include a non-transitory computer readable storage medium storing instructions executable by a processor to perform any of the methods described above. Yet another implementation may include a system with memory loaded from a computer readable storage medium with program instructions to perform any of the methods described above. The system can be loaded from either a transitory or a non-transitory computer readable storage medium.

### Production - Sample-Independent and Sample-Dependent Probes

Another implementation of the technology disclosed can be practiced as a production method for scoring whether to reevaluate a sample after one or more inconclusive sample evaluation runs. The method can use sample-independent and sample-dependent groupings of process probes. The method can include scoring one or more sample evaluation runs that produced inconclusive results for a sample. The scoring uses a combination of call rates indicating a percentage of sample locations that capture genotype data with quality scores above a threshold and of pluralities of readouts from process probes in the sections. The call rates indicate a percentage of sample locations that capture genotype data with quality scores above a threshold for sections of an image-generating chip. The pluralities of readouts are from sample-independent and sample-dependent process probes for the sections, which generate signals indicative of processing parameters at successive stages of sample evaluation. The method includes using a classifier trained to predict whether a further sample evaluation run of the sample will produce a conclusive result.

The process probes are grouped into sample-independent and sample-dependent process probes. Readouts from one or more sample-independent probes can be selected from a set of four sample-independent process probes presented below. The sample-independent process probes include a plurality of first hybridization process probes that respond to synthetic sequences mixed with a reagent in high, medium, and low concentration levels and respectively produce high, medium, and low intensity signals indicative of good reagent delivery. Alternatively, two or four or five concentration levels and corresponding process probes could be used instead of the trio of high, medium, and low.

The sample-independent process probes can include a plurality of second extension process probes including built-in hairpin complementary sequences that respond to chemicals mixed in reagent to perform single-base extensions. The extension process probes produce signals indicative of good conditions for single base extensions.

The sample-independent process probes can include a plurality of third target removal process probes engineered to block extensions on the 3' end of probe sequences, such that synthetic targets mixed in reagent and extensions of the synthetic targets are removed after extension and staining. Target removal process probes produce low intensity signals indicative of good conditions for target removal.

The sample-independent process probes can include a plurality of fourth staining process probes covered with chemicals that bind fluorescent labels mixed in reagent and produce high intensity signals indicative of a good quality staining process.

This method includes using readouts from one or more sample-dependent process probes that are selected from a set of four sample-dependent process probes presented below. The sample-dependent process probes include a plurality of fifth perfect match stringency process probes that respond to a common sequence (also known as wild-type allele) in human DNA and produce high intensity signals indicative of good sample composition and binding conditions.

The sample-dependent process probes can include a plurality of sixth perfect mismatch stringency process probes including mismatched complementary bases that respond to a common sequence in human DNA by binding weakly to human DNA. This weak binding results in separation of the human DNA from the process probes with mismatched complementary bases and produces approximately background-level intensity signals.

The sample-dependent process probes can include a plurality of seventh non-specific binding process probes that respond to non-human bacterial DNA not present in human DNA and produce signals indicative of contamination of the sample by non-human bacterial DNA.

Each of the plurality of eighth sample-dependent process probes, called non-polymorphic process probes, is a collection of four process probes corresponding to the four types of nucleotide bases. The non-polymorphic process probes produce signals responsive to extensions by A, C, T and G bases that target bases at non-polymorphic sites in the genome and produce signals indicative of good extensions for each of four alternative extension reagents.

The method includes generating, from the trained classifier, at least a retry success confidence score indicative of whether a further sample evaluation run of the sample will produce a conclusive result. The method includes reporting at least the retry success confidence score to an operator to evaluate when determining whether to conduct an additional sample evaluation run of the sample.

Each of the features discussed in the particular implementation section for the method implementations presented above apply equally to this method implementation. As indicated above, all the features not repeated here should be considered repeated by reference.

Other implementations consistent with this method may include a non-transitory computer readable storage medium storing instructions executable by a processor to perform any of the methods described above. Yet another implementation may include a system with memory loaded from a computer readable storage medium with program instructions to perform any of the methods described above. The system can be loaded from either a transitory or a non-transitory computer readable storage medium.

### Short Training - Probes for Three Stages of Genotyping

One implementation of the technology disclosed can be practiced as a training method for scoring whether to reevaluate a sample after one or more inconclusive sample evaluation runs. The method can include assembling a training set of sample evaluations. The training set can include data for one or more inconclusive sample evaluation runs that produced inconclusive results for samples, followed by an additional sample evaluation run. The training data for each of the inconclusive sample evaluation runs can include one or more call rates indicating a percentage of sample locations with quality scores above a threshold. The training data can also include a plurality of readouts of radiant signals from probes during at least a first, pre-hybridization stage of the sample evaluation run in which sample DNA is in a liquid form, a second stage of the sample evaluation run in which sample DNA is hybridized to the image-generating chip, and a third stage of the sample evaluation run in which probe DNA is extended and the extension is labeled with a fluorescent label. The training data for each of the additional sample evaluation runs can include one or more ground truth indicators of a conclusive or inconclusive result. The method includes using the training data to train the classifier, to score whether an additional sample evaluation run for a particular sample is likely to produce a conclusive result. The method includes saving parameters of the trained classifier for use in determining whether to reevaluate production samples after one or more inconclusive sample evaluation runs.

This method and other implementations of the technology disclosed can include one or more of the following features and/or features described in connection with additional methods disclosed. In the interest of conciseness, the combinations of features disclosed in this application are not individually enumerated and are not repeated with each base set of features.

This method of training the classifier includes the grouping of process probes according to three stages of the sample evaluation process. Readouts from one or more types of process probes from each stage of the sample evaluation process can be used by the training method disclosed to train the classifier.

The readouts from the following two process probes correspond to pre-hybridization process steps in the first stage of the sample evaluation process. A first process probe (non-specific binding process probe) from the group of probes for the first stage responds to non-human contamination not present in human samples and produces radiant signals indicative of contamination of the sample by non-human sequences. A second process probe (non-polymorphic process probe) from the group of probes for the first stage includes four complementary process probes that respond to extensions of target bases at non-polymorphic sites of common human sample sequences, and produce radiant signals indicative of good extensions for each of four complementary extension reagents.

The readouts from the following three process probes correspond to hybridization process steps in the second stage of the sample evaluation process. A first process probe (stringency - perfect match) from the group of probes for the second stage responds to a common sequence (wild-type allele) in a human sample and produces high radiant signals indicative of good sample composition and binding conditions. A second process probe (stringency - mismatch) from the group of probes for the second stage includes mismatched complementary bases, which respond to a common sequence in a human sample by binding weakly, resulting in separation of the common sequence from the probes with the mismatched complementary bases, and producing approximately background-level radiant signals. A third process probe (hybridization process probe) from the group of probes for the second stage responds to synthetic sequences mixed with a reagent in high, medium, and low concentration levels and respectively produces high, medium, and low radiant signals indicative of good reagent delivery.

The readouts from the following three process probes correspond to extension and target removal process steps in the third stage of the sample evaluation process. A first process probe (extension process probe) from the group of probes for the third stage includes a hairpin complementary sequence that responds to chemicals mixed in reagent to perform single-base extensions and to produce radiant signals indicative of good conditions for single base extensions. A second process probe (target removal process probe) from the group of probes for the third stage is engineered to block extensions on a 3' end of probe sequences, such that synthetic targets mixed in reagent and extensions of the synthetic targets are removed after extension and staining, and to produce low radiant signals indicative of good conditions for target removal. A third process probe (staining process probe) from the group of probes for the third stage is covered with chemicals that bind fluorescent labels mixed in reagent and produces high radiant signals indicative of a good quality staining process.

Each of the features discussed in the particular implementation section for the method implementations presented above apply equally to this method implementation. As indicated above, all the features not repeated here should be considered repeated by reference.

Other implementations consistent with this method may include a non-transitory computer readable storage medium storing instructions executable by a processor to perform any of the methods described above. Yet another implementation may include a system with memory loaded from a computer readable storage medium with program instructions to perform any of the methods described above. The system can be loaded from either a transitory or a non-transitory computer readable storage medium.

## Long Training - Probes for Three Stages of Genotyping

Another implementation of the technology disclosed can be practiced as a training method for scoring whether to reevaluate a sample after one or more inconclusive sample evaluation runs. This method can use a grouping of process probes based on three stages of the sample evaluation process. The method can include scoring one or more sample evaluation runs that produced inconclusive results. The scoring can include a combination of call rates and a plurality of readouts from process probes. The call rates can indicate a percentage of sample locations captured by genotype data with quality scores above a threshold. The percentage of sample locations can be calculated for a sample region on the image-generating chip such as a section. A plurality of readouts from process probes for the sections generates signals indicative of processing parameters at successive stages of sample evaluation. The method can include using a classifier trained to predict whether a further sample evaluation run of the sample will produce a conclusive result. This method can use the grouping of process probes according to three stages of the sample evaluation process. The descriptions of process probes in each group are presented below.

In a first, pre-hybridization stage of the sample evaluation run the sample DNA is in a liquid form. In the first stage, readouts from one or more probes can be selected from the following types of process probes. The readouts can include a plurality of first readouts from non-specific binding process probes that respond to non-human bacterial DNA not present in human DNA and produce signals indicative of contamination of the sample by non-human bacterial DNA. The readouts can also include a plurality of second readouts from four complementary non-polymorphic process probes, which respond to extensions of target bases at non-polymorphic sites of common human sample sequences. These process probes produce radiant signals indicative of good extensions for each of four complementary extension reagents.

In a second, hybridization stage, the sample DNA is hybridized to the image-generating chip. In the second stage, readouts from one or more process probes can be selected from a group including the following three types of process probes. The readouts from process probes for the second stage include a plurality of third readouts from perfect match stringency process probes that respond to a common sequence (wild-type allele) in a human sample and produce high radiant signals indicative of good sample composition and binding conditions. The readouts from the process probes for the second stage include a plurality of fourth readouts from mismatch stringency process probes that include mismatched complementary bases, which respond to a common sequence in a human sample by binding weakly, resulting in separation of the common sequence from the probes with the mismatched complementary bases. These process probes produce approximately background-level radiant signals. The readout from the process probes for the second stage include a plurality of fifth readouts from hybridization process probes that respond to synthetic sequences mixed with a reagent in high, medium, and low concentration levels and respectively produce high, medium, and low radiant signals indicative of good reagent delivery.

In a third stage of the sample evaluation run, the sample DNA is extended and the extension is labeled with a fluorescent label. The readouts from process probes for the third stage can include readouts of one or more of the following types of process probes. The readout from process probes for the third stage include a plurality of sixth readouts from extension process probes that include a hairpin complementary sequence that responds to chemicals mixed in reagent to perform single-base extensions and produce radiant signals indicative of good conditions for single base extensions. The readouts from process probes for the third stage include a plurality of seventh readouts from target removal process probes engineered to block extensions on a 3' end of probe sequences. When synthetic targets mixed in reagent and extensions of the synthetic targets are removed after extension and staining, the target removal process probes produce low radiant signals indicative of good conditions for target removal. The readout from process probes for the third stage include a plurality of eighth readouts from staining process probes covered with chemicals that bind fluorescent labels mixed in reagent and produce high radiant signals indicative of a good quality staining process.

This method includes generating, from the trained classifier, at least a retry success confidence score indicative of whether a further sample evaluation run of the sample will produce a conclusive result. The method includes reporting at least the retry success confidence score to an operator to evaluate when determining whether to conduct an additional sample evaluation run of the sample.

Each of the features discussed in the particular implementation section for the method implementations presented above apply equally to this method implementation. As indicated above, all the features not repeated here should be considered repeated by reference.

Other implementations consistent with this method may include a non-transitory computer readable storage medium storing instructions executable by a processor to perform any of the methods described above. Yet another implementation may include a system with memory loaded from a computer readable storage medium with program instructions to perform any of the methods described above. The system can be loaded from either a transitory or a non-transitory computer readable storage medium.

### Short Production - Probes for Three Stages of Genotyping

Another implementation of the technology disclosed can be practiced as a production method of scoring whether to reevaluate a sample after one or more inconclusive sample evaluation runs. This method can use a grouping of probes based on three stages of the sample evaluation process. The method includes using a trained classifier to score from the one or more sample evaluation runs that produced inconclusive results. The score is determined using a combination of one or more call rates and a plurality of readouts from process probes. The one or more call rates indicate a percentage of sample locations with a quality score above a threshold. The plurality of readouts of radiant signals can be selected from process probes for a first, a second and a third stage of sample evaluation. In a first, pre-hybridization stage of the sample evaluation run the sample DNA is in a liquid form. In a second stage of the sample evaluation run the sample DNA is hybridized to an image-generating chip. In a third stage of the sample evaluation run the probe DNA is extended and the extension is labeled with a fluorescent label. The method includes generating from the trained classifier, at least a retry success confidence score indicative of whether a further sample evaluation run of the sample will produce the conclusive result. The method includes reporting at least the retry success confidence score to an operator to evaluate when determining whether to conduct an additional sample evaluation run of the sample.

In one implementation, the readouts can be from types of process probes from a first pre-hybridization stage, a second stage in which the sample is hybridized to an image-generating chip and a third stage in which the probe DNA is extended, and extension is fluorescently labeled.

For the first stage, the readouts of one or more process probes can be selected from a group including a plurality of first readouts and a plurality of second readouts. The plurality of first readouts are from process probes that respond to non-human bacterial DNA not present in human DNA and produce signals indicative of contamination of the sample by non-human bacterial DNA. The plurality of second readouts are from four complementary process probes, that respond to extensions of target bases at non-polymorphic sites of common human sample sequences, and produce radiant signals indicative of good extensions for each of four complementary extension reagents. The method can use any combination of readouts from process probes selected from the plurality of first readouts and the plurality of second readouts.

For the second stage, the readouts of one or more process probes can be selected from a group including a plurality of third readouts, a plurality of fourth readouts, and a plurality of fifth readouts. The plurality of third readouts are from process probes that respond to a common sequence known as wild-type allele in a human sample and produce high radiant signals indicative of good sample composition and binding conditions. The plurality of fourth readouts are from process probes that include mismatched complementary bases, which respond to a common sequence in a human sample by binding weakly, resulting in separation of the common sequence from the probes with the mismatched complementary bases. These process probes produce approximately background level radiant signals. The plurality of fifth readouts are from process probes that respond to synthetic sequences mixed with reagent in high, medium, and low concentration levels, respectively producing high, medium, and low radiant signals indicative of good reagent delivery. The method can use any combination of readouts from process probes selected from the plurality of third readouts, the plurality of fourth readouts and the plurality of fifth readouts.

For the third stage, the readouts of one or more process probes can be selected from a group including of a plurality of sixth readouts, a plurality of seventh readouts, and a plurality of eighth readouts. The plurality of sixth readouts are from process probes that include a hairpin complementary sequence that responds to chemicals mixed in reagent to perform single-base extensions and to produce radiant signals indicative of good conditions for single base extensions. The plurality of seventh readouts are from process probes engineered to block extensions on a 3' end of probe sequences such that synthetic targets mixed in reagent and extensions of the synthetic targets are removed after extension and staining. These process probes produce low radiant signals indicative of good conditions for target removal. The plurality of eighth readouts are from process probes covered with chemicals that bind fluorescent labels mixed in reagent and produce high radiant signals indicative of a good quality staining process. The method can use any combination of readouts from process probes selected from the plurality of sixth readouts, the plurality of seventh readouts and the plurality of eighth readouts.

Each of the features discussed in the particular implementation section for the method implementations presented above apply equally to this method implementation. As indicated above, all the features not repeated here should be considered repeated by reference.

A system implementation of the technology disclosed includes one or more processors coupled to memory. The memory is loaded with the computer program instructions to score whether to reevaluate a sample after one or more inconclusive sample evaluation runs. The instructions when executed on a processor implement the method described above.

Each of the features discussed in the particular implementation section for the method implementations presented above apply equally to this system implementation. As indicated above, all the features not repeated here should be considered repeated by reference.

Other implementations consistent with this method may include a non-transitory computer readable storage medium storing instructions executable by a processor to perform any of the methods described above. Yet another implementation may include a system with memory loaded from a computer readable storage medium with program instructions to perform any of the methods described above. The system can be loaded from either a transitory or a non-transitory computer readable storage medium.

## Long Production - Probes for Three Stages of Genotyping

Another implementation of the technology disclosed can be practiced as a production method of scoring whether to reevaluate a sample after one or more inconclusive sample evaluation runs. This method can use the grouping of probes based on three stages of the sample evaluation process. The method includes scoring one or more sample evaluation runs that produced inconclusive results for a sample. The scoring can use a combination of call rates indicating a percentage of sample locations that capture genotype data with quality scores above a threshold and of pluralities of readouts from four or more type of process probes. The call rates indicate a percentage of sample locations that capture genotype data with quality scores above a threshold for sections of an image-generating chip. The pluralities of readouts are from process probes which generate signals indicative of processing parameters at successive stages of a sample evaluation run. The method can include using a classifier trained to predict whether a further sample evaluation run of the sample will produce a conclusive result.

The process probes are grouped in three groups corresponding to three stages of the sample evaluation process cycle. Readouts from one or more probes can be selected from each of the three groups of process probes. The description of process probes for each of the three groups is presented below.

In a first, pre-hybridization stage of the sample evaluation run the sample DNA is in a liquid form. In the first stage, readouts from one or more probes can be selected from the following types of process probes. The readouts can include a plurality of first readouts from non-specific binding process probes that respond to non-human bacterial DNA not present in human DNA and produce signals indicative of contamination of the sample by non-human bacterial DNA. The readouts can also include a plurality of second readouts from four complementary non-polymorphic process probes, which respond to extensions of target bases at non-polymorphic sites of common human sample sequences. These process probes produce radiant signals indicative of good extensions for each of four complementary extension reagents.

In a second, hybridization stage, the sample DNA is hybridized to the image-generating chip. In the second stage, readouts from one or more process probes can be selected from a group including the following three types of process probes. The readouts from process probes for the second stage include a plurality of third readouts from perfect match stringency process probes that respond to a common sequence (wild-type allele) in a human sample and produce high radiant signals indicative of good sample composition and binding conditions. The readouts from the process probes for the second stage include a plurality of fourth readouts from mismatch stringency process probes that include mismatched complementary bases, which respond to a common sequence in a human sample by binding weakly, resulting in separation of the common sequence from the probes with the mismatched complementary bases. These process probes produce approximately background-level radiant signals. The readout from the process probes for the second stage include a plurality of fifth readouts from hybridization process probes that respond to synthetic sequences mixed with a reagent in high, medium, and low concentration levels and respectively produce high, medium, and low radiant signals indicative of good reagent delivery.

In a third stage of the sample evaluation run, the sample DNA is extended and the extension is labeled with a fluorescent label. The readouts from process probes for the third stage can include readouts of one or more of the following types of process probes. The readout from process probes for the third stage include a plurality of sixth readouts from extension process probes that include a hairpin complementary sequence that responds to chemicals mixed in reagent to perform single-base extensions and produce radiant signals indicative of good conditions for single base extensions. The readouts from process probes for the third stage include a plurality of seventh readouts from target removal process probes engineered to block extensions on a 3' end of probe sequences. When synthetic targets mixed in reagent and extensions of the synthetic targets are removed after extension and staining, the target removal process probes produce low radiant signals indicative of good conditions for target removal. The readout from process probes for the third stage include a plurality of eighth readouts from staining process probes covered with chemicals that bind fluorescent labels mixed in reagent and produce high radiant signals indicative of a good quality staining process.

This method includes generating, from the trained classifier, at least a retry success confidence score indicative of whether a further sample evaluation run of the sample will produce a conclusive result. The method includes reporting at least the retry success confidence score to an operator to evaluate when determining whether to conduct an additional sample evaluation run of the sample.

Each of the features discussed in the particular implementation section for the method implementations presented above apply equally to this method implementation. As indicated above, all the features not repeated here should be considered repeated by reference.

Other implementations consistent with this method may include a non-transitory computer readable storage medium storing instructions executable by a processor to perform any of the methods described above. Yet another implementation may include a system with memory loaded from a computer readable storage medium with program instructions to perform any of the methods described above. The system can be loaded from either a transitory or a non-transitory computer readable storage medium.

The computer-implemented methods described above can be practiced in a system that includes computer hardware. The computer-implemented system can practice one or more of the methods described above. The computer-implemented system can incorporate any of the features of methods described immediately above or throughout this application that apply to the method implemented by the system. In the interest of conciseness, alternative combinations of system features are not individually enumerated. Features applicable to systems, methods, and articles of manufacture are not repeated for each statutory class set of base features. The reader will understand how features identified in this section can readily be combined with base features in other statutory classes.

As an article of manufacture, rather than a method, a non-transitory computer readable medium (CRM) can be loaded with program instructions executable by a processor. The program instructions, when executed, implement one or more of the computer-implemented methods described above. Alternatively, the program instructions can be loaded on a non-transitory CRM and, when combined with appropriate hardware, become a component of one or more of the computer-implemented systems that practice the methods disclosed.

Each of the features discussed in this particular implementation section for the method implementation apply equally to CRM and system implementations. As indicated above, all the method features are not repeated here, in the interest of conciseness, and should be considered repeated by reference.

## Computer System

**FIG. 13** is a simplified block diagram of a computer system **1300 that can** be used to implement the technology disclosed. The computer system typically includes at least one processor **1372** that communicates with a number of peripheral devices via a bus subsystem **1355.** These peripheral devices can include a storage subsystem **1310** including, for example, a memory subsystem **1322** and a file storage subsystem **1336,** user interface input devices **1338,** user interface output devices **1376,** and a network interface subsystem **1374.** The input and output devices allow user interaction with the computer system. The network interface subsystem provides an interface to outside networks, including an interface to corresponding interface devices in other computer systems.

In one implementation, the retry classifier **151** is communicably linked to the storage subsystem and user interface input devices.

User interface input devices **1338** can include a keyboard; pointing devices such as a mouse, trackball, touchpad, or graphics tablet; a scanner; a touch screen incorporated into the display; audio input devices such as voice recognition systems and microphones; and other types of input devices. In general, use of the term "input device" is intended to include all possible types of devices and ways to input information into a computer system.

User interface output devices **1376** can include a display subsystem, a printer, a fax machine, or non-visual displays such as audio output devices. The display subsystem can include a cathode ray tube (CRT), a flat-panel device such as a liquid crystal display (LCD), a projection device, or some other mechanism for creating a visible image. The display subsystem can also provide a non-visual display such as with audio output devices. In general, use of the term "output device" is intended to include all possible types of devices and ways to output information from a computer system to the user or to another machine or computer system.

The storage subsystem **1310** stores programming and data constructs that provide the functionality of some or all of the modules and methods described herein. These software modules are generally executed by the processor alone or in combination with other processors.

The memory used in the storage subsystem can include a number of memories including a main random access memory (RAM) **1332** for storage of instructions and data during program execution and a read only memory (ROM) **1334** in which fixed instructions are stored. The file storage subsystem **1336** can provide persistent storage for program and data files, and can include a hard disk drive, a floppy disk drive along with associated removable media, a CD-ROM drive, an optical drive, or removable media cartridges. The modules implementing the functionality of certain implementations can be stored by the file storage subsystem in the storage subsystem, or in other machines accessible by the processor.

The bus subsystem **1355** provides a mechanism for letting the various components and subsystems of the computer system communicate with each other as intended. Although the bus subsystem is shown schematically as a single bus, alternative implementations of the bus subsystem can use multiple busses.

The computer system itself can be of varying types including a personal computer, a portable computer, a workstation, a computer terminal, a network computer, a television, a mainframe, a server farm, a widely-distributed set of loosely networked computers, or any other data processing system or user device. Due to the ever-changing nature of computers and networks, the description of the computer system depicted in **FIG. 13** is intended only as a specific example for the purposes of illustrating the technology disclosed. Many other configurations of the computer system are possible having more or less components than the computer system depicted in **FIG. 13.**

The computer system **1300** includes GPUs or FPGAs **1378.** It can also include machine learning processors hosted by machine learning cloud platforms such as Google Cloud Platform, Xilinx, and Cirrascale. Examples of deep learning processors include Google's Tensor Processing Unit (TPU), rackmount solutions like GX4 Rackmount Series, GX8 Rackmount Series, NVIDIA DGX-1, Microsoft' Stratix V FPGA, Graphcore's Intelligent Processor Unit (IPU), Qualcomm's Zeroth platform with Snapdragon processors, NVIDIA's Volta, NVIDIA's DRIVE PX, NVIDIA's JETSON TX1/TX2 MODULE, Intel's Nirvana, Movidius VPU, Fujitsu DPI, ARM's DynamicIQ, IBM TrueNorth, and others.

**Claims**

1. A non-transitory computer readable storage medium impressed with computer program instructions to score whether to reevaluate a sample after one or more inconclusive sample evaluation runs, the instructions, when executed on a processor, implement a method comprising:

scoring, from the one or more sample evaluation runs that produced inconclusive results, using a classifier trained, a combination of:

one or more call rates indicating a percentage of sample locations with a quality score above a threshold, and
a plurality of readouts of radiant signals from process probes during at least a first, pre-hybridization stage of the sample evaluation run in which sample DNA is in a liquid form, a second stage of the sample evaluation run in which sample DNA is hybridized to an image-generating chip, and a third stage of the sample evaluation run in which probe DNA is extended and the extension is labeled with a fluorescent label, wherein each process probe is configured to produce the radiant signals indicative of one or more processing conditions during the sample evaluation run;

generating, from the trained classifier, at least a retry success confidence score indicative of whether a further sample evaluation run of the sample will produce a conclusive result; and reporting at least the retry success confidence score to an operator to evaluate when determining whether to conduct an additional sample evaluation run of the sample.

2. The non-transitory computer readable storage medium of claim 1, wherein the readouts are from types of process probes that include:

the readouts from a first, pre-hybridization stage of the sample evaluation run in which sample DNA is in a liquid form, the readouts of one or more probes selected from a group including:

a plurality of first readouts from process probes that respond to non-human bacterial DNA not present in human DNA and produce signals indicative of contamination of the sample by non-human bacterial DNA, and
a plurality of second readouts from four complementary process probes that respond to extensions of target bases at non-polymorphic sites of common human sample sequences, and produce radiant signals indicative of good extensions for each of four complementary extension reagents, and combinations thereof;

the readouts from a second stage of the sample evaluation run in which sample DNA is hybridized to an image-generating chip, the readouts of one or more probes selected from a group including:

a plurality of third readouts from process probes that respond to a common sequence known as wild-type allele in a human sample and produce high radiant signals indicative of good sample composition and binding conditions,

a plurality of fourth readouts from process probes that include mismatched complementary bases, which respond to a common sequence in a human sample by binding weakly, resulting in separation of the common sequence from the probes with the mismatched complementary bases, and producing approximately background level radiant signals, and

a plurality of fifth readouts from process probes that respond to synthetic sequences mixed with reagent in high, medium, and low concentration levels by respectively producing high, medium, and low radiant signals indicative of good reagent delivery, and combinations thereof; and

the readouts from a third stage of the sample evaluation run in which probe DNA is extended and the extension is labeled with a fluorescent label, the readouts from one or more probes selected from a group including:

a plurality of sixth readouts from process probes that include a hairpin complementary sequence that respond to chemicals mixed in reagent to perform single-base extensions to produce radiant signals indicative of good conditions for single base extensions,

a plurality of seventh readouts from process probes engineered to block extensions on a 3' end of probe sequences, such that synthetic targets mixed in reagent and extensions of the synthetic targets are removed after extension and staining, and to produce low radiant signals indicative of good conditions for target removal, and

a plurality of eighth readouts from process probes covered with chemicals that bind fluorescent labels mixed in reagent to produce high radiant signals indicative of a good quality staining process, and combinations thereof.

3. A system including one or more processors coupled to memory, the memory loaded with the computer program instructions of claim 1 to score whether to reevaluate the sample after one or more inconclusive sample evaluation runs.

4. The system of claim 3 wherein the readouts are from types of process probes that include:

the readouts from a first, pre-hybridization stage of the sample evaluation run in which sample DNA is in a liquid form, the readouts of one or more probes selected from a group including:

a plurality of first readouts from process probes that respond to non-human bacterial DNA not present in human DNA and produce signals indicative of contamination of the sample by non-human bacterial DNA, and

a plurality of second readouts from four complementary process probes that respond to extensions of target bases at non-polymorphic sites of common human sample sequences, and produce radiant signals indicative of good extensions for each of four complementary extension reagents, and combinations thereof;

the readouts from a second stage of the sample evaluation run in which sample DNA is hybridized to an image-generating chip, the readouts of one or more probes selected from a group including:

a plurality of third readouts from process probes that respond to a common sequence known as wild-type allele in a human sample and produce high radiant signals indicative of good sample composition and binding conditions,

a plurality of fourth readouts from process probes that include mismatched complementary bases, which respond to a common sequence in a human sample by binding weakly, resulting in separation of the common sequence from the probes with the mismatched complementary bases, and producing approximately background level radiant signals, and

a plurality of fifth readouts from process probes that respond to synthetic sequences mixed with reagent in high, medium, and low concentration levels by respectively producing high, medium, and low radiant signals indicative of good reagent delivery, and combinations thereof; and

the readouts from a third stage of the sample evaluation run in which probe DNA is extended and the extension is labeled with a fluorescent label, the readouts from one or more probes selected from a group including:

a plurality of sixth readouts from process probes that include a hairpin complementary sequence that responds to chemicals mixed in reagent to perform single-base extensions to produce radiant signals indicative of good conditions for single base extensions,
a plurality of seventh readouts from process probes engineered to block extensions on a 3' end of probe sequences, such that synthetic targets mixed in reagent and extensions of the synthetic targets are removed after extension and staining, and to produce low radiant signals indicative of good conditions for target removal, and
a plurality of eighth readouts from process probes covered with chemicals that bind fluorescent labels mixed in reagent to produce high radiant signals indicative of a good quality staining process, and combinations thereof.

5. The system of claim 3, wherein one of the process probes from the first stage responds to non-human contamination not present in human samples and produces radiant signals indicative of contamination of the sample by non-human sequences.

6. The system of claim 3, wherein the probes from the first stage include four complementary process probes that respond to extensions of target bases at non-polymorphic sites of common human sample sequences, and produce radiant signals indicative of good extensions for each of four complementary extension reagents.

7. The system of claim 3, wherein one of the process probes from the second stage responds to a common sequence known as wild-type allele in a human sample and produce high radiant signals indicative of good sample composition and binding conditions.

8. The system of claim 3, wherein one of the process probes from the second stage includes mismatched complementary bases, which respond to a common sequence in a human sample by binding weakly, resulting in separation of the common sequence from the probes with the mismatched complementary bases, and producing approximately background level radiant signals.

9. The system of claim 3, wherein one of the process probes from the second stage responds to synthetic sequences mixed with reagent in high, medium, and low concentration levels by respectively producing high, medium, and low radiant signals indicative of good reagent delivery.

10. The system of claim 3, wherein one of the process probes from the third stage includes a hairpin complementary sequence that responds to chemicals mixed in reagent to perform single-base extensions to produce radiant signals indicative of good conditions for single base extensions.

11. The system of claim 3, wherein one of the process probes from the third stage is engineered to block extensions on a 3' end of probe sequences, such that synthetic targets mixed in reagent and extensions of the synthetic targets are removed after extension and staining, and to produce low radiant signals indicative of good conditions for target removal.

12. The system of claim 3, wherein one of the process probes from the third stage is covered with chemicals that bind fluorescent labels mixed in reagent to produce high radiant signals indicative of a good quality staining process.

13. A method of scoring whether to reevaluate a sample after one or more inconclusive sample evaluation runs, including:

scoring, from the one or more sample evaluation runs that produced inconclusive results, using a classifier trained, a combination of:

one or more call rates indicating a percentage of sample locations with a quality score above a threshold, and
a plurality of readouts of radiant signals from process probes during at least a first, pre-hybridization stage of the sample evaluation run in which sample DNA is in a liquid form, a second stage of the sample evaluation run in which sample DNA is hybridized to an image-generating chip, and a third stage of the sample evaluation run in which probe DNA is extended and the extension is labeled with a fluorescent label, wherein each process probe is configured to produce the radiant signals indicative of one or more processing conditions during the sample evaluation run;

generating, from the trained classifier, at least a

55 EP 4 158 636 B1 56

retry success confidence score indicative of whether a further sample evaluation run of the sample will produce a conclusive result; and reporting at least the retry success confidence score to an operator to evaluate when determining whether to conduct an additional sample evaluation run of the sample.

14. The method of claim 13, wherein the readouts are from types of process probes that include:

the readouts from a first, pre-hybridization stage of the sample evaluation run in which sample DNA is in a liquid form, the readouts of one or more probes selected from a group including:

a plurality of first readouts from process probes that respond to non-human bacterial DNA not present in human DNA and produce signals indicative of contamination of the sample by non-human bacterial DNA, and
a plurality of second readouts from four complementary process probes that respond to extensions of target bases at non-polymorphic sites of common human sample sequences, and produce radiant signals indicative of good extensions for each of four complementary extension reagents, and combinations thereof;

the readouts from a second stage of the sample evaluation run in which sample DNA is hybridized to an image-generating chip, the readouts of one or more probes selected from a group including:

a plurality of third readouts from process probes that respond to a common sequence known as wild-type allele in a human sample and produce high radiant signals indicative of good sample composition and binding conditions,
a plurality of fourth readouts from process probes that include mismatched complementary bases, which respond to a common sequence in a human sample by binding weakly, resulting in separation of the common sequence from the probes with the mismatched complementary bases, and producing approximately background level radiant signals, and

a plurality of fifth readouts from process probes that respond to synthetic sequences mixed with reagent in high, medium, and low concentration levels by respectively producing high, medium, and low radiant signals indicative of good reagent delivery, and combinations thereof; and

the readouts from a third stage of the sample evaluation run in which probe DNA is extended and the extension is labeled with a fluorescent label, the readouts from one or more probes selected from a group including:

a plurality of sixth readouts from process probes that include a hairpin complementary sequence that responds to chemicals mixed in reagent to perform single-base extensions to produce radiant signals indicative of good conditions for single base extensions,
a plurality of seventh readouts from process probes engineered to block extensions on a 3' end of probe sequences, such that synthetic targets mixed in reagent and extensions of the synthetic targets are removed after extension and staining, and to produce low radiant signals indicative of good conditions for target removal, and
a plurality of eighth readouts from process probes covered with chemicals that bind fluorescent labels mixed in reagent to produce high radiant signals indicative of a good quality staining process, and combinations thereof.

15. A method of scoring whether to reevaluate a sample after one or more inconclusive sample evaluation runs, including:

assembling a training set of sample evaluations, each including one or more inconclusive sample evaluation runs that produced inconclusive results for samples, followed by an additional sample evaluation run,
wherein training data for each of the inconclusive sample evaluation runs includes

one or more call rates indicating a percentage of sample locations with a quality score above a threshold and
a plurality of readouts of radiant signals from process probes during at least a first, pre-hybridization stage of the sample evaluation run in which sample DNA is in a liquid form, a second stage of the sample evaluation run in which sample DNA is hybridized to an image-generating chip, and a third stage of the sample evaluation run in which probe DNA is extended and the extension is labeled with a fluorescent label, wherein each process probe is configured to produce the radiant signals indicative of one or more processing conditions during the sample evaluation run;

wherein training data for each of the additional sample evaluation runs includes one or more ground truth indicators of a conclusive or inconclusive result;

training a classifier, using the training data, to score whether an additional sample evaluation run for a particular sample is likely to produce the conclusive result; and

saving parameters of the trained classifier for use determining whether to reevaluate production samples after one or more inconclusive sample evaluation runs.

16. The method of claim 15 wherein the plurality of readouts are from types of process probes that include:

the readouts from a first, pre-hybridization stage of the sample evaluation run in which sample DNA is in a liquid form, the readouts of one or more probes selected from a group including:

a plurality of first readouts from process probes that respond to non-human bacterial DNA not present in human DNA and produce signals indicative of contamination of the sample by non-human bacterial DNA, and

a plurality of second readouts from four complementary process probes that respond to extensions of target bases at non-polymorphic sites of common human sample sequences, and produce radiant signals indicative of good extensions for each of four complementary extension reagents, and combinations thereof;

the readouts from a second stage of the sample evaluation run in which sample DNA is hybridized to an image-generating chip, the readouts of one or more probes selected from a group including:

a plurality of third readouts from process probes that respond to a common sequence known as wild-type allele in a human sample and produce high radiant signals indicative of good sample composition and binding conditions,

a plurality of fourth readouts from process probes that include mismatched complementary bases, which respond to a common sequence in a human sample by binding weakly, resulting in separation of the common sequence from the probes with the mismatched complementary bases, and producing approximately background level radiant signals, and

a plurality of fifth readouts from process probes that respond to synthetic sequences mixed with reagent in high, medium, and low concentration levels by respectively producing high, medium, and low radiant signals indicative of good reagent delivery, and combinations thereof; and

the readouts from a third stage of the sample evaluation run in which probe DNA is extended and the extension is labeled with a fluorescent label, the readouts from one or more probes selected from a group including:

a plurality of sixth readouts from process probes that include a hairpin complementary sequence that responds to chemicals mixed in reagent to perform single-base extensions to produce radiant signals indicative of good conditions for single base extensions,

a plurality of seventh readouts from process probes engineered to block extensions on a 3' end of probe sequences, such that synthetic targets mixed in reagent and extensions of the synthetic targets are removed after extension and staining, and to produce low radiant signals indicative of good conditions for target removal, and

a plurality of eighth readouts from process probes covered with chemicals that bind fluorescent labels mixed in reagent to produce high radiant signals indicative of a good quality staining process, and combinations thereof.

17. A non-transitory computer readable storage medium impressed with computer program instructions to train a classifier to score whether to reevaluate a sample after one or more inconclusive sample evaluation runs, the instructions, when executed on a processor, implement a method according to claim 15 or 16.

18. A system including one or more processors coupled to memory, the memory loaded with computer program instructions of claim 17 to train a classifier to score whether to reevaluate the sample after one or more inconclusive sample evaluation runs.

**Patentansprüche**

1. Nichtflüchtiges, computerlesbares Speichermedium, das mit Computerprogrammanweisungen versehen ist, um zu beurteilen, ob eine Probe nach einem oder mehreren nicht eindeutigen Probenauswertungsläufen erneut ausgewertet werden soll, wobei die Anweisungen, wenn sie auf einem Prozessor ausgeführt werden, ein Verfahren implementieren, das Folgendes umfasst:

Beurteilen, anhand des einen oder der mehreren Probenauswertungsläufe, die zu keinem eindeutigen Ergebnis geführt haben, unter Verwendung eines trainierten Klassifikators, einer Kombination aus Folgendem:

einer oder mehreren Genotypisierungsraten, die einen Prozentsatz von Probenpositionen mit einer Qualitätsbeurteilung über einem Schwellenwert angeben, und einer Vielzahl von Auslesedaten von Strahlungssignalen von Prozesssonden während mindestens einer ersten Vorhybridisierungsphase des Probenauswertungslaufs, in der die Proben-DNA in flüssiger Form vorliegt, einer zweiten Phase des Probenauswertungslaufs, in der die Proben-DNA an einen Bilderzeugungschip hybridisiert wird, und einer dritten Phase des Probenauswertungslaufs, in der die Sonden-DNA verlängert und die Verlängerung mit einem Fluoreszenzmarker markiert wird, wobei jede Prozesssonde so ausgelegt ist, dass sie die Strahlungssignale erzeugt, die auf einen oder mehrere Verarbeitungsbedingungen während des Probenauswertungslaufs hinweisen;

Erzeugen, anhand des trainierten Klassifikators, von mindestens einem Konfidenzwert für den Wiederholungserfolg, der angibt, ob ein weiterer Probenauswertungslauf mit der Probe ein eindeutiges Ergebnis liefern wird; und Mitteilen von mindestens dem Konfidenzwert für den Wiederholungserfolg an einen Bediener, damit dieser ihn bei der Entscheidung, ob ein zusätzlicher Probenauswertungslauf mit der Probe durchgeführt werden soll, auswerten kann.

2. Nichtflüchtiges, computerlesbares Speichermedium nach Anspruch 1, wobei die Auslesedaten von Arten von Prozesssonden stammen, die Folgendes beinhalten:

die Auslesedaten aus einer ersten Vorhybridisierungsphase des Probenauswertungslaufs, in der die Proben-DNA in flüssiger Form vorliegt, wobei die Auslesedaten einer oder mehrerer Sonden aus einer Gruppe ausgewählt sind, die Folgendes beinhaltet:

eine Vielzahl von ersten Auslesedaten von Prozesssonden, die auf nicht-menschliche bakterielle DNA reagieren, die nicht in menschlicher DNA vorkommt, und Signale erzeugen, die auf eine Kontamination der Probe durch nicht-menschliche bakterielle DNA hinweisen, und eine Vielzahl von zweiten Auslesedaten von vier komplementären Prozesssonden, die auf Verlängerungen von Zielbasen an nicht-polymorphen Stellen gängiger menschlicher Probensequenzen reagieren und Strahlungssignale erzeugen, die auf gute Verlängerungen für jedes von vier komplementären Verlängerungsreagenzien hinweisen, sowie Kombinationen davon;

die Auslesedaten aus einer zweiten Phase des Probenauswertungslaufs, in der die Proben-DNA an einen Bilderzeugungschip hybridisiert wird, wobei die Auslesedaten einer oder mehrerer Sonden aus einer Gruppe ausgewählt sind, die Folgendes beinhaltet:

eine Vielzahl von dritten Auslesedaten von Prozesssonden, die auf eine gängige Sequenz, bekannt als Wildtyp-Allel, in einer menschlichen Probe reagieren und hohe Strahlungssignale erzeugen, die auf eine gute Probenzusammensetzung und Bindungsbedingungen hinweisen, eine Vielzahl von vierten Auslesedaten von Prozesssonden, die nicht übereinstimmende komplementäre Basen beinhalten, die auf eine gängige Sequenz in einer menschlichen Probe durch schwache Bindung reagieren, was zu einer Abtrennung der gängigen Sequenz von den Sonden mit den nicht übereinstimmenden komplementären Basen führt und Strahlungssignale in etwa auf Hintergrundniveau erzeugt, und eine Vielzahl von fünften Auslesedaten von Prozesssonden, die auf synthetische Sequenzen, die mit Reagenz in hoher, mittlerer und niedriger Konzentration vermischt sind, durch Erzeugung hoher, mittlerer beziehungsweise niedriger Strahlungssignale reagieren, die auf eine gute Reagenzzufuhr hinweisen, sowie Kombinationen davon; und

die Auslesedaten aus einer dritten Phase des Probenauswertungslaufs, in der die Sonden-DNA verlängert und die Verlängerung mit einem Fluoreszenzmarker markiert wird, wobei die Auslesedaten einer oder mehrerer Sonden aus einer Gruppe ausgewählt sind, die Folgendes beinhaltet:

eine Vielzahl von sechsten Auslesedaten von Prozesssonden, die eine komplementäre Haarnadelsequenz beinhalten, die auf in Reagenzien gemischte Chemikalien mit der Durchführung von Einzelbasenverlängerungen reagiert, und Strahlungssignale erzeugen, die auf gute Bedingungen für Einzelbasenverlängerungen hinweisen, eine Vielzahl von siebten Auslesedaten von Prozesssonden, die so konstruiert sind, dass sie Verlängerungen am 3'-Ende von Sondensequenzen blockieren, sodass in Reagenzien gemischte synthetische Zielmoleküle und Verlängerungen der synthetischen Zielmoleküle nach der Verlängerung und Färbung entfernt werden, und niedrige Strahlungssignale erzeugen, die auf gute Bedingungen für die Zielmolekülentfernung hinweisen, und eine Vielzahl von achten Auslesedaten von Prozesssonden, die mit Chemikalien bedeckt sind, die in Reagenzien gemischte Fluoreszenzmarker binden, und hohe Strahlungssignale erzeugen, die auf einen qualitativ hochwertigen Färbungsprozess hinweisen, sowie Kombinationen davon.

3. System beinhaltend einen oder mehrere Prozessoren, die mit einem Speicher verbunden sind, wobei in den Speicher die Computerprogrammanweisungen nach Anspruch 1 geladen sind, um zu beurteilen, ob die Probe nach einem oder mehreren nicht eindeutigen Probenauswertungsläufen erneut ausgewertet werden soll.

4. System nach Anspruch 3, wobei die Auslesedaten von Arten von Prozesssonden stammen, die Folgendes beinhalten:

die Auslesedaten aus einer ersten Vorhybridisierungsphase des Probenauswertungslaufs, in der die Proben-DNA in flüssiger Form vorliegt, wobei die Auslesedaten einer oder mehrerer Sonden aus einer Gruppe ausgewählt sind, die Folgendes beinhaltet:

eine Vielzahl von ersten Auslesedaten von Prozesssonden, die auf nicht-menschliche bakterielle DNA reagieren, die nicht in menschlicher DNA vorkommt, und Signale erzeugen, die auf eine Kontamination der Probe durch nicht-menschliche bakterielle DNA hinweisen, und eine Vielzahl von zweiten Auslesedaten von vier komplementären Prozesssonden, die auf Verlängerungen von Zielbasen an nicht-polymorphen Stellen gängiger menschlicher Probensequenzen reagieren und Strahlungssignale erzeugen, die auf gute Verlängerungen für jedes von vier komplementären Verlängerungsreagenzien hinweisen, sowie Kombinationen davon;

die Auslesedaten aus einer zweiten Phase des Probenauswertungslaufs, in der die Proben-DNA an einen Bilderzeugungschip hybridisiert wird, wobei die Auslesedaten einer oder mehrerer Sonden aus einer Gruppe ausgewählt sind, die Folgendes beinhaltet:

eine Vielzahl von dritten Auslesedaten von Prozesssonden, die auf eine gängige Sequenz, bekannt als Wildtyp-Allel, in einer menschlichen Probe reagieren und hohe Strahlungssignale erzeugen, die auf eine gute Probenzusammensetzung und Bindungsbedingungen hinweisen, eine Vielzahl von vierten Auslesedaten von Prozesssonden, die nicht übereinstimmende komplementäre Basen beinhalten, die auf eine gängige Sequenz in einer menschlichen Probe durch schwache Bindung reagieren, was zu einer Abtrennung der gängigen Sequenz von den Sonden mit den nicht übereinstimmenden komplementären Basen führt und Strahlungssignale in etwa auf Hintergrundniveau erzeugt, und eine Vielzahl von fünften Auslesedaten von Prozesssonden, die auf synthetische Sequenzen, die mit Reagenz in hoher, mittlerer und niedriger Konzentration vermischt sind, durch Erzeugung hoher, mittlerer beziehungsweise niedriger Strahlungssignale reagieren, die auf eine gute Reagenzzufuhr hinweisen, sowie Kombinationen davon; und

die Auslesedaten aus einer dritten Phase des Probenauswertungslaufs, in der die Sonden-DNA verlängert und die Verlängerung mit einem Fluoreszenzmarker markiert wird, wobei die Auslesedaten einer oder mehrerer Sonden aus einer Gruppe ausgewählt sind, die Folgendes beinhaltet:

eine Vielzahl von sechsten Auslesedaten von Prozesssonden, die eine komplementäre Haarnadelsequenz beinhalten, die auf in Reagenzien gemischte Chemikalien mit der Durchführung von Einzelbasenverlängerungen reagiert, und Strahlungssignale erzeugen, die auf gute Bedingungen für Einzelbasenverlängerungen hinweisen, eine Vielzahl von siebten Auslesedaten von Prozesssonden, die so konstruiert sind, dass sie Verlängerungen am 3'-Ende von Sondensequenzen blockieren, sodass in Reagenzien gemischte synthetische Zielmoleküle und Verlängerungen der synthetischen Zielmoleküle nach der Verlängerung und Färbung entfernt werden, und niedrige Strahlungssignale erzeugen, die auf gute Bedingungen für die Zielmolekülentfernung hinweisen, und

eine Vielzahl von achten Auslesedaten von Prozesssonden, die mit Chemikalien bedeckt sind, die in Reagenzien gemischte Fluoreszenzmarker binden, und hohe Strahlungssignale erzeugen, die auf einen qualitativ hochwertigen Färbungsprozess hinweisen, sowie Kombinationen davon.

5. System nach Anspruch 3, wobei eine der Prozesssonden aus der ersten Phase auf eine nicht-menschliche Kontamination reagiert, die in menschlichen Proben nicht vorhanden ist, und Strahlungssignale erzeugt, die auf eine Kontamination der Probe durch nicht-menschliche Sequenzen hinweisen.

6. System nach Anspruch 3, wobei die Sonden aus der ersten Phase vier komplementäre Prozesssonden beinhalten, die auf Verlängerungen von Zielbasen an nicht-polymorphen Stellen gängiger menschlicher Probensequenzen reagieren und Strahlungssignale erzeugen, die auf gute Verlängerungen für jedes von vier komplementären Verlängerungsreagenzien hinweisen.

7. System nach Anspruch 3, wobei eine der Prozesssonden aus der zweiten Phase auf eine gängige Sequenz, bekannt als Wildtyp-Allel, in einer menschlichen Probe reagiert und hohe Strahlungssignale erzeugt, die auf eine gute Probenzusammensetzung und Bindungsbedingungen hinweisen.

8. System nach Anspruch 3, wobei eine der Prozesssonden aus der zweiten Phase nicht übereinstimmende komplementäre Basen beinhaltet, die auf eine gängige Sequenz in einer menschlichen Probe durch schwache Bindung reagieren, was zu einer Abtrennung der gängigen Sequenz von den Sonden mit den nicht übereinstimmenden komplementären Basen führt und Strahlungssignale in etwa auf Hintergrundniveau erzeugt.

9. System nach Anspruch 3, wobei eine der Prozesssonden aus der zweiten Phase auf synthetische Sequenzen, die mit Reagenz in hoher, mittlerer und niedriger Konzentration vermischt sind, durch Erzeugung hoher, mittlerer beziehungsweise niedriger Strahlungssignale reagiert, die auf eine gute Reagenzzufuhr hinweisen.

10. System nach Anspruch 3, wobei eine der Prozesssonden aus der dritten Phase eine komplementäre Haarnadelsequenz beinhaltet, die auf in Reagenzien gemischte Chemikalien mit der Durchführung von Einzelbasenverlängerungen reagiert, und Strahlungssignale erzeugt, die auf gute Bedingungen für Einzelbasenverlängerungen hinweisen.

11. System nach Anspruch 3, wobei eine der Prozesssonden aus der dritten Phase so konstruiert ist, dass sie Verlängerungen am 3'-Ende von Sondensequenzen blockiert, sodass in Reagenzien gemischte synthetische Zielmoleküle und Verlängerungen der synthetischen Zielmoleküle nach der Verlängerung und Färbung entfernt werden, und niedrige Strahlungssignale erzeugt, die auf gute Bedingungen für die Zielmolekülentfernung hinweisen.

12. System nach Anspruch 3, wobei eine der Prozesssonden aus der dritten Phase mit Chemikalien bedeckt ist, die in Reagenzien gemischte Fluoreszenzmarker binden, und hohe Strahlungssignale erzeugt, die auf einen qualitativ hochwertigen Färbungsprozess hinweisen.

13. Verfahren zur Beurteilung, ob eine Probe nach einem oder mehreren nicht eindeutigen Probenauswertungsläufen erneut ausgewertet werden soll, beinhaltend:

Beurteilen, anhand des einen oder der mehreren Probenauswertungsläufe, die zu keinem eindeutigen Ergebnis geführt haben, unter Verwendung eines trainierten Klassifikators, einer Kombination aus Folgendem:

einer oder mehreren Genotypisierungsraten, die einen Prozentsatz von Probenpositionen mit einer Qualitätsbeurteilung über einem Schwellenwert angeben, und

eine Vielzahl von Auslesedaten von Strahlungssignalen von Prozesssonden während mindestens einer ersten Vorhybridisierungsphase des Probenauswertungslaufs, in der die Proben-DNA in flüssiger Form vorliegt, einer zweiten Phase des Probenauswertungslaufs, in der die Proben-DNA an einen Bilderzeugungschip hybridisiert wird, und einer dritten Phase des Probenauswertungslaufs, in der die Sonden-DNA verlängert und die Verlängerung mit einem Fluoreszenzmarker markiert wird, wobei jede Prozesssonde so ausgelegt ist, dass sie die Strahlungssignale erzeugt, die auf einen oder mehrere Verarbeitungsbedingungen während des Probenauswertungslaufs hinweisen;

Erzeugen, anhand des trainierten Klassifikators, von mindestens einem Konfidenzwert für den Wiederholungserfolg, der angibt, ob ein weiterer Probenauswertungslauf mit der Probe ein eindeutiges Ergebnis liefern wird; und Mitteilen von mindestens dem Konfidenzwert für den Wiederholungserfolg an einen Bediener, damit dieser ihn bei der Entscheidung, ob ein zusätzlicher Probenauswertungslauf mit der Probe durchgeführt werden soll, auswerten kann.

14. Verfahren nach Anspruch 13, wobei die Auslesedaten von Arten von Prozesssonden stammen, die Folgendes beinhalten:

die Auslesedaten aus einer ersten Vorhybridisierungsphase des Probenauswertungslaufs, in der die Proben-DNA in flüssiger Form vorliegt, wobei die Auslesedaten einer oder mehrerer Sonden aus einer Gruppe ausgewählt sind, die Folgendes beinhaltet:

eine Vielzahl von ersten Auslesedaten von Prozesssonden, die auf nicht-menschliche bakterielle DNA reagieren, die nicht in menschlicher DNA vorkommt, und Signale erzeugen, die auf eine Kontamination der Probe durch nicht-menschliche bakterielle DNA hinweisen, und eine Vielzahl von zweiten Auslesedaten von vier komplementären Prozesssonden, die auf Verlängerungen von Zielbasen an nicht-polymorphen Stellen gängiger menschlicher Probensequenzen reagieren und Strahlungssignale erzeugen, die auf gute Verlängerungen für jedes von vier komplementären Verlängerungsreagenzien hinweisen, sowie Kombinationen davon;

die Auslesedaten aus einer zweiten Phase des Probenauswertungslaufs, in der die Proben-DNA an einen Bilderzeugungschip hybridisiert wird, wobei die Auslesedaten einer oder mehrerer Sonden aus einer Gruppe ausgewählt sind, die Folgendes beinhaltet:

eine Vielzahl von dritten Auslesedaten von Prozesssonden, die auf eine gängige Sequenz, bekannt als Wildtyp-Allel, in einer menschlichen Probe reagieren und hohe Strahlungssignale erzeugen, die auf eine gute Probenzusammensetzung und Bindungsbedingungen hinweisen, eine Vielzahl von vierten Auslesedaten von Prozesssonden, die nicht übereinstimmende komplementäre Basen beinhalten, die auf eine gängige Sequenz in einer menschlichen Probe durch schwache Bindung reagieren, was zu einer Abtrennung der gängigen Sequenz von den Sonden mit den nicht übereinstimmenden komplementären Basen führt und Strahlungssignale in etwa auf Hintergrundniveau erzeugt, und eine Vielzahl von fünften Auslesedaten von Prozesssonden, die auf synthetische Sequenzen, die mit Reagenz in hoher, mittlerer und niedriger Konzentration vermischt sind, durch Erzeugung hoher, mittlerer beziehungsweise niedriger Strahlungssignale reagieren, die auf eine gute Reagenzzufuhr hinweisen, sowie Kombinationen davon; und

die Auslesedaten aus einer dritten Phase des Probenauswertungslaufs, in der die Sonden-DNA verlängert und die Verlängerung mit einem Fluoreszenzmarker markiert wird, wobei die Auslesedaten einer oder mehrerer Sonden aus einer Gruppe ausgewählt sind, die Folgendes beinhaltet:

eine Vielzahl von sechsten Auslesedaten von Prozesssonden, die eine komplementäre Haarnadelsequenz beinhalten, die auf in Reagenzien gemischte Chemikalien mit der Durchführung von Einzelbasenverlängerungen reagiert, und Strahlungssignale erzeugen, die auf gute Bedingungen für Einzelbasenverlängerungen hinweisen, eine Vielzahl von siebten Auslesedaten von Prozesssonden, die so konstruiert sind, dass sie Verlängerungen am 3'-Ende von Sondensequenzen blockieren, sodass in Reagenzien gemischte synthetische Zielmoleküle und Verlängerungen der synthetischen Zielmoleküle nach der Verlängerung und Färbung entfernt werden, und niedrige Strahlungssignale erzeugen, die auf gute Bedingungen für die Zielmolekülentfernung hinweisen, und eine Vielzahl von achten Auslesedaten von Prozesssonden, die mit Chemikalien bedeckt sind, die in Reagenzien gemischte Fluoreszenzmarker binden, und hohe Strahlungssignale erzeugen, die auf einen qualitativ hochwertigen Färbungsprozess hinweisen, sowie Kombinationen davon.

15. Verfahren zur Beurteilung, ob eine Probe nach einem oder mehreren nicht eindeutigen Probenauswertungsläufen erneut ausgewertet werden soll, beinhaltend:

Zusammenstellen eines Trainingsdatensatzes von Probenauswertungen, wobei jeder einen oder mehrere nicht eindeutige Probenauswertungsläufe beinhaltet, die zu nicht eindeutigen Ergebnissen für Proben geführt haben, gefolgt von einem zusätzlichen Probenauswertungslauf,

wobei Trainingsdaten für jeden der nicht eindeutigen Probenauswertungsläufe Folgendes beinhalten:

eine oder mehrere Genotypisierungsraten, die einen Prozentsatz von Probenpositionen mit einer Qualitätsbeurteilung über einem Schwellenwert angeben, und einer Vielzahl von Auslesedaten von Strahlungssignalen von Prozesssonden während mindestens einer ersten Vorhybridisierungsphase des Probenauswertungslaufs, in der die Proben-DNA in flüssiger Form vorliegt, einer zweiten Phase des Probenauswertungslaufs, in der die Proben-DNA an einen Bilderzeugungschip hybridisiert wird, und einer dritten Phase des Probenauswertungslaufs, in der die Sonden-DNA verlängert und die Verlängerung mit einem Fluoreszenzmarker markiert wird, wobei jede Prozesssonde so ausgelegt ist, dass sie die Strahlungssignale erzeugt, die auf einen oder mehrere Verarbeitungsbedingungen während des Probenauswertungslaufs hinweisen;

wobei die Trainingsdaten für jeden der zusätzlichen Probenauswertungsläufe einen oder mehrere geprüfte, korrekte Referenzindikatoren für ein eindeutiges oder nicht eindeutiges Ergebnis beinhalten;
Trainieren eines Klassifikators unter Verwendung der Trainingsdaten, um zu beurteilen, ob ein zusätzlicher Probenauswertungslauf für eine bestimmte Probe wahrscheinlich zu dem eindeutigen Ergebnis führen wird; und
Speichern von Parametern des trainierten Klassifikators zur Verwendung bei der Entscheidung, ob Produktionsproben nach einem oder mehreren nicht eindeutigen Probenauswertungsläufen erneut ausgewertet werden sollen.

16. Verfahren nach Anspruch 15, wobei die Vielzahl von Auslesedaten von Arten von Prozesssonden stammen, die Folgendes beinhalten:

die Auslesedaten aus einer ersten Vorhybridisierungsphase des Probenauswertungslaufs, in der die Proben-DNA in flüssiger Form vorliegt, wobei die Auslesedaten einer oder mehrerer Sonden aus einer Gruppe ausgewählt sind, die Folgendes beinhaltet:

eine Vielzahl von ersten Auslesedaten von Prozesssonden, die auf nicht-menschliche bakterielle DNA reagieren, die nicht in menschlicher DNA vorkommt, und Signale erzeugen, die auf eine Kontamination der Probe durch nicht-menschliche bakterielle DNA hinweisen, und
eine Vielzahl von zweiten Auslesedaten von vier komplementären Prozesssonden, die auf Verlängerungen von Zielbasen an nicht-polymorphen Stellen gängiger menschlicher Probensequenzen reagieren und Strahlungssignale erzeugen, die auf gute Verlängerungen für jedes von vier komplementären Verlängerungsreagenzien hinweisen, sowie Kombinationen davon;

die Auslesedaten aus einer zweiten Phase des Probenauswertungslaufs, in der die Proben-DNA an einen Bilderzeugungschip hybridisiert wird, wobei die Auslesedaten einer oder mehrerer Sonden aus einer Gruppe ausgewählt sind, die Folgendes beinhaltet:

eine Vielzahl von dritten Auslesedaten von Prozesssonden, die auf eine gängige Sequenz, bekannt als Wildtyp-Allel, in einer menschlichen Probe reagieren und hohe Strahlungssignale erzeugen, die auf eine gute Probenzusammensetzung und Bindungsbedingungen hinweisen,
eine Vielzahl von vierten Auslesedaten von Prozesssonden, die nicht übereinstimmende komplementäre Basen beinhalten, die auf eine gängige Sequenz in einer menschlichen Probe durch schwache Bindung reagieren, was zu einer Abtrennung der gängigen Sequenz von den Sonden mit den nicht übereinstimmenden komplementären Basen führt und Strahlungssignale in etwa auf Hintergrundniveau erzeugt, und
eine Vielzahl von fünften Auslesedaten von Prozesssonden, die auf synthetische Sequenzen, die mit Reagenz in hoher, mittlerer und niedriger Konzentration vermischt sind, durch Erzeugung hoher, mittlerer beziehungsweise niedriger Strahlungssignale reagieren, die auf eine gute Reagenzzufuhr hinweisen, sowie Kombinationen davon; und

die Auslesedaten aus einer dritten Phase des Probenauswertungslaufs, in der die Sonden-DNA verlängert und die Verlängerung mit einem Fluoreszenzmarker markiert wird, wobei die Auslesedaten einer oder mehrerer Sonden aus einer Gruppe ausgewählt sind, die Folgendes beinhaltet:

eine Vielzahl von sechsten Auslesedaten von Prozesssonden, die eine komplementäre Haarnadelsequenz beinhalten, die auf in Reagenzien gemischte Chemikalien mit der Durchführung von Einzelbasenverlängerungen reagiert, und Strahlungssignale erzeugen, die auf gute Bedingungen für Einzelbasenverlängerungen hinweisen, eine Vielzahl von siebten Auslesedaten von Prozesssonden, die so konstruiert sind, dass sie Verlängerungen am 3'-Ende von Sondensequenzen blockieren, sodass in Reagenzien gemischte synthetische Zielmoleküle und Verlängerungen der synthetischen Zielmoleküle nach der Verlängerung und Färbung entfernt werden, und niedrige Strahlungssignale erzeugen, die auf gute Bedingungen für die Zielmolekülentfernung hinweisen, und eine Vielzahl von achten Auslesedaten von Prozesssonden, die mit Chemikalien bedeckt sind, die in Reagenzien gemischte Fluoreszenzmarker binden, und hohe Strahlungssignale erzeugen, die auf einen qualitativ hochwertigen Färbungsprozess hinweisen, sowie Kombinationen davon.

17. Nichtflüchtiges, computerlesbares Speichermedium, das mit Computerprogrammanweisungen versehen ist, um einen Klassifikator so zu trainieren, dass er beurteilt, ob eine Probe nach einem oder mehreren nicht eindeutigen Probenauswertungsläufen erneut ausgewertet werden soll; wobei die Anweisungen, wenn sie auf einem Prozessor ausgeführt werden, ein Verfahren nach Anspruch 15 oder 16 implementieren.

18. System beinhaltend einen oder mehrere Prozessoren, die mit einem Speicher verbunden sind, wobei in den Speicher Computerprogrammanweisungen nach Anspruch 17 geladen sind, um einen Klassifikator so zu trainieren, dass er beurteilt, ob die Probe nach einem oder mehreren nicht eindeutigen Probenauswertungsläufen erneut ausgewertet werden soll.

**Revendications**

1. Support de stockage non transitoire lisible par ordinateur contenant des instructions de programme informatique pour établir un score précisant s'il convient de réévaluer un échantillon après une ou plusieurs réalisations d'évaluation d'échantillon peu concluantes, les instructions, lorsqu'elles sont exécutées sur un processeur, mettent en œuvre un procédé comprenant :

l'établissement d'un score, à partir des une ou plusieurs réalisations d'évaluation d'échantillon qui ont produit des résultats peu concluants, à l'aide d'un classifieur entraîné, pour une combinaison :

d'un ou plusieurs taux d'appel indiquant un pourcentage d'emplacements d'échantillon avec un score de qualité supérieur à un seuil, et
d'une pluralité de lectures de signaux de rayonnement provenant de sondes de traitement durant au moins une première étape de préhybridation de la réalisation d'évaluation d'échantillon dans laquelle l'ADN d'échantillon est sous forme liquide, une deuxième étape de la réalisation d'évaluation d'échantillon dans laquelle l'ADN d'échantillon est hybridé à une puce de génération d'image, et une troisième étape de la réalisation d'évaluation d'échantillon dans laquelle l'ADN de sonde est étendu et l'extension est marquée avec un marqueur fluorescent, dans lequel chaque sonde de traitement est configurée pour produire les signaux de rayonnement indiquant une ou plusieurs conditions de traitement durant la réalisation d'évaluation d'échantillon ;

la génération, à partir du classifieur entraîné, d'au moins un score de confiance de réussite de relance indiquant si une autre réalisation d'évaluation d'échantillon de l'échantillon produira un résultat concluant ; et
la communication au moins du score de confiance de réussite de relance à un opérateur pour évaluer le moment où déterminer s'il convient de procéder à une réalisation d'évaluation d'échantillon supplémentaire de l'échantillon.

2. Support de stockage non transitoire lisible par ordinateur selon la revendication 1, dans lequel les lectures proviennent de types de sondes de traitement qui incluent :

les lectures provenant d'une première étape de préhybridation de la réalisation d'évaluation d'échantillon dans laquelle l'ADN d'échantillon est sous forme liquide, les lectures d'une ou plusieurs sondes étant sélectionnées dans un groupe incluant :

une pluralité de premières lectures provenant de sondes de traitement qui réagissent à de l'ADN bactérien non humain qui n'est pas présent dans l'ADN humain et produisent des signaux indiquant une contamination de l'échantillon par de l'ADN bactérien non humain, et

une pluralité de deuxièmes lectures provenant de quatre sondes de traitement complémentaires qui réagissent à des extensions de bases cibles au niveau de sites non polymorphes de séquences d'échantillons humains communes, et produisent des signaux de rayonnement indiquant de bonnes extensions pour chacun des quatre réactifs d'extension complémentaires, et des combinaisons de celles-ci ;

les lectures provenant d'une deuxième étape de la réalisation d'évaluation d'échantillon dans laquelle l'ADN d'échantillon est hybridé à une puce de génération d'image, les lectures d'une ou plusieurs sondes étant sélectionnées dans un groupe incluant :

une pluralité de troisièmes lectures provenant de sondes de traitement qui réagissent à une séquence commune connue sous le nom d'allèle de type sauvage dans un échantillon humain et produisent des signaux de rayonnement élevés indiquant une bonne composition d'échantillon et de bonnes conditions de liaison,

une pluralité de quatrièmes lectures provenant de sondes de traitement qui incluent des bases complémentaires non appariées, qui réagissent à une séquence commune dans un échantillon humain en se liant faiblement, ce qui a pour résultat une séparation de la séquence commune des sondes avec les bases complémentaires non appariées et ce qui produit des signaux de rayonnement d'un niveau proche du fond, et

une pluralité de cinquièmes lectures provenant de sondes de traitement qui réagissent à des séquences synthétiques mélangées à un réactif à des niveaux de concentration élevés, moyens et faibles en produisant respectivement des signaux de rayonnement élevés, moyens et faibles indiquant une bonne distribution de réactif, et des combinaisons de celles-ci ; et

les lectures provenant d'une troisième étape de la réalisation d'évaluation d'échantillon dans laquelle l'ADN de sonde est étendu et l'extension est marquée avec un marqueur fluorescent, les lectures provenant d'une ou plusieurs sondes étant sélectionnées dans un groupe incluant :

une pluralité de sixièmes lectures provenant de sondes de traitement qui incluent une séquence complémentaire en épingle à cheveux qui réagit à des produits chimiques mélangés dans un réactif pour effectuer des extensions de base unique afin de produire des signaux de rayonnement indiquant de bonnes conditions pour des extensions de base unique,

une pluralité de septièmes lectures provenant de sondes de traitement conçues pour bloquer des extensions sur une extrémité 3' de séquences de sonde, de telle sorte que des cibles synthétiques mélangées dans un réactif et des extensions des cibles synthétiques soient éliminées après extension et coloration, et pour produire des signaux de rayonnement faibles indiquant de bonnes conditions pour l'élimination de cibles, et

une pluralité de huitièmes lectures provenant de sondes de traitement recouvertes de produits chimiques qui lient des marqueurs fluorescents mélangés dans un réactif pour produire des signaux de rayonnement élevés indiquant un processus de coloration de bonne qualité, et des combinaisons de celles-ci.

3. Système incluant un ou plusieurs processeurs couplés à une mémoire, la mémoire étant chargée des instructions de programme informatique de la revendication 1 pour établir un score précisant s'il convient de réévaluer l'échantillon après une ou plusieurs réalisations d'évaluation d'échantillon peu concluantes.

4. Système selon la revendication 3, dans lequel les lectures proviennent de types de sondes de traitement qui incluent :

les lectures provenant d'une première étape de préhybridation de la réalisation d'évaluation d'échantillon dans laquelle l'ADN d'échantillon est sous forme liquide, les lectures d'une ou plusieurs sondes étant sélectionnées dans un groupe incluant :

une pluralité de premières lectures provenant de sondes de traitement qui réagissent à de l'ADN bactérien non humain qui n'est pas présent dans l'ADN humain et produisent des signaux indiquant une contamination de l'échantillon par de l'ADN bactérien non humain, et

une pluralité de deuxièmes lectures provenant de quatre sondes de traitement complémentaires qui réagissent à des extensions de bases cibles au niveau de sites non polymorphes de séquences d'échantillons humains communes, et produisent des signaux de rayonnement indiquant de bonnes extensions pour chacun des quatre réactifs d'extension complémentaires, et des combinaisons de celles-ci ;

les lectures provenant d'une deuxième étape de la réalisation d'évaluation d'échantillon dans laquelle l'ADN d'échantillon est hybridé à une puce de génération d'image, les lectures d'une ou plusieurs sondes étant sélectionnées dans un groupe incluant :

une pluralité de troisièmes lectures provenant de sondes de traitement qui réagissent à une séquence commune connue sous le nom d'allèle de type sauvage dans un échantillon humain et produisent des signaux de rayonnement élevés indiquant une bonne composition d'échantillon et de bonnes conditions de liaison, une pluralité de quatrièmes lectures provenant de sondes de traitement qui incluent des bases complémentaires non appariées, qui réagissent à une séquence commune dans un échantillon humain en se liant faiblement, ce qui a pour résultat une séparation de la séquence commune des sondes avec les bases complémentaires non appariées et ce qui produit des signaux de rayonnement d'un niveau proche du fond, et une pluralité de cinquièmes lectures provenant de sondes de traitement qui réagissent à des séquences synthétiques mélangées à un réactif à des niveaux de concentration élevés, moyens et faibles en produisant respectivement des signaux de rayonnement élevés, moyens et faibles indiquant une bonne distribution de réactif, et des combinaisons de celles-ci ; et

les lectures provenant d'une troisième étape de la réalisation d'évaluation d'échantillon dans laquelle l'ADN de sonde est étendu et l'extension est marquée avec un marqueur fluorescent, les lectures provenant d'une ou plusieurs sondes étant sélectionnées dans un groupe incluant :

une pluralité de sixièmes lectures provenant de sondes de traitement qui incluent une séquence complémentaire en épingle à cheveux qui réagit à des produits chimiques mélangés dans un réactif pour effectuer des extensions de base unique afin de produire des signaux de rayonnement indiquant de bonnes conditions pour des extensions de base unique, une pluralité de septièmes lectures provenant de sondes de traitement conçues pour bloquer des extensions sur une extrémité 3' de séquences de sonde, de telle sorte que des cibles synthétiques mélangées dans un réactif et des extensions des cibles synthétiques soient éliminées après extension et coloration, et pour produire des signaux de rayonnement faibles indiquant de bonnes conditions pour l'élimination de cibles, et une pluralité de huitièmes lectures provenant de sondes de traitement recouvertes de produits chimiques qui lient des marqueurs fluorescents mélangés dans un réactif pour produire des signaux de rayonnement élevés indiquant un processus de coloration de bonne qualité, et des combinaisons de celles-ci.

5.　Système selon la revendication 3, dans lequel une des sondes de traitement de la première étape réagit à une contamination non humaine qui n'est pas présente dans des échantillons humains et produit des signaux de rayonnement indiquant une contamination de l'échantillon par des séquences non humaines.

6.　Système selon la revendication 3, dans lequel les sondes de la première étape incluent quatre sondes de traitement complémentaires qui réagissent à des extensions de bases cibles au niveau de sites non polymorphes de séquences d'échantillons humains communes, et produisent des signaux de rayonnement indiquant de bonnes extensions pour chacun des quatre réactifs d'extension complémentaires.

7.　Système selon la revendication 3, dans lequel une des sondes de traitement de la deuxième étape réagit à une séquence commune connue sous le nom d'allèle de type sauvage dans un échantillon humain et produit des signaux de rayonnement élevés indiquant une bonne composition d'échantillon et de bonnes conditions de liaison.

8.　Système selon la revendication 3, dans lequel une des sondes de traitement de la deuxième étape inclut des bases complémentaires non appariées, qui réagissent à une séquence commune dans un échantillon humain en se liant faiblement, ce qui a pour résultat une séparation de la séquence commune des sondes avec les bases complémentaires non appariées et ce qui produit des signaux de rayonnement d'un niveau proche du fond.

9.　Système selon la revendication 3, dans lequel une des sondes de traitement de la deuxième étape réagit à des séquences synthétiques mélangées à un réactif à des niveaux de concentration élevés, moyens et faibles en produisant respectivement des signaux de rayonnement élevés, moyens et faibles indiquant une bonne distribution de réactif.

10. Système selon la revendication 3, dans lequel une des sondes de traitement de la troisième étape inclut une séquence complémentaire en épingle à cheveux qui réagit à des produits chimiques mélangés dans un réactif pour effectuer des extensions de base unique afin de produire des signaux de rayonnement indiquant de bonnes conditions pour des extensions de base unique.

11. Système selon la revendication 3, dans lequel une des sondes de traitement de la troisième étape est conçue pour bloquer des extensions sur une extrémité 3' de séquences de sonde, de telle sorte que des cibles synthétiques mélangées dans un réactif et des extensions des cibles synthétiques soient éliminées après extension et coloration, et pour produire des signaux de rayonnement faibles indiquant de bonnes conditions pour l'élimination de cibles.

12. Système selon la revendication 3, dans lequel une des sondes de traitement de la troisième étape est recouverte de produits chimiques qui lient des marqueurs fluorescents mélangés dans un réactif pour produire des signaux de rayonnement élevés indiquant un processus de coloration de bonne qualité.

13. Procédé d'établissement d'un score précisant s'il convient de réévaluer un échantillon après une ou plusieurs réalisations d'évaluation d'échantillon peu concluantes, incluant :

l'établissement d'un score, à partir des une ou plusieurs réalisations d'évaluation d'échantillon qui ont produit des résultats peu concluants, à l'aide d'un classifieur entraîné, pour une combinaison :

d'un ou plusieurs taux d'appel indiquant un pourcentage d'emplacements d'échantillon avec un score de qualité supérieur à un seuil, et
d'une pluralité de lectures de signaux de rayonnement provenant de sondes de traitement durant au moins une première étape de préhybridation de la réalisation d'évaluation d'échantillon dans laquelle l'ADN d'échantillon est sous forme liquide, une deuxième étape de la réalisation d'évaluation d'échantillon dans laquelle l'ADN d'échantillon est hybridé à une puce de génération d'image, et une troisième étape de la réalisation d'évaluation d'échantillon dans laquelle l'ADN de sonde est étendu et l'extension est marquée avec un marqueur fluorescent, dans lequel chaque sonde de traitement est configurée pour produire les signaux de rayonnement indiquant une ou plusieurs conditions de traitement durant la réalisation d'évaluation d'échantillon ;

la génération, à partir du classifieur entraîné, d'au moins un score de confiance de réussite de relance indiquant si une autre réalisation d'évaluation d'échantillon de l'échantillon produira un résultat concluant ; et
la communication au moins du score de confiance de réussite de relance à un opérateur pour évaluer le moment où déterminer s'il convient de procéder à une réalisation d'évaluation d'échantillon supplémentaire de l'échantillon.

14. Procédé selon la revendication 13, dans lequel les lectures proviennent de types de sondes de traitement qui incluent :

les lectures provenant d'une première étape de préhybridation de la réalisation d'évaluation d'échantillon dans laquelle l'ADN d'échantillon est sous forme liquide, les lectures d'une ou plusieurs sondes étant sélectionnées dans un groupe incluant :

une pluralité de premières lectures provenant de sondes de traitement qui réagissent à de l'ADN bactérien non humain qui n'est pas présent dans l'ADN humain et produisent des signaux indiquant une contamination de l'échantillon par de l'ADN bactérien non humain, et
une pluralité de deuxièmes lectures provenant de quatre sondes de traitement complémentaires qui réagissent à des extensions de bases cibles au niveau de sites non polymorphes de séquences d'échantillons humains communes, et produisent des signaux de rayonnement indiquant de bonnes extensions pour chacun des quatre réactifs d'extension complémentaires, et des combinaisons de celles-ci ;

les lectures provenant d'une deuxième étape de la réalisation d'évaluation d'échantillon dans laquelle l'ADN d'échantillon est hybridé à une puce de génération d'image, les lectures d'une ou plusieurs sondes étant sélectionnées dans un groupe incluant :

une pluralité de troisièmes lectures provenant de sondes de traitement qui réagissent à une séquence commune connue sous le nom d'allèle de type sauvage dans un échantillon humain et produisent des signaux de rayonnement élevés indiquant une bonne composition d'échantillon et de bonnes conditions de liaison,

une pluralité de quatrièmes lectures provenant de sondes de traitement qui incluent des bases complémentaires non appariées, qui réagissent à une séquence commune dans un échantillon humain en se liant faiblement,

ce qui a pour résultat une séparation de la séquence commune des sondes avec les bases complémentaires non appariées, et ce qui produit des signaux de rayonnement d'un niveau proche du fond, et

une pluralité de cinquièmes lectures provenant de sondes de traitement qui réagissent à des séquences synthétiques mélangées à un réactif à des niveaux de concentration élevés, moyens et faibles en produisant respectivement des signaux de rayonnement élevés, moyens et faibles indiquant une bonne distribution de réactif, et des combinaisons de celles-ci ; et

les lectures provenant d'une troisième étape de la réalisation d'évaluation d'échantillon dans laquelle l'ADN de sonde est étendu et l'extension est marquée avec un marqueur fluorescent, les lectures provenant d'une ou plusieurs sondes étant sélectionnées dans un groupe incluant :

une pluralité de sixièmes lectures provenant de sondes de traitement qui incluent une séquence complémentaire en épingle à cheveux qui réagit à des produits chimiques mélangés dans un réactif pour effectuer des extensions de base unique afin de produire des signaux de rayonnement indiquant de bonnes conditions pour des extensions de base unique,

une pluralité de septièmes lectures provenant de sondes de traitement conçues pour bloquer des extensions sur une extrémité 3' de séquences de sonde, de telle sorte que des cibles synthétiques mélangées dans un réactif et des extensions des cibles synthétiques soient éliminées après extension et coloration, et pour produire des signaux de rayonnement faibles indiquant de bonnes conditions pour l'élimination de cibles, et

une pluralité de huitièmes lectures provenant de sondes de traitement recouvertes de produits chimiques qui lient des marqueurs fluorescents mélangés dans un réactif pour produire des signaux de rayonnement élevés indiquant un processus de coloration de bonne qualité, et des combinaisons de celles-ci.

15. Procédé d'établissement d'un score précisant s'il convient de réévaluer un échantillon après une ou plusieurs réalisations d'évaluation d'échantillon peu concluantes, incluant :

la constitution d'un ensemble d'entraînement d'évaluations d'échantillon, chacune incluant une ou plusieurs réalisations d'évaluation d'échantillon peu concluantes qui ont produit des résultats peu concluants pour des échantillons, suivies d'une réalisation d'évaluation d'échantillon supplémentaire,
dans lequel des données d'entraînement pour chacune des réalisations d'évaluation d'échantillon peu concluantes incluent

un ou plusieurs taux d'appel indiquant un pourcentage d'emplacements d'échantillon avec un score de qualité supérieur à un seuil et
une pluralité de lectures de signaux de rayonnement provenant de sondes de traitement durant au moins une première étape de préhybridation de la réalisation d'évaluation d'échantillon dans laquelle l'ADN d'échantillon est sous forme liquide, une deuxième étape de la réalisation d'évaluation d'échantillon dans laquelle l'ADN d'échantillon est hybridé à une puce de génération d'image, et une troisième étape de la réalisation d'évaluation d'échantillon dans laquelle l'ADN de sonde est étendu et l'extension est marquée avec un marqueur fluorescent, dans lequel chaque sonde de traitement est configurée pour produire les signaux de rayonnement indiquant une ou plusieurs conditions de traitement durant la réalisation d'évaluation d'échantillon ;

dans lequel des données d'entraînement pour chacune des réalisations d'évaluation d'échantillon supplémentaires incluent un ou plusieurs indicateurs de réalité de terrain d'un résultat concluant ou peu concluant ;
l'entraînement d'un classifieur, à l'aide des données d'entraînement, pour établir un score précisant si une réalisation d'évaluation d'échantillon supplémentaire pour un échantillon particulier risque de produire le résultat concluant ; et
la sauvegarde de paramètres du classifieur entraîné pour une utilisation servant à déterminer s'il convient de réévaluer des échantillons de production après une ou plusieurs réalisations d'évaluation d'échantillon peu concluantes.

16. Procédé selon la revendication 15, dans lequel la pluralité de lectures proviennent de types de sondes de traitement qui incluent :

les lectures provenant d'une première étape de préhybridation de la réalisation d'évaluation d'échantillon dans laquelle l'ADN d'échantillon est sous forme liquide, les lectures d'une ou plusieurs sondes étant sélectionnées dans un groupe incluant :

une pluralité de premières lectures provenant de sondes de traitement qui réagissent à de l'ADN bactérien non humain qui n'est pas présent dans l'ADN humain et produisent des signaux indiquant une contamination de l'échantillon par de l'ADN bactérien non humain, et

une pluralité de deuxièmes lectures provenant de quatre sondes de traitement complémentaires qui réagissent à des extensions de bases cibles au niveau de sites non polymorphes de séquences d'échantillons humains communes, et produisent des signaux de rayonnement indiquant de bonnes extensions pour chacun des quatre réactifs d'extension complémentaires, et des combinaisons de celles-ci ;

les lectures provenant d'une deuxième étape de la réalisation d'évaluation d'échantillon dans laquelle l'ADN d'échantillon est hybridé à une puce de génération d'image, les lectures d'une ou plusieurs sondes étant sélectionnées dans un groupe incluant :

une pluralité de troisièmes lectures provenant de sondes de traitement qui réagissent à une séquence commune connue sous le nom d'allèle de type sauvage dans un échantillon humain et produisent des signaux de rayonnement élevés indiquant une bonne composition d'échantillon et de bonnes conditions de liaison,

une pluralité de quatrièmes lectures provenant de sondes de traitement qui incluent des bases complémentaires non appariées, qui réagissent à une séquence commune dans un échantillon humain en se liant faiblement, ce qui a pour résultat une séparation de la séquence commune des sondes avec les bases complémentaires non appariées et ce qui produit des signaux de rayonnement d'un niveau proche du fond, et

une pluralité de cinquièmes lectures provenant de sondes de traitement qui réagissent à des séquences synthétiques mélangées à un réactif à des niveaux de concentration élevés, moyens et faibles en produisant respectivement des signaux de rayonnement élevés, moyens et faibles indiquant une bonne distribution de réactif, et des combinaisons de celles-ci ; et

les lectures provenant d'une troisième étape de la réalisation d'évaluation d'échantillon dans laquelle l'ADN de sonde est étendu et l'extension est marquée avec un marqueur fluorescent, les lectures provenant d'une ou plusieurs sondes étant sélectionnées dans un groupe incluant :

une pluralité de sixièmes lectures provenant de sondes de traitement qui incluent une séquence complémentaire en épingle à cheveux qui réagit à des produits chimiques mélangés dans un réactif pour effectuer des extensions de base unique afin de produire des signaux de rayonnement indiquant de bonnes conditions pour des extensions de base unique,

une pluralité de septièmes lectures provenant de sondes de traitement conçues pour bloquer des extensions sur une extrémité 3' de séquences de sonde, de telle sorte que des cibles synthétiques mélangées dans un réactif et des extensions des cibles synthétiques soient éliminées après extension et coloration, et pour produire des signaux de rayonnement faibles indiquant de bonnes conditions pour l'élimination de cibles, et

une pluralité de huitièmes lectures provenant de sondes de traitement recouvertes de produits chimiques qui lient des marqueurs fluorescents mélangés dans un réactif pour produire des signaux de rayonnement élevés indiquant un processus de coloration de bonne qualité, et des combinaisons de celles-ci.

17. Support de stockage non transitoire lisible par ordinateur contenant des instructions de programme informatique pour entraîner un classifieur afin d'établir un score précisant s'il convient de réévaluer un échantillon après une ou plusieurs réalisations d'évaluation d'échantillon peu concluantes, les instructions, lorsqu'elles sont exécutées sur un processeur, mettent en œuvre un procédé selon la revendication 15 ou 16.

18. Système incluant un ou plusieurs processeurs couplés à une mémoire, la mémoire étant chargée des instructions de programme informatique de la revendication 17 pour entraîner un classifieur afin d'établir un score précisant s'il convient de réévaluer l'échantillon après une ou plusieurs réalisations d'évaluation d'échantillon peu concluantes.

100

FIG. 1

200

Feature Generator 185

Process Probe Readout Encoder 225

Call Rates Organizer 245

FIG. 2

Genotyping Process Steps

ACCESSION | EXTRACTION | AMP -> FRAG -> PRECIP -> RESUSPEND | HYB -> XSTAIN | SCAN -> AUTOCALL | QC -> REQUEUE -> DELIVERY

300

FIG. 3

EP 4 158 636 B1

Images of Sections on Image Generating Chip

EP 4 158 636 B1

FIG. 4

EP 4 158 636 B1

**500**

Process Control Probes

FIG. 5

_600_

Process Probe Readouts

Sample-Independent Process Probe Readouts
- Staining Controls
- Extension Controls
- Target Removal Controls
- Hybradization Controls

Sample-Dependent Process Probe Readouts
- Stringency Controls
- Non-specific Binding Controls
- Non-polymorphic Controls

**FIG. 6**

Staining Process Probes

705 ⟶

Bead —————— **DNP**

Bead —————— **Biotin**

706 ⟶

① High intensities

② Low intensities

③ Background

**FIG. 7A-1**

EP 4 158 636 B1

Signal Intensities from Staining Process Probes

707

FIG. 7A-2

EP 4 158 636 B1

FIG. 7B-1

**FIG. 7B-2**

Target Removal Process Probes

FIG. 7C-1

727

Target Removal
Red

Target Removal
Green

EP 4 158 636 B1

**FIG. 7C-2**

Hybridization Process Probes

730

Bead

B    Extension

3'              Synthetic targets              5'

B = Biotin

Synthetic targets in high/medium/low
concentrations

**FIG. 7D-1**

732

**FIG. 7D-2**

EP 4 158 636 B1

Stringency Process Probes

733

Perfect Match
(PM) Sequence   734

Extension

D   3'

Bead

3'                                                                        5'

Mismatch   735
(MM) Sequence

Extension

D   3'

Bead

3'                                                                        5'

D = DNP

**FIG. 7E-1**

Signal Intensities from Stringency Process Probes

FIG. 7E-2

EP 4 158 636 B1

Non-polymorphic Process Probes

740

NP (A)

NP (T)

NP (C)

NP (G)

**FIG. 7F-1**

Signal Intensities from Non-polymorphic Process Probes

**FIG. 7F-2**

**FIG. 7G**

EP 4 158 636 B1

**FIG. 8**

**FIG. 9**

EP 4 158 636 B1

**FIG. 10**

EP 4 158 636 B1

1100

## Convolutional Neural Network

FIG. 11

**Training Convolutional Neural Network**

FIG. 12

Storage Subsystem 1310

Memory Subsystem 1322

RAM
1332

ROM
1334

File Storage
Subsystem
1336

Retry Classifier
151

User Interface
Input Devices
1338

Bus Subsystem 1355

CPU
1372

Network Interface
Subsystem
1374

User Interface
Output Devices
1376

GPU, FPGA
1378

**FIG. 13**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 17332904 B
- US 63032083
- US 62968950

**Non-patent literature cited in the description**

- **HUANG et al.** *BMC Bioinformatics*, 2004, vol. 5, 36